# EUROPEAN PATENT SPECIFICATION

(11) **EP 1 948 821 B1**
(45) Date of publication and mention of the grant of the patent: **04.03.2015**
(21) Application number: 06749784.2
(22) Date of filing: 11.04.2006
(51) Int. Cl.: C12Q 1/68

(54) **REPAIR OF NUCLEIC ACIDS FOR IMPROVED AMPLIFICATION**
REPARATUR VON NUKLEINSÄUREN FÜR VERBESSERTE AMPLIFIKATION
RÉPARATION DES ACIDES NUCLÉIQUES POUR UNE AMPLIFICATION AMÉLIORÉE

(30) Priority: 20.10.2005 WO PCT/US2005/038281
(43) Date of publication of application: 30.07.2008
(73) Proprietor: NEW ENGLAND BIOLABS, INC., Ipswich, MA 01938-2723 (US)
(72) Inventor: EVANS, Thomas C., Topsfield, Massachusetts 01983 (US); SLATKO, Barton, Ipswich, Massachusetts 01938 (US); CHEN, Lixin, Beverly, Massachusetts 01915 (US); VAISVILA, Romaldas, Ipswich, Massachusetts 01938 (US); GUAN, Chudi, Wenham, Massachusetts 01984 (US); KUCERA, Rebecca, Hamilton, Massachusetts 01982 (US)
(74) Representative: Griffin, Philippa Jane
(86) International application number: PCT/US2006/013513
(87) International publication number: WO 2007/046854

(56) References cited:
- WO-A-2005/017173
- WO-A-2006/019784
- WO-A-2006/047461
- US-A- 5 459 039
- PUSCH C M ET AL: "Repair of degraded duplex DNA from prehistoric samples using Escherichia coli DNA polymerase I and T4 DNA ligase." NUCLEIC ACIDS RESEARCH. 1 FEB 1998, vol. 26, no. 3, 1 February 1998 (1998-02-01), pages 857-859, XP002369708 ISSN: 0305-1048
- DI BERNARDO GIOVANNI ET AL: "Enzymatic repair of selected cross-linked homoduplex molecules enhances nuclear gene rescue from Pompeii and Herculaneum remains." NUCLEIC ACIDS RESEARCH. 15 FEB 2002, vol. 30, no. 4, 15 February 2002 (2002-02-15), page e16, XP002369709 ISSN: 1362-4962 cited in the application
- KOW Y W: "Repair of deaminated bases in DNA" FREE RADICAL BIOLOGY AND MEDICINE 01 OCT 2002 UNITED STATES, vol. 33, no. 7, 1 October 2002 (2002-10-01), pages 886-893, XP002395857 ISSN: 0891-5849
- FROMENTY ET AL: "Escherichia coli exonuclease III enhances long PCR amplification of damaged DNA templates" NUCLEIC ACIDS RESEARCH, OXFORD UNIVERSITY PRESS, SURREY, GB, vol. 28, no. 11, June 2000 (2000-06), page e50, XP002151230 ISSN: 0305-1048
- WELLINGER R E ET AL: "Taq DNA polymerase blockage at pyrimidine dimers." NUCLEIC ACIDS RESEARCH. 15 APR 1996, vol. 24, no. 8, 15 April 1996 (1996-04-15), pages 1578-1579, XP002369825 ISSN: 0305-1048 cited in the application
- KAYSER K.: "RestoraseTM: A novel DNA Polymerase Blend that repairs damaged DNA" ORIGINS SIGMA-ALDRICH, [Online] vol. 14, March 2004 (2004-03), pages 4-5, XP002369826 Retrieved from the Internet: URL:http://www.sigma-origins.co.uk/pdfs/ba ckissues/1100689612.pdf> [retrieved on 2006-02-24]
- MOSER MICHAEL J ET AL: "Enzymatic repair of an expanded genetic information system.", NUCLEIC ACIDS RESEARCH, vol. 31, no. 17, 1 September 2003 (2003-09-01), pages 5048-5053, ISSN: 0305-1048
- LIU PINGFANG ET AL: "DNA ligases ensure fidelity by interrogating minor groove contacts", NUCLEIC ACIDS RESEARCH, vol. 32, no. 15, 2004, pages 4503-4511, ISSN: 0305-1048

### SEQUENCE LISTING

<110> New England Biolabs, Inc. Evans, Thomas slatko, Barton chen, Lixin Vaisvila, Romaldus Guan, Chudi Kucera, Rebecca
<120> Repair of Nucleic Acids for Improved Amplification
<130> NEB-256-PCT-2
<150> 11/255,290
   <151> 2005-10-20
<150> 60/620,896
   <151> 2004-10-21
<150> 60/646,728
   <151> 2005-01-24
<150> 60/673,925
   <151> 2005-04-21
<160> 44
<170> PatentIn version 3.2
<210> 1
   <211> 23
   <212> DNA
   <213> artificial
<220>
   <223> primer
<400> 1
   cgaacgtcgc gcagagaaac agg 23
<210> 2
   <211> 20
   <212> DNA
   <213> artificial
<220>
   <223> primer
<400> 2
   cctgctctgc cgcttcacgc 20
<210> 3
   <211> 25
   <212> DNA
   <213> artificial
<220>
   <223> primer
<400> 3
   tttttagcaa tacactacac agcaa 25
<210> 4
   <211> 19
   <212> DNA
   <213> artificial
<220>
   <223> primer
<400> 4
   attacgccaa tcgatcacg 19
<210> 5
   <211> 27
   <212> DNA
   <213> artificial
<220>
   <223> primer
<400> 5
   gcacagaagc tattatgcgt ccccagg 27
<210> 6
   <211> 25
   <212> DNA
   <213> artificial
<220>
   <223> primer
<220>
   <221> misc_feature
   <222> (6)..(6)
   <223> 8-oxo-Guanine
<220>
   <221> misc_feature
   <222> (18)..(18)
   <223> m=c or a
<400> 6
   ggggggagag tttgatcmtg gctca 25
<210> 7
   <211> 28
   <212> DNA
   <213> artificial
<220>
   <223> primer
<220>
   <221> misc_feature
   <222> (6)..(6)
   <223> 8-oxo-Guanine
<220>
   <221> misc_feature
   <222> (12)..(12)
   <223> y=c or t
<400> 7
   ggggggtacg gytaccttgt tacgactt 28
<210> 8
   <211>. 24
   <212> DNA
   <213> artificial
<220>
   <223> primer
<400> 8
   ccatgattca gtgtgcccgt ctgg 24
<210> 9
   <211> 29
   <212> DNA
   <213> artificial
<220>
   <223> primer
<400> 9
   tgtcgatcag gatgatctgg acgaagagc 29
<210> 10
   <211> 33
   <212> DNA
   <213> artificial
<220>
   <223> primer
<400> 10
   cgaaagcttt caaggatctt accgctgttg aga 33
<210> 11
   <211> 813
   <212> DNA
   <213> unknown
<220>
   <223> Thermus thermophilus endonuclease IV
<400> 11
   atgccgcgct acgggttcca cctttccatc gccgggaaaa agggcgtggc cggggcggtg      60
   gaggaggcca ccgccctcgg cctcaccgct ttccagatct tcgccaaaag cccgcggagc     120
   tggcgcccaa gggccctctc cccggccgag gtggaggcct tccgcgcctt aagggaggcc      180
   tccgggggcc tccccgccgt gatccacgcc tcctacctgg tcaacctggg ggcggagggg     240
   gagctttggg agaagagcgt ggcgagcctg gcggacgacc tggagaaggc cgccctcctc     300
   ggggtggagt acgtggtcgt ccaccccggc tcgggccgcc ccgagcgggt caaggaaggg     360
   gccctcaagg ccctgcgcct cgccggcgtc cgctcccgcc ccgtcctcct cgtggagaac     420
   accgccgggg gcggggagaa ggtgggggcg cggtttgagg agctcgcctg gctcgtggcg     480
   gacacccccc tccaggtctg cctggacacc tgccacgcct acgccgccgg gtacgacgtg      540
   gccgaggacc ccttgggggt cctggacgcc ctggaccggg ccgtgggcct ggagcgggtg     600
   cccgtggtcc acctcaacga ctccgtgggc ggcctcggaa gccgcgtgga ccaccacgcc     660
   cacctcctcc agggaaagat cggggagggg ctcaagcgcg tcttcttgga cccgaggctc     720
   aaggaccggg tcttcatcct ggaaaccccc aggggaccgg aggaggacgc ctggaacctc     780
   cgggtcctca gggcctggct cgaggaggcc taa                                  813
<210> 12
   <211> 24
   <212> DNA
   <213> artificial
<220>
   <223> primer
<400> 12
   cgccagggtt ttcccagtca cgac 24
<210> 13
   <211> 24
   <212> DNA
   <213> artificial
<220>
   <223> primer
<400> 13
   agcggataac aatttcacac agga 24
<210> 14
   <211> 45
   <212> DNA
   <213> unknown
<220>
   <223> synthetic oligo
<400> 14
   ggcggcctcg aggcgaaacg ccgcaactgc tttccgggcg atacc 45
<210> 15
   <211> 45
   <212> DNA
   <213> unknown
<220>
   <223> synthetic oligo
<400> 15
   cggcacgcca tcaatctgca ccagaatgcg ggtatcgccc ggaaa 45
<210> 16
   <211> 45
   <212> DNA
   <213> unknown
<220>
   <223> synthetic oligo
<400> 16
   attgatggcg tgccgcagaa aattaccctg cgcgaactgt atgaa 45
<210> 17
   <211> 45
   <212> DNA
   <213> unknown
<220>
   <223> synthetic oligo
<400> 17
   catgttttca tagcgttcat cttcaaacag ttcatacagt tcgcg 45
<210> 18
   <211> 45
   <212> DNA
   <213> unknown
<220>
   <223> synthetic oligo
<400> 18
   cgctatgaaa acatggtgta tgtgcgcaaa aaaccgaaac gcgaa 45
<210> 19
   <211> 45
   <212> DNA
   <213> unknown
<220>
   <223> synthetic oligo
<400> 19
   ggtttccagg tcaatgctat acactttaat ttcgcgtttc ggttt 45
<210> 20
   <211> 46
   <212> DNA
   <213> unknown
<220>
   <223> synthetic oligo
<400> 20
   attgacctgg aaaccggcaa agtggtgctg accgatatta gaagat 46
<210> 21
   <211> 45
   <212> DNA
   <213> unknown
<220>
   <223> synthetic oligo
<400> 21
   cagatgatcg gtcgccggcg ctttaatcac atcttcaata tcggt 45
<210> 22
   <211> 45
   <212> DNA
   <213> unknown
<220>
   <223> synthetic oligo
<400> 22
   gcgaccgatc atctgattcg ctttgaactg gaagatggcc gcagc 45
<210> 23
   <211> 45
   <212> DNA
   <213> unknown
<220>
   <223> synthetic oligo
<400> 23
   cagcaccgga tgatccacgg tggtttcaaa gctgcggcca tcttc 45
<210> 24
   <211> 45
   <212> DNA
   <213> unknown
<220>
   <223> synthetic
<400> 24
   gatcatccgg tgctggtgta tgaaaacggc cgctttattg aaaaa 45
<210> 25
   <211> 45
   <212> DNA
   <213> unknown
<220>
   <223> synthetic oligo
<400> 25
   tttatcgcct tctttcactt caaacgcgcg tttttcaata aagcg 45
<210> 26
   <211> 45
   <212> DNA
   <213> unknown
<220>
   <223> synthetic oligo
<400> 26
   aaagaaggcg ataaagtgct ggtgagcgaa ctggaactgg tggaa 45
<210> 27
   <211> 45
   <212> DNA
   <213> unknown
<220>
   <223> synthetic oligo
<400> 27
   tttcgggtta tcctggctgc tgctgctctg ttccaccagt tccag 45
<210> 28
   <211> 45
   <212> DNA
   <213> unknown
<220>
   <223> synthetic oligo
<400> 28
   caggataacc cgaaaaacga aaacctgggc agcccggaac atgat 45
<210> 29
   <211> 45
   <212> DNA
   <213> unknown
<220>
   <223> synthetic oligo
<400> 29
   atatttaatg tttttaattt ccagcagctg atcatgttcc gggct 45
<210> 30
   <211> 48
   <212> DNA
   <213> unknown
<220>
   <223> synthetic oligo
<400> 30
   aaaaacatta aatatgtgcg cgcgaacgat gattttgtgt ttagcctg 48
<210> 31
   <211> 47
   <212> DNA
   <213> unknown
<220>
   <223> synthetic oligo
<400> 31
   ttaataatca cgttatgata ttttttcgcg ttcaggctaa acacaaa 47
<210> 32
   <211> 44
   <212> DNA
   <213> unknown
<220>
   <223> synthetic oligo
<400> 32
   taacgtgatt attaacgaaa acattgtgac ccatgcgtgc gatg 44
<210> 33
   <211> 47
   <212> DNA
   <213> unknown
<220>
   <223> synthetic oligo
<400> 33
   gccgccctgc agaccggtca gatcttcatc gccatcgcac gcatggg 47
<210> 34
   <211> 24
   <212> DNA
   <213> artificial
<220>
   <223> primer
<400> 34
   ggggcacctt ctccaactca tact 24
<210> 35
   <211> 23
   <212> DNA
   <213> artificial
<220>
   <223> primer
<400> 35
   cctcatttgg ggaggggtta tct 23
<210> 36
   <211> 24
   <212> DNA
   <213> artificial
<220>
   <223> primer
<400> 36
   cctgaaacaa ggttgtggca tagc 24
<210> 37
   <211> 25
   <212> DNA
   <213> artificial
<220>
   <223> primer
<400> 37
   gagtgagttg aaagtgctcc ataca 25
<210> 38
   <211> 27
   <212> DNA
   <213> artificial
<220>
   <223> primer
<400> 38
   ctatttaaac tattccctgg tacatac 27
<210> 39
   <211> 18
   <212> DNA
   <213> artificial
<220>
   <223> primer
<400> 39
   ggagcgagag gtacacgt 18
<210> 40
   <211> 27
   <212> DNA
   <213> artificial
<220>
   <223> primer
<400> 40
   ctatttaaac tattccctgg tacatac 27
<210> 41
   <211> 20
   <212> DNA
   <213> artificial
<220>
   <223> primer
<400> 41
   gccccatgca tataagcatg 20
<210> 42
   <211> 3474
   <212> DNA
   <213> unknown
<220>
   <223> plasmid pNEB0.92U
<400> 42
   gacgaaaggg cctcgtgata cgcctatttt tataggttaa tgtcatgata ataatggttt 60
   cttagacgtc aggtggcact tttcggggaa atgtgcgcgg aacccctatt tgtttatttt 120
   tctaaataca ttcaaatatg tatccgctca tgagacaata accctgataa atgcttcaat 180
   aatattgaaa aaggaagagt atgagtattc aacatttccg tgtcgccctt attccctttt 240
   ttgcggcatt ttgccttcct gtttttgctc acccagaaac gctggtgaaa gtaaaagatg 300
   ctgaagatca gttgggtgca cgagtgggtt acatcgaact ggatctcaac agcggtaaga 360
   tccttgagag ttttcgcccc gaagaacgtt ttccaatgat gagcactttt aaagttctgc 420
   tatgtggcgc ggtattatcc cgtattgacg ccgggcaaga gcaactcggt cgccgcatac 480
   actattctca gaatgacttg gttgagtact caccagtcac agaaaagcat cttacggatg 540
   gcatgacagt aagagaatta tgcagtgctg ccataaccat gagtgataac actgcggcca 600
   acttacttct gacaacgatc ggaggaccga aggagctaac cgcttttttg cacaacatgg 660
   gggatcatgt aactcgcctt gatcgttggg aaccggagct gaatgaagcc ataccaaacg 720
   acgagcgtga caccacgatg cctgtagcaa tggcaacaac gttgcgcaaa ctattaactg 780
   gcgaactact tactctagct tcccggcaac aattaataga ctggatggag gcggataaag 840
   ttgcaggacc acttctgcgc tcggcccttc cggctggctg gtttattgct gataaatctg 900
   gagccggtga gcgtgggtct cgcggtatca ttgcagcact ggggccagat ggtaagccct 960
   cccgtatcgt agttatctac acgacgggga gtcaggcaac tatggatgaa cgaaatagac 1020
   agatcgctga gataggtgcc tcactgatta agcattggta actgtcagac caagtttact 1080
   catatatact ttagattgat ttaaaacttc atttttaatt taaaaggatc taggtgaaga 1140
   tcctttttga taatctcatg accaaaatcc cttaacgtga gttttcgttc cactgagcgt 1200
   cagaccccgt agaaaagatc aaaggatctt cttgagatcc tttttttctg cgcgtaatct 1260
   gctgcttgca aacaaaaaaa ccaccgctac cagcggtggt ttgtttgccg gatcaagagc 1320
   taccaactct ttttccgaag gtaactggct tcagcagagc gcagatacca aatactgttc 1380
   ttctagtgta gccgtagtta ggccaccact tcaagaactc tgtagcaccg cctacatacc 1440
   tcgctctgct aatcctgtta ccagtggctg ctgccagtgg cgataagtcg tgtcttaccg 1500
   ggttggactc aagacgatag ttaccggata aggcgcagcg gtcgggctga acggggggtt 1560
   cgtgcacaca gcccagcttg gagcgaacga cctacaccga actgagatac ctacagcgtg 1620
   agctatgaga aagcgccacg cttcccgaag ggagaaaggc ggacaggtat ccggtaagcg 1680
   gcagggtcgg aacaggagag cgcacgaggg agcttccagg gggaaacgcc tggtatcttt 1740
   atagtcctgt cgggtttcgc cacctctgac ttgagcgtcg atttttgtga tgctcgtcag 1800
   gggggcggag cctatggaaa aacgccagca acgcggcctt tttacggttc ctggcctttt 1860
   gctggccttt tgctcacatg ttctttcctg cgttatcccc tgattctgtg gataaccgta 1920
   ttaccgcctt tgagtgagct gataccgctc gccgcagccg aacgaccgag cgcagcgagt 1980
   cagtgagcga ggaagcggaa gagcgcccaa tacgcaaacc gcctctcccc gcgcgttggc 2040
   cgattcatta atgcagctgg cacgacaggt ttcccgactg gaaagcgggc agtgagcgca 2100
   acgcaattaa tgtgagttag ctcactcatt aggcacccca ggctttacac tttatgcttc 2160
   cggctcgtat gttgtgtgga attgtgagcg gataacaatt tcacacagga aacagctatg 2220
   accatgatta cgccaagctt cctgcagggt ttaaacgctg aggagacata tggccgccga 2280
   gtctcagtta aaacgtgtga tcgaaacgct gcgccgtctg ggtattgaag aggtgctgaa 2340
   actggaacgt cgtgatcctc agtatcgcgc tgtttgcaat gtggtcaagc gtcatggcga 2400
   aactgtgggc agccgtttag ctatgttaaa cgccctgatt tcatatcgcc tgaccggtaa 2460
   gggtgaggag cattgggaat atttcggcaa atatttcagt cagttagaag tgattgatct 2520
   gtgccgtgat ttcttaaaat atattgagac cagcccgttc ctgaaaatcg gtgtcgaggc 2580
   gcgcaagaaa cgcgcgttaa aggcctgcga ctacgtccct aacttggaag acttgggcct 2640
   gaccctgcgt caattaagcc acatcgttgg tgcacgccgt gagcagaaga cgttggtctt 2700
   cacaatcaag atcctgaact atgcatatat gtgcagccgc ggtgttatcg cgtgttgccg 2760
   ttcgatattc caattcctgt ggattaccgt gttgcacgct tgacctggtg cgccggtctg 2820
   atcgatttcc cgccggagga ggccttgcgc cgctacgagg ctgtgcagaa aatctgggat 2880
   gccgtggcgc gcgaaactgg tattcctcca ttgcacttgg acaccctgtt atggttggcc 2940
   ggtcgcgcgg tgctgtatgg tgaaaacctg catggtgtgc cgaaagaggt catcgctctg 3000
   ttccaatggc gcggcggctg ccgtccgcct agcgagtaaa ccccctcagc ttaattaagg 3060
   cgcgcctgag ctcgaattca ctggccgtcg ttttacaacg tcgtgactgg gaaaaccctg 3120
   gcgttaccca acttaatcgc cttgcagcac atcccccttt cgccagctgg cgtaatagcg 3180
   aagaggcccg caccgatcgc ccttcccaac agttgcgcag cctgaatggc gaatggcgcc 3240
   tgatgcggta ttttctcctt acgcatctgt gcggtatttc acaccgcata tggtgcactc 3300
   tcagtacaat ctgctctgat gccgcatagt taagccagcc ccgacacccg ccaacacccg 3360
   ctgacgcgcc ctgacgggct tgtctgctcc cggcatccgc ttacagacaa gctgtgaccg 3420
   tctccgggag ctgcatgtgt cagaggtttt caccgtcatc accgaaacgc gcga 3474
<210> 43
   <211> 22
   <212> DNA
   <213> artificial
<220>
   <223> primer
<400> 43
   aaaatgccct ttggatctta aa 22
<210> 44
   <211> 18
   <212> DNA
   <213> artificial
<220>
   <223> primer
<400> 44
   actgctgtat cccgtggg 18

## Description

### BACKGROUND

Copying of polynucleotides, more particularly amplification, is commonly used in molecular biology for studying, for example, the properties of genes. Problems in copying arise when the polynucleotide is damaged in some way.

By way of illustration, U.S. Patent No. 5,035,996 describes a process for controlling contamination of polymerase chain reaction amplification reactions that uses the modified nucleotide, dUTP, in the amplification reaction. This process uses uracil DNA glycosylase (UDG) to eliminate those PCR products containing uracil to prevent contaminating subsequent PCR reactions, U.S. patent publication No. 2004-0067559 A1 also relies on modified bases in primer DNA prior to amplification and uses, for example, dUTP for incorporation into the amplicon. The amplicon can then be fragmented by adding, for example, UDG and Endonuclease (Endo) IV.

One amplification methodology referred to as hot start nucleic acid amplification has been used to lower mis-priming during polymerase chain reaction (PCR). In one type of hot start amplification, prevention of extension by the polymerase relies on the presence of a PCR primer with a blocked 3' terminus in the PCR reaction (see for example U.S. Publication No. 2003-0119150). The primer is unblocked by a thermostable 3'-5' exonuclease that is active at >37°C. Therefore, the polymerase will only extend the PCR primers once the exonuclease unblocks the 3' end at >37°C. Alternatively the *Taq* polymerase is blocked and then activated at amplification temperatures.

Barnes, W. M. Proc. Natl. Acad. Sci. USA 91:2216-2220 (1994) describes the use of Vent® polymerase and *Taq* polymerase as an improvement over the use of *Taq* polymerase only in amplification. Ghadessy et al. reported a mutant *Taq* polymerase, that is not halted by damaged or abasic sites (Ghadessy et al. Nature Biotechnol. 22(6):755-9 (2004)).

It has been reported that conventional amplification techniques are compromised if the DNA is substantially damaged (Di Bernardo et al. Nucl. Acids Res.30:e16 (2002)). Degradation and/or fragmentation of DNA resulting from exposure to the environment and microorganisms which contain DNA nucleases is a frequent problem in forensics, diagnostic tests and routine amplification and affects fidelity and yield of the amplification product. In addition, the problem of degraded DNA is also faced by researchers who are analyzing the DNA obtained from extinct or extremely rare organisms that have been stored, frozen or fossilized.

Fromenty, B., et al. Nucl. Acids Res. 28(11):e50 (2000) and International Publication No. WO/0151656 reported that treatment with Exonuclease (Exo) III improved yields of long PCR. However, Fromenty also reported decreased yields of amplicon for DNA<500 bp when Exo III was used. One of the problems associated with the use of Exo III is that it degrades template and primers.

Di Benardo et al. Null. Acids Res. 30(4):e16 (2002) described the use of T4 DNA ligase (T4 ligase) and an *E. coli* polymerase as a pretreatment to am.plify short regions of single-stranded DNA between cross-linked regions of double-stranded DNA.

Another approach to amplification of damaged DNA has been described in U.S. Publication No. 2003-0077581. Degraded nucleic acid was hybridized to non-degraded nucleic acid having a sequence homologous to the degraded nucleic acid. Regions of the degraded nucleic acid were then filled in with nucleotide precursors. The fragmented strands were then covalently linked using a polymerizing and/or ligating enzyme.

Preparations for improving amplification of damaged DNA can be obtained commercially from Sigma, St. Louis, MO, and Qbiogene, now MP Biomedicals, Irvine, CA. Although the compositions of these preparations are not stated, it is assumed that Exo III is contained in the preparation.

Others report the use of a combination of *E. coli* DNA Poll and T4 ligase for preamplification repair (Pusch, et al., Nucl, Acids Res. 26:857 (1998)). However, according to Pusch *et al.*, the pre-amplification product must be purified before initiation of amplification.

In addition to those mentioned above, reference may also be made to the following: US-A-5, 459, 039, which relates to method for mapping genetic mutations; Kayser, K., "Restorase ™": A novel DNA polymerase blend that repairs damaged DNA", Origins Sigma-Aldrich, 14, March 2004, 4-5; WO-A-2005/017173, which relates to methods and compositions for amplification of DNA; WO-A-2006/019784, which relates to methods for repairing degraded DNA; and WO-A-2006/047461, which relates to repair of nucleic acids for improved amplification.

Michael J. Moser et al. Nucleic Acids Research, vol.31, no. 17, 1 September 2003, pages 5048-5053 describes enzymatic repair of an expanded genetic information system.

Pingfang Liu et al. Nucleic Acids Research, vol.32, no. 15, 2004, pages 4503-4511 describes that DNA ligases ensure fidelity by interrogating minor groove contacts.

### SUMMARY

In an embodiment of the invention, a method is provided for enhancing at least one of fidelity and yield of a copied or amplified product by repairing a damaged polynucleotide where the method comprises: incubating the polynucleotide in the absence of Endonuclease VI (Endo VI) in a reaction mixture comprising a ligase, an NAD+ cofactor, a polymerase and at least one AP endonuclease, as recited in the claims. The polynucleotide may be derived from any natural or synthetic source including from preserved biological material, forensic evidence, ancient material of biological origin, and tissue biopsy material. Examples of damage to the polynucleotide can include at least one of a apurinic/apyrimidinic (AP) site, a chemically modified nucleotide, a nick, a gap and DNA-DNA, DNA-protein cross-links, DNA-RNA cross-links or RNA-RNA cross-links.

In one example of the method, the ligase is a thermostable ligase. *Taq* DNA ligase is an example of a thermostable ligase that uses NAD⁺ as a cofactor. *E. coli* DNA ligase is another example of a ligase where the cofactor is NAD⁺. Other components of the reaction mixture containing the ligase may include one or more of a T7 Endo I or mutant thereof, a polymerase and an AP endonuclease. Examples of a polymerase for use in the reaction mixture include a *Taq* polymerase, an *E. coli* polymerase, or an archaeal polymerase or mutant thereof. Examples of archeal polymerases or chimeric polymerases derived therefrom include one or more of a Pfu, Vent^{®}, Deep Vent^{®}, 9°North, KOD and Phusion™ polymerases and Pwo. Examples of other polymerases for use in the reaction mixture include members of the Y family of polymerases such as *E. coli* polIV, *E. coli* polV, human pol kappa, human pol eta, Sso Dpo4; Sac Dbh, Sce pol zeta, and human pol iota.

Examples of an endonuclease for use in the reaction mixture may include at least one of: at least one endonuclease from any of Phage T4, *E. coli,* human and *Thermus* species. The reaction mixture may further include one or more enzymes selected from the following: T4 pyrimidine dimer glycosylase, [fapy]-DNA glycosylase (Fpg), UvrA, UvrB, UvrC, UvrD, Cho, Endo V, Endo III, Endo VIII, UDG, lambda beta protein, RecA and Aag.

In an embodiment of the invention, improved yield is achieved after copying of the polynucleotide by means selected from the group consisting of: PCR amplification, helicase-dependent amplification, transcription-medicated amplification, strand-displacement amplification, rolling circle amplification and whole genome amplification. Where the polynucleotide is an RNA, the amplification may be RT-amplification. Where the polynucleotide is DNA, the reaction mixture may comprise 1-100 units of *E. coli* Endo IV, 0.05-0.25 units of *E. coli* PolI and 5-500 units of *Taq* ligase added to 1-1000 ng DNA in a reaction volume of 10-1000 µL.

Improved or enhanced yield is determined where less than about 50% of the concentration of the starting polynucleotide preparation provides at least a similar amount of copied product obtainable absent the reaction mixture. Preferably, the improvement should be reproducible meaning that about 75% or more of replicate samples will demonstrate the improvement. Moreover, optionally, an additional feature of the method is that it is capable of achieving an improved result at a single temperature of incubation.

In an embodiment of the invention, a method is provided where amplification of the polynucleotide is capable of producing an amplicon in a size range of 50 nucleotides to 100,000 nucleotides in a polymerase chain reaction.

In an embodiment of the invention, a kit is provided that comprises a ligase, a polymerase, and at least one AP endonuclease, not including Endo VI; the enzymes being formulated for addition to a polynucleotide preparation to enhance (as described above) copying of the polynucleotide and instructions for their use.

In an embodiment of the invention, a composition is provided that includes an effective amount of a ligase, a polymerase, and an AP endonuclease, not including Endo VI, for providing enhancement of at least one of yield and fidelity of a copied polynucleotide compared with a copied polynucleotide in the absence of the composition. In an example of the composition, the concentration of certain enzymes is as follows: 1-100 units of AP endonuclease, 0.05-5 units of the polymerase, and 5-500 units of the ligase in a volume 10-1000 µL for use in a pre-amplification mixture.

Examples of particular enzymes in the composition include an AP endonuclease, more particularly Type II AP endonuclease, more particularly, *E. coli* Endo IV. The polymerase is exemplified by *E. coli* polI. In addition, at least one of the following enzymes may be further included in the composition: T4 pyrimidine dimer glycosylase, [fapy]-DNA glycosylase (Fpg), UvrA, UvrB, UvrC, UvrD, Cho, Endo V, Endo III, Endo VIII, UDG, RecA, lambda beta protein and Aag.

In an embodiment of the invention, a method is provide for enhancing the yield of a copied or amplified polynucleotide, which includes: (a) obtaining at least a first pair and a second pair of primers wherein the second pair of primers is nested within the first set of primers when hybridized to the polynucleotide; (b) subjecting the polynucleotide to a composition described above; (c) amplifying the polynucleotide with the first set of primers; (d) amplifying the product of (c) with the second set of primers; and (e) obtaining an enhanced yield of amplified polynucleotide.

In an additional embodiment of the invention, a method is provided for cloning a polynucleotide fragment that includes: repairing sequence errors in the polynucleotide fragment by means of a composition such as described above. This composition may have the advantageous property of blunt-ending the polynucleotide fragment, which eliminates a step in cloning a fragment into a vector.

In an additional embodiment of the invention, a method is provided for sequencing a polynucleotide, which includes contacting the polynucleotide with the composition described above prior to sequencing. The quality of the sequence results is generally improved with respect to sensitivity and to accuracy.

In an additional embodiment of the invention, a method for copying or amplifying a fragmented DNA is provided which involves (a) contacting the fragmented DNA with the composition above; (b) optionally adding a recombination competent protein; and c) amplifying or copying the fragmented DNA. Examples of recombination competent proteins are RecA and beta protein from, for example, lambda phage.

### BRIEF DESCRIPTION OF THE DRAWINGS

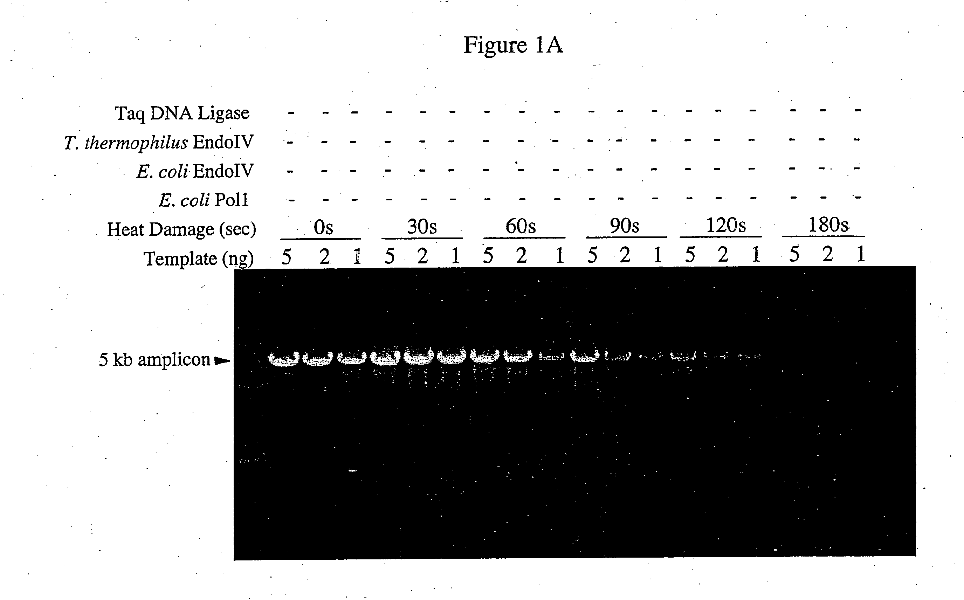

Figures 1A-1D show enhanced amplicon yield from heat-damaged lambda DNA after pre-incubation with specified enzymes.
Figure 1A shows DNA template damaged to differing extents by heat and the effect of this damage on amplification of a 5 kb segment of lambda DNA where 5 ng, 2 ng and 1 ng of heat-treated lambda DNA were amplified after prior damage by 99°C heat treatment for 0 sec, 30 sec, 60 sec, 90 sec, 120 sec or 180 sec. The damaged DNA was not subjected to enzyme treatment prior to amplification. The amount of amplification was determined after electrophoresis and was found to be substantially reduced by 120 sec heat treatment. The first lane on the gel contains 1 µg of a 2-log ladder size standard (NEB#N3200, New England Biolabs, Inc., Ipswich, MA).

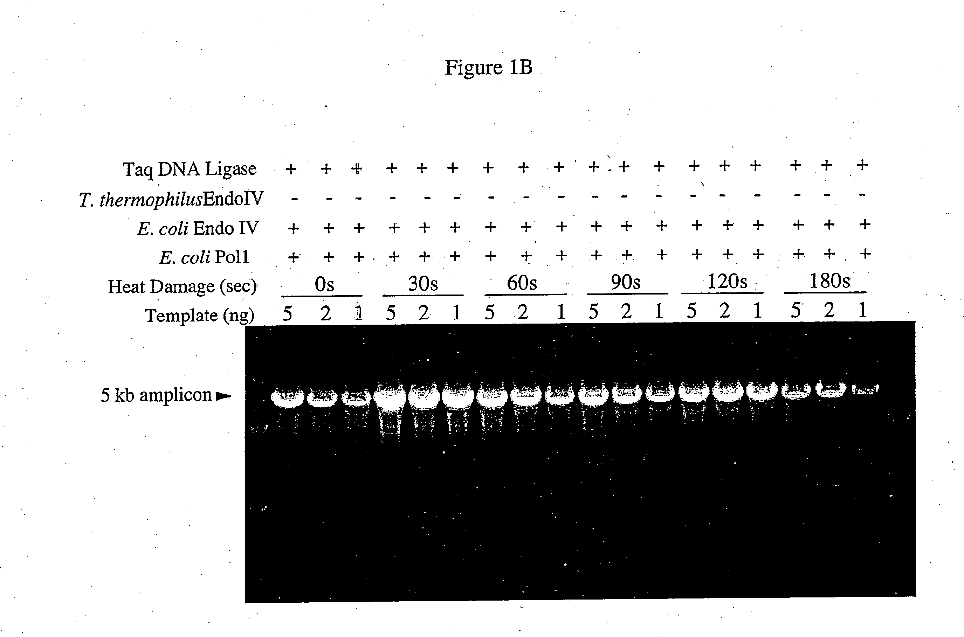

Figure 1B shows enhanced amplicon yields from heat-damaged lambda DNA using *Taq* ligase, *E. coli* Endo IV and *E. coli* Poll on amplification of a 5 kb segment of lambda DNA. DNA was heat damaged as described in Figure 1A but the damaged DNA was subjected to enzyme treatment prior to amplification. The results of amplification are shown after a 10-minute pretreatment reaction with *Taq* ligase, *E. coli* Endo IV and E. *coli* Poll. The amplicon yield was increased throughout but was especially noticeable with 120 sec and 180 sec heat damaged DNA. First and last lanes on the gel contain 1 µg of a 2-log ladder size standard (NEB#N3200, New England Biolabs, Inc., Ipswich, MA.

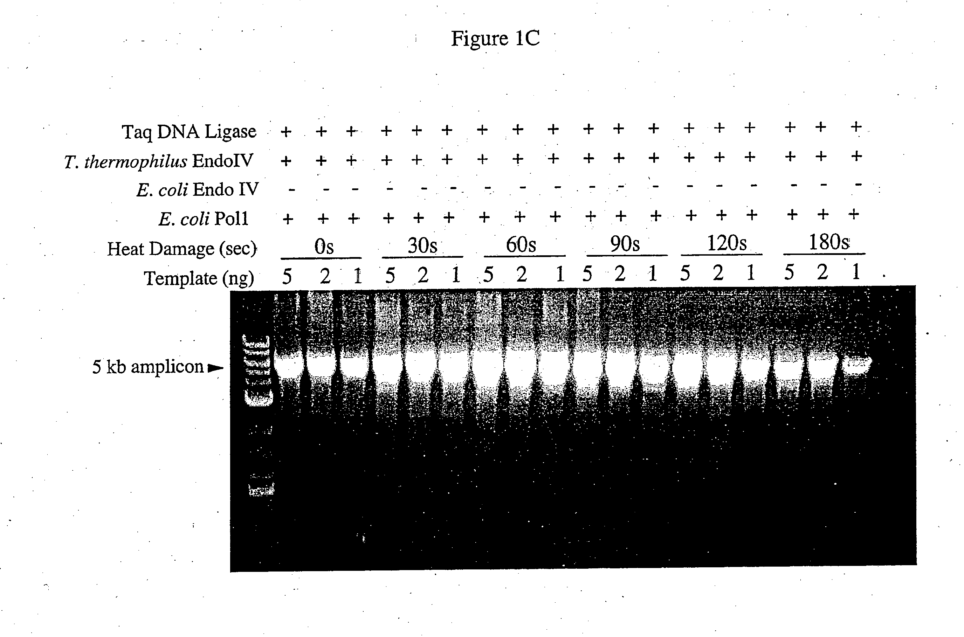

Figure 1C shows enhanced amplicon yields from heat-damaged lambda DNA using *Taq* ligase, *Tth* Endo IV and *E. coli* Poll. The amplification was performed according to Figure 1B but the enzyme treatment prior to amplification contained *Thermus thermophilus (Tth)* Endo IV in place of *E. coli* Endo IV. The results of amplification are shown after a 10-minute pretreatment reaction with *Thermus aquaticus (Taq)* ligase, *Tth* Endo IV and *E. coli* Poll. The amplicon yield was increased throughout but was especially noticeable with 120 sec and 180 sec heat-damaged DNA. Only the first lane contains the molecular weight marker ladder.

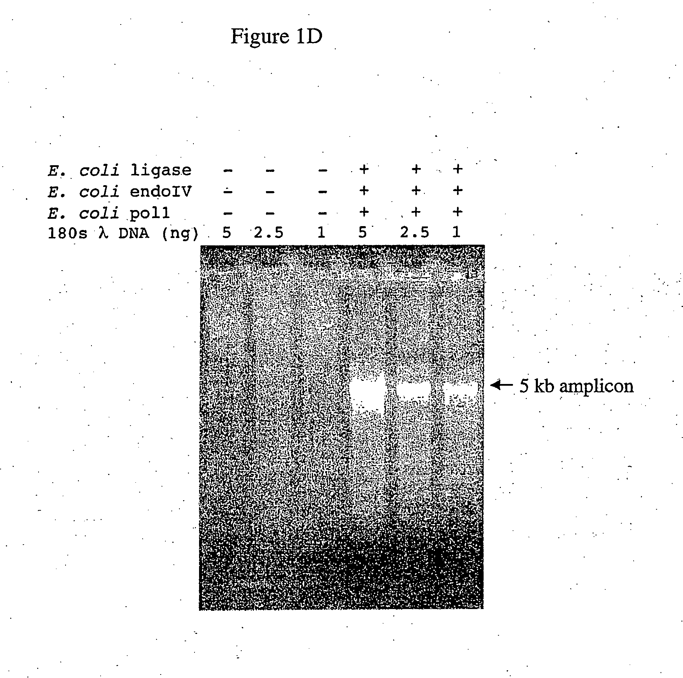

Figure 1D shows enhanced amplicon yields from heat-damaged lambda DNA using *E. coli* ligase, *E. coli* Endo IV and *E. coli* DNA polI. The amplification was performed according to Figure 1B but the enzyme treatment prior to amplification contained *E. coli* ligase in place of *Taq* ligase. The lambda DNA subjected to 99°C for 180 sec was used as a template. The amount of template DNA used is indicated above each lane. The yield of 5kb amplicon is enhanced for each of the template amounts by enzyme pretreatment.

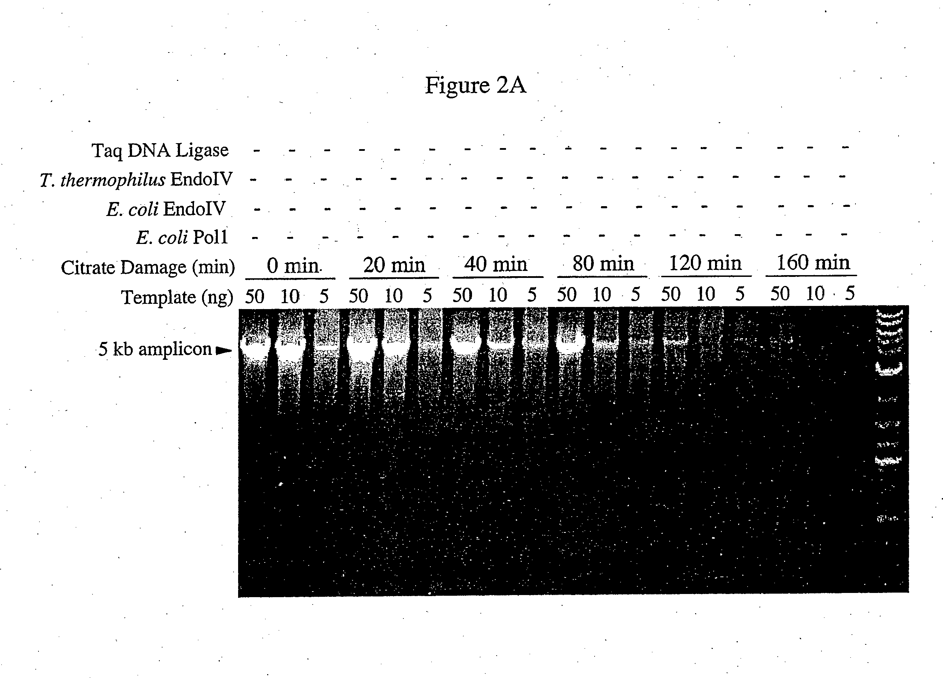

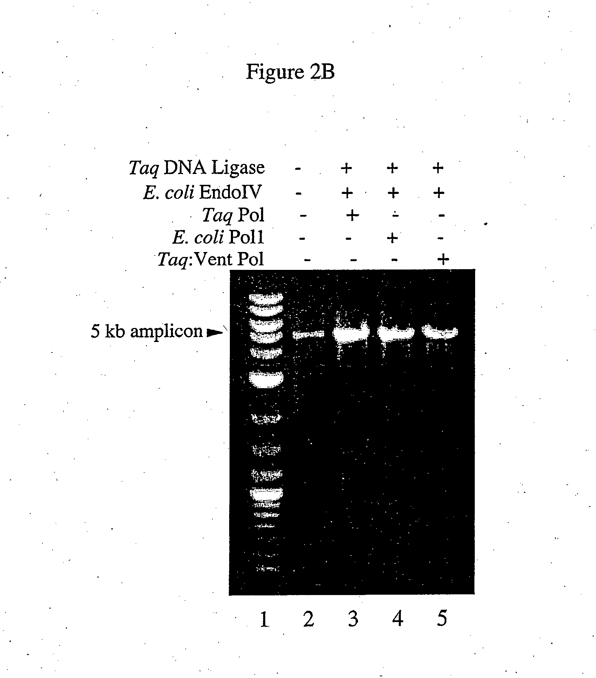

Figures 2A and 2B show the effect of citrate buffer pH 5 treatment of template DNA on amplicon yield.
Figure 2A shows the results of amplification of a 5 kb segment of lambda DNA where lambda DNA was heated to 70°C in citrate buffer pH 5 for 0, 20, 40, 80, 120, and 160 minutes. 50 ng, 10 ng and 5 ng of each citrate treated sample were amplified and the resulting products were visualized on a gel to determine the extent of amplification. The DNA was not treated with selected enzymes prior to amplification. The last lane on the right contains 1 µg of 2-log ladder.
Figure 2B shows the increase in yield of a 5 kb amplicon of lambda DNA regardless of which polymerase was used in the enzyme mixture. 120-minute citrate-damaged lambda DNA was treated with various enzymes prior to amplification.
   Lane 1: 1µg 2-log ladder (NEB# N3200, New England Biolabs, Inc., Ipswich, MA).
   Lane 2: no pretreatment.
   Lane 3: Pretreatment with *Taq* ligase, *Taq* DNA polymerase and *E. coli* Endo IV.
   Lane 4: Pretreatment with *Taq* ligase, *E. coli* PolI, and *E. coli* Endo IV.
   Lane 5: Pretreatment with *Taq* ligase, *Taq*:Vent^{®} DNA polymerase mix, and *E. coli* Endo IV.

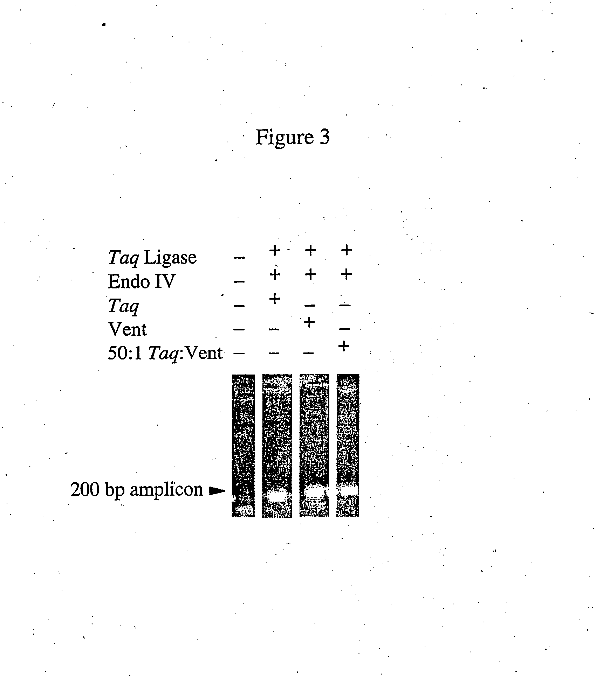

Figure 3 shows the results of amplification of a 200 bp segment of krill genome that has been extracted from an ethanol stored sample of krill and pretreated with an enzyme mixture containing one of various polymerases, a ligase and an AP endonuclease that enhances amplification yields.
   Lane 1: No pretreatment of krill DNA with enzymes.
   Lane 2: Pretreatment of krill DNA with *Taq* ligase, *E. coli* Endo IV, and *Taq* polymerase.
   Lane 3: Pretreatment of krill DNA with *Taq* ligase, *E. coli* Endo IV, and Vent^{®} polymerase.
   Lane 4: Pretreatment of krill DNA with *Taq* ligase, *E. coli* Endo IV, and 50:1 Taq:Vent^{®} polymerase.

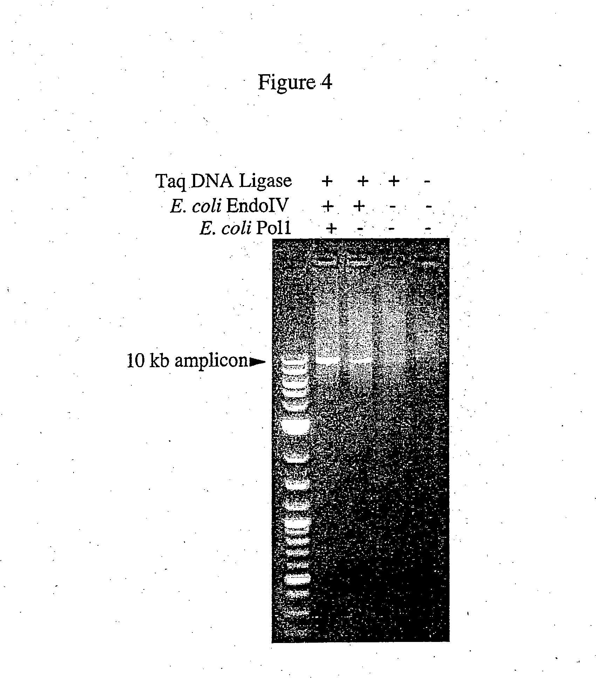

Figure 4 shows an increase in yield of a 10 kb amplicon from heat-damaged DNA. 180 sec heat-damaged DNA was pretreated with an enzyme mixture and then amplified.
   Lane 1: 1 µg of a 2-log ladder size standard (NEB#N3200, New England Biolabs, Inc., Ipswich, MA).
   Lane 2: Pre-treatment with *Taq* ligase, *E. coli* Endo IV, and *E. coli* PolI.
   Lane 3: Pre-treatment with *Taq* ligase and *E. coli* Endo IV.
   Lane 4: Pretreatment with *Taq* ligase.
   Lane 5: Control - untreated DNA.

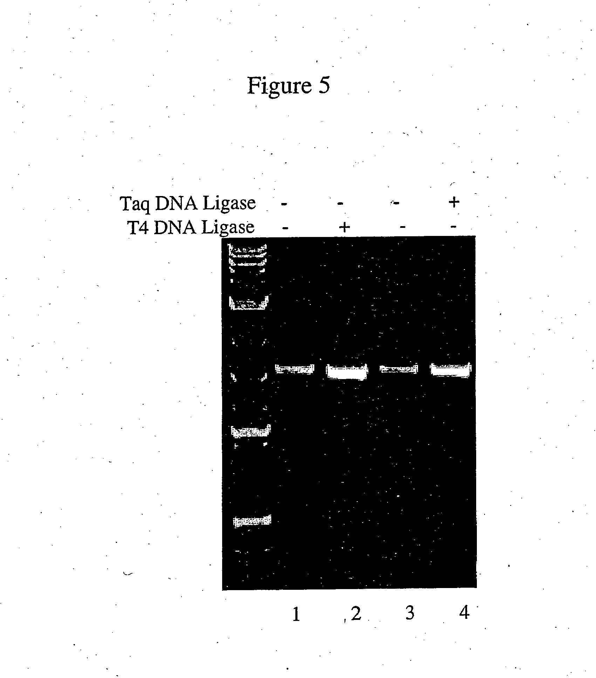

Figure 5 shows that ligase pretreatment increases amplicon yield from environmental DNA (soil sample extract).
   Lane 1: A 2-log ladder size standard (NEB# N3200, New England Biolabs, Inc., Ipswich, MA).
   Lane 1: No enzyme pretreatment.
   Lane 2: Pre-treatment with T4 ligase.
   Lane 3: No enzyme pre-treatment.
   Lane 4: Pretreatment with *Taq* ligase.
Figures 6A-1 - 6A-9 and 6B-1 - 6B-2: Blast P search at NCBI using *E. coli* DNA ligase (A) and T4 DNA ligase (B).
Figure 7: DNA sequence of *Tth* Endo IV (SEQ ID NO:11).
Figures 8A, 8B and 8C show the effect of UV light on amplicon yield using lambda DNA.

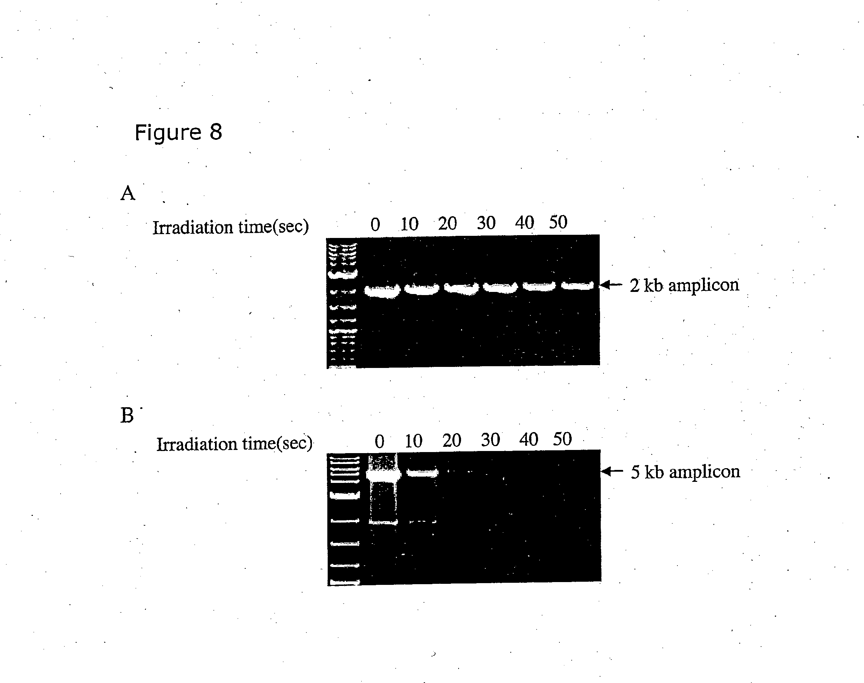

Figure 8A: Lambda DNA is subjected to UV irradiation for up to 50 sec and a slight reduction in yield of a 2 kb amplicon produced is shown.
Figure 8B: Lambda DNA is subjected to UV irradiation for up to 50 seconds and the reduction in yield of a 5kb amplicon is shown.

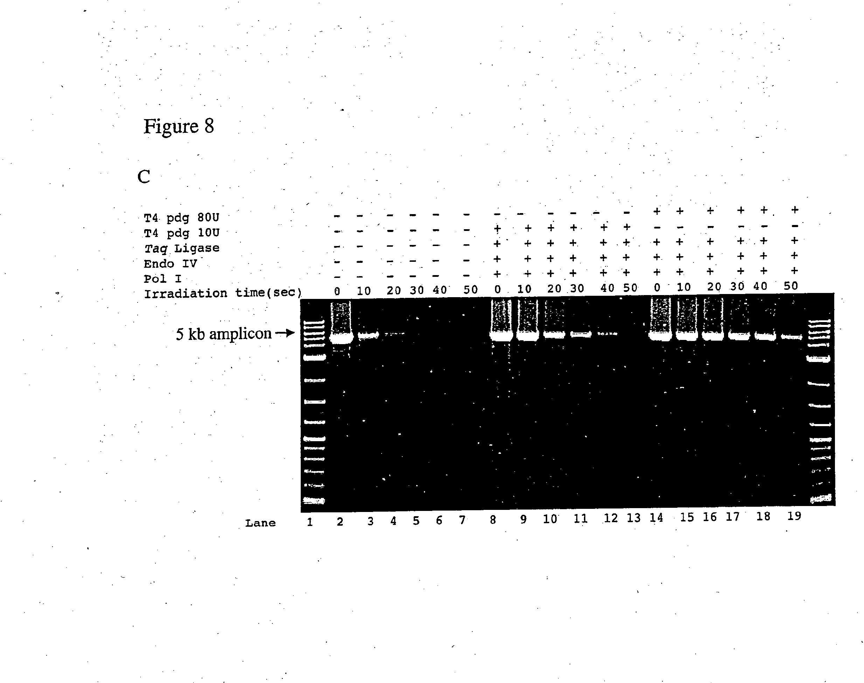

Figure 8C: The effect of various reaction mixtures added to lambda DNA on yield of a 5 kb amplicon after UV irradiation is shown.
   Lanes 2-7 are controls in the absence of a reaction mixture.
   Lanes 8-13 show the increased beneficial effect of adding ligase, polymerase and AP endonuclease plus 10 units of T4 pdg.
   Lanes 14-19 show the increased beneficial effect of adding ligase, polymerase and AP endonuclease plus 80 units of T4 pdg.
   Lanes 1 and 20: A 2-log ladder size standard (NEB#N3200, New England Biolabs, Inc., Ipswich, MA).

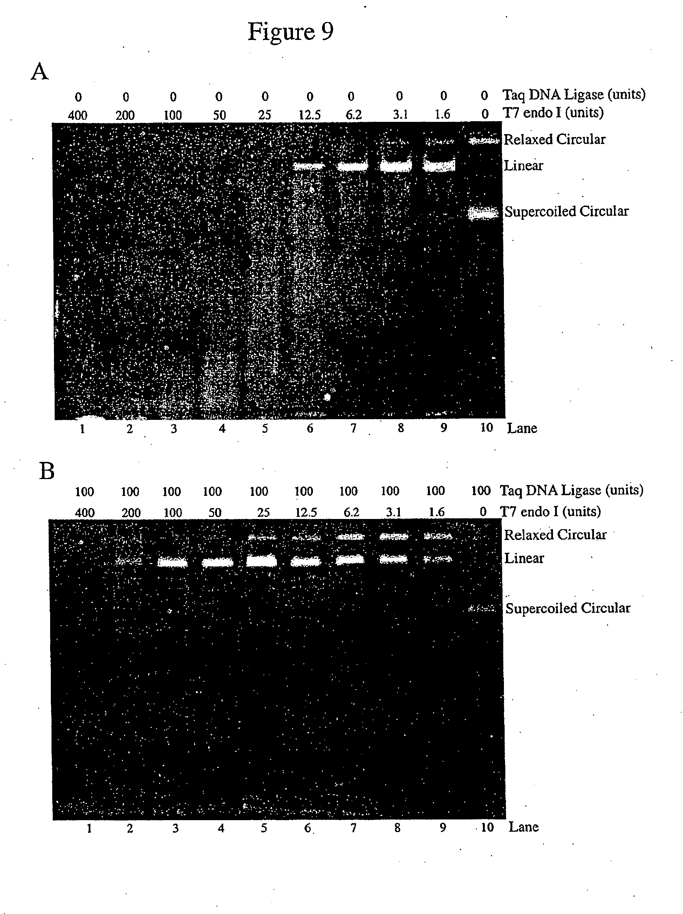

Figures 9A and 9B show that adding ligase to T7 Endo I expands the useful range of the EndoI: DNA ratio in which the product is not degraded. *Taq* ligase and T7 Endo I were added to supercoiled DNA in varying amounts as indicated for each lane.
Figure 9A is the control in which no *Taq* ligase has been added but increasing amounts of T7 Endo I are used. The supercoiled DNA is predominantly cleaved into fragments of various sizes with 12.5-25 units of T7 Endo I.
Figure 9B shows how the addition of 100 units of *Taq* ligase protects DNA from non-specific cleavage in the presence of T7 Endo I such that even at 200 units of T7 Endo I, there is a clear band corresponding to linear DNA not present in the absence of ligase.

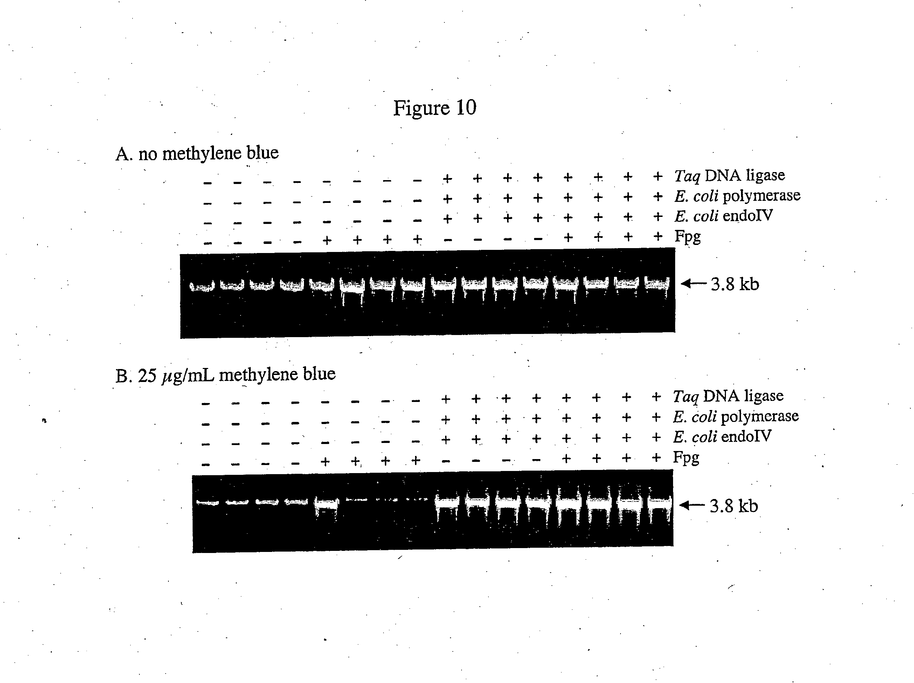

Figures 10A and 10B show the effect of repair enzyme treatment on amplicon yield from oxidatively damaged DNA or undamaged template.
Figure 10A shows that the addition of repair enzymes to an undamaged template, pWB407 has no effect on amplicon yield.
Figure 10B shows that the addition of Fpg to a damaged template, plasmid pWB407, which was previously incubated in the presence of methylene blue, gives inconsistent effects on yield. The addition of *Taq* ligase, *E. coli* DNA polymerase, and *E. coli* Endo IV in the presence or absence of Fpg consistently increases amplicon yield.

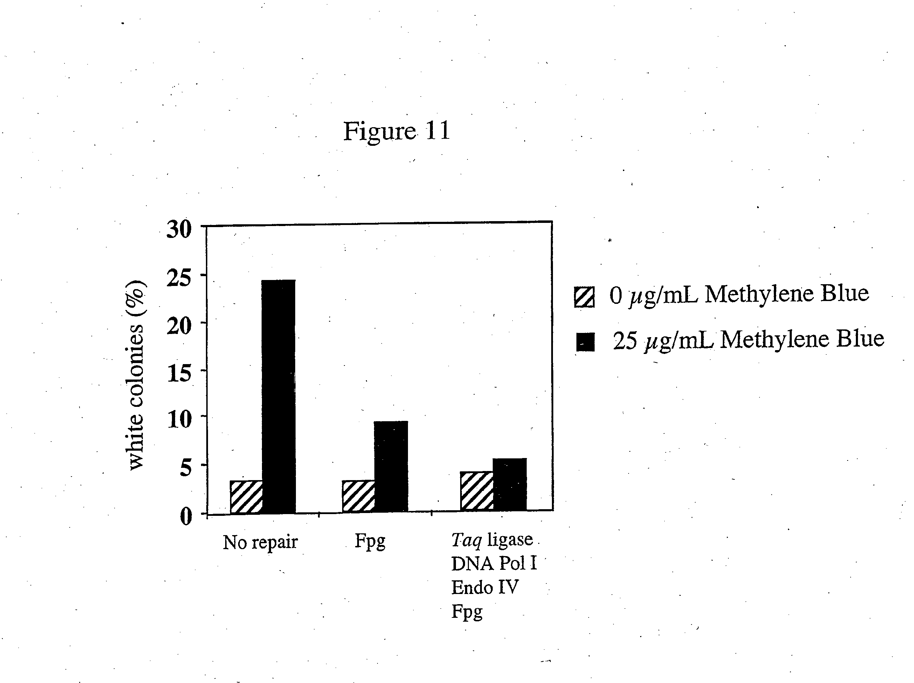

Figure 11 shows increased PCR reaction fidelity from damaged DNA after treatment with repair enzymes. Repair enzyme treatment of undamaged template, plasmid pWB407, prior to PCR has no significant effect on fidelity. Treatment of a damaged template, plasmid pWB407 incubated with methylene blue, with Fpg alone or also with *Taq* ligase, *E. coli* DNA polymerase I, and *E. coli* endonuclease increases the fidelity of PCR. The measure of fidelity is the number of white colonies verses the number of blue colonies after cloning a lacZ-containing amplicon as discussed below. The higher the percentage of white colonies the greater the error rate.

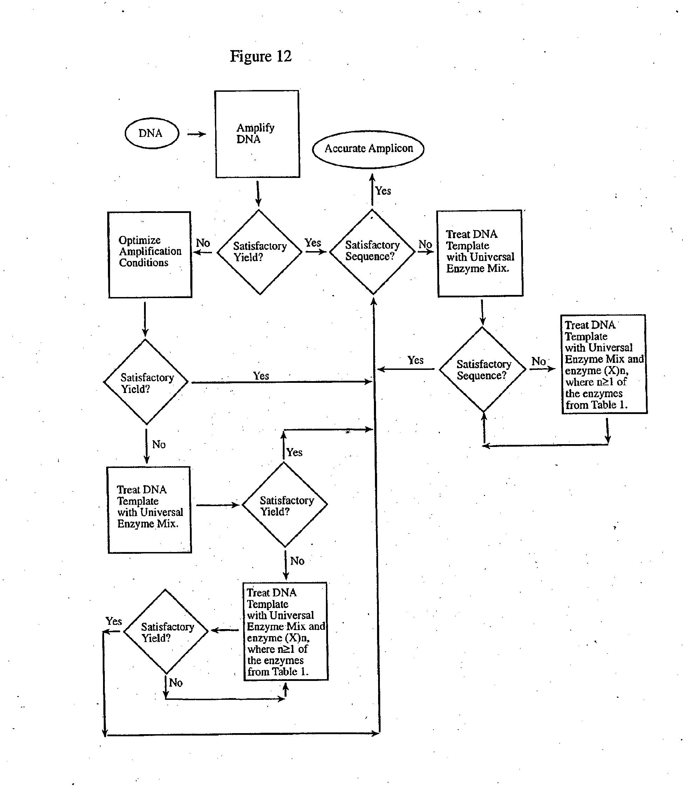

Figure 12 shows a flow diagram for treating damaged DNA or to increase at least of one of fidelity or yield.

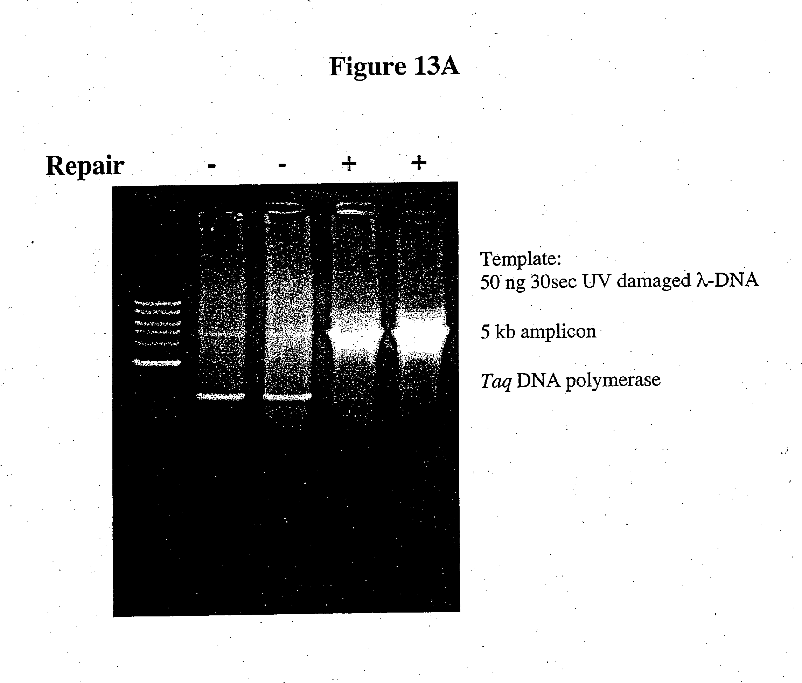

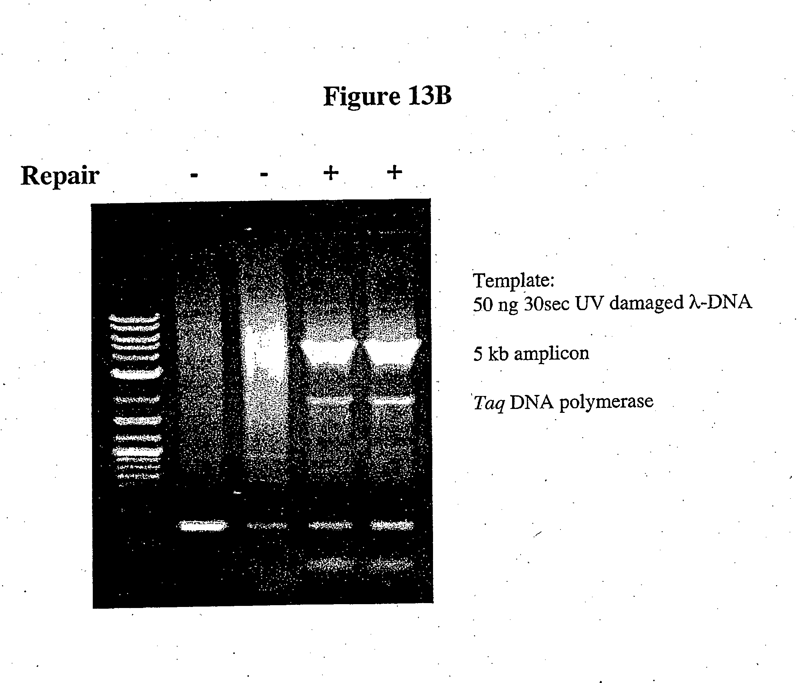

Figures 13A and 13B show how yield of amplicon is increased for a 5 kb fragment of 30s UV damaged DNA incubated for 15 minutes at room temperature or at 4°C overnight with a multi-enzyme repair mix.
Figure 13A: Room temperature incubation:
   Lane 1: 2-log ladder DNA molecular weight standard.
   Lanes 2 and 3: the two reactions incubated without the multi-enzyme repair mix at room temperature for 15 minutes.
   Lanes 4 and 5: the reactions incubated with the repair mix at room temperature for 15 minutes have the expected 5 kb amplicon.
Figure 13B: 4°C incubation:
   Lane 1: 2-log ladder DNA molecular weight standard.
   Lanes 2 and 3: the two reactions incubated without the multi-enzyme repair mix overnight at 4°C.
   Lanes 4 and 5: the reactions incubated with the repair mix overnight at 4°C have the expected 5 kb amplicon.

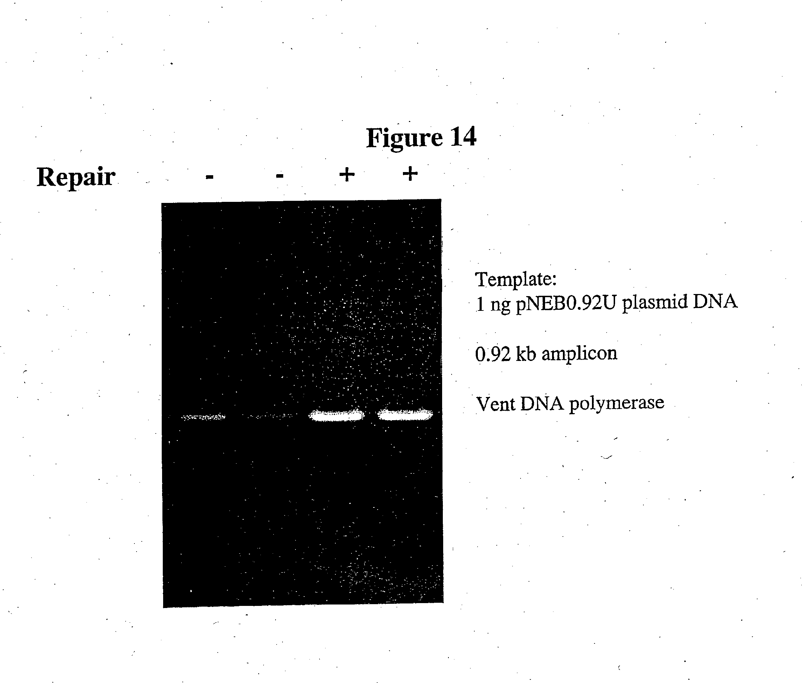

Figure 14 shows enhanced amplicon yield from a uracil-containing plasmid after treatment with a repair enzyme mix for 15 mins at room temperature and PCR amplification using an archaeal polymerase. Lanes 1 and 2 shows the product of PCR amplification of pNEB0.92U using vent DNA polymerase. There is a weakly visible band at 920 bp. Lanes 3 and 4 show the product of PCR amplification from pNEB0.92U treated with a repair enzyme mix.

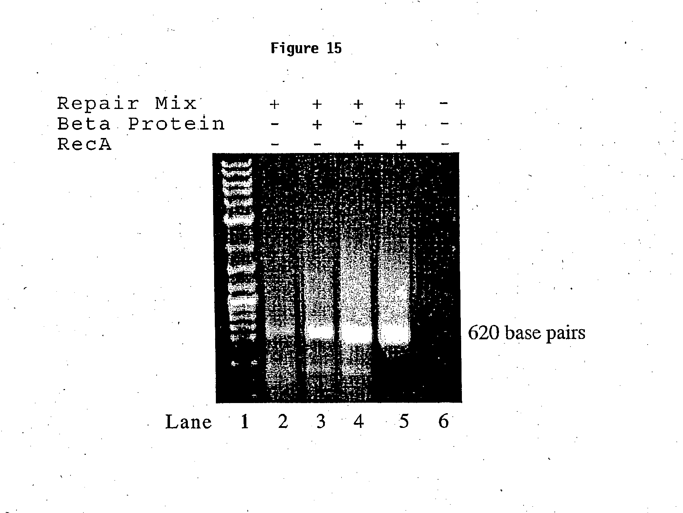

Figure 15 shows an agarose gel on which a band corresponding to an amplified DNA of 620 base pairs is identified. The 620 bp amplicon was obtained from 20 overlapping single strand oligonucleotides of 48 nucleotides or smaller.
   Lane 1: 2-log DNA molecular weight standards (NEB#N3200S, New England Biolabs, Inc., Ipswich, MA).
   Lane 2: 20 oligonucleotides incubated with 400 units *Taq* DNA ligase, 0.1 units E. coli PolI, 5 units T4 pdg, and 20 units EndoIV during the assembly step.
   Lane 3: 20 oligonucleotides incubated with 400 units *Taq* DNA ligase, 0.1 units *E. coli* PolI, 5 units T4 pdg, 20 units Endo IV, and lambda beta protein during the assembly step.
   Lane 4: 20 oligonucleotides incubated with 400 units *Taq* DNA ligase, 0.1 units *E. coli* PolI, 5 units T4 pdg, 20 units Endo IV, and *E. coli* RecA during the assembly step.
   Lane 5: 20 oligonucleotides incubated with 400 units *Taq* DNA ligase, 0.1 units *E. coli* PolI, 5 units T4 pdg, 20 units Endo IV, lambda beta protein and RecA during the assembly step.
   Lane 6: the control, 20 oligonucleotides with no added repair enzymes during the assembly step.

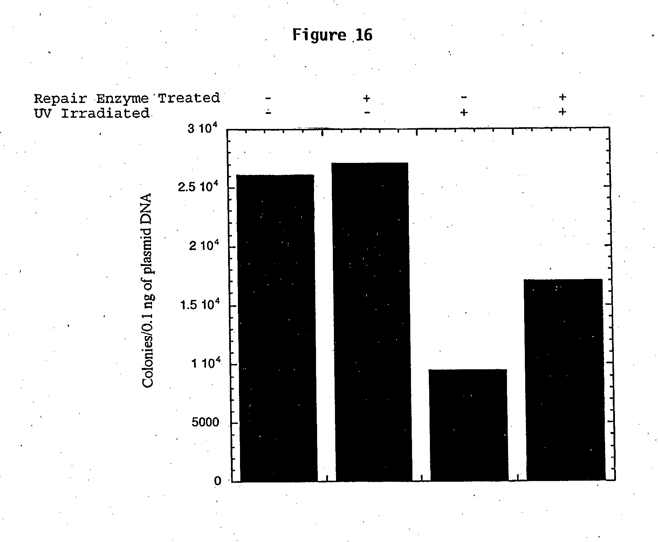

Figure 16 shows the effect of DNA repair treatment on nonirradiated (-) and irradiated DNA (+) as determined by the number of colonies obtained when the DNA containing a selection marker is used to transform cells.

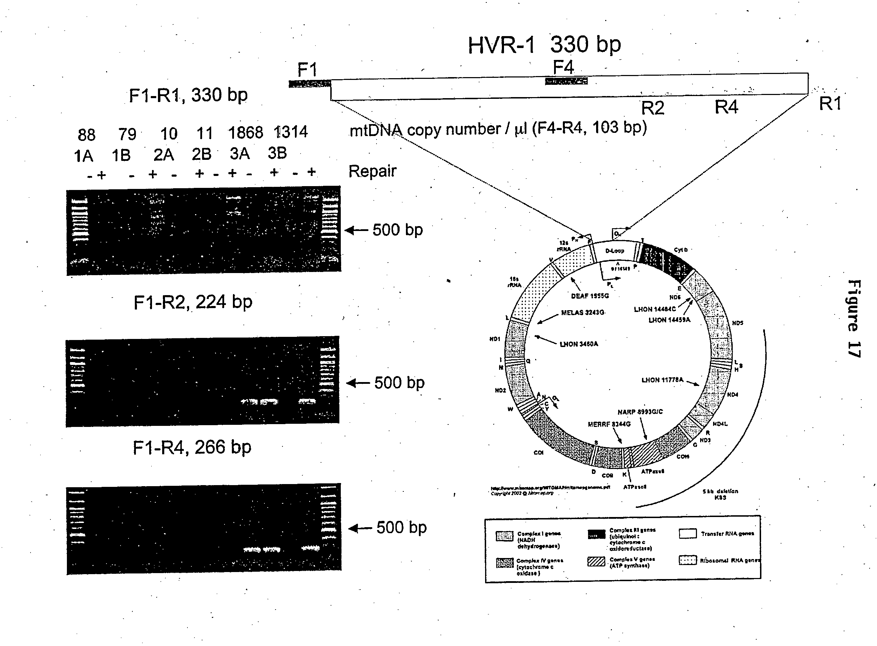

Figure 17 shows improved yields of amplicon from ancient cave bear DNA after 2 sets of amplification reactions using different nested primer pairs. The gene map shows the location of primer pairs F1-R1, F1-R2 and F1-R4. Above the gels, a set of numbers is provided (88, 79, 10, 11, 1868 and 1314) that represent the estimated amount of mitochondrial DNA in each sample. Lanes 3A and 3B contain the most DNA. +/- indicates whether a repair mix was used prior to the first amplification using F1-R1. In Lane 3B, a sharp band corresponding to repaired cave bear DNA was observed that was not present in the absence of repair.

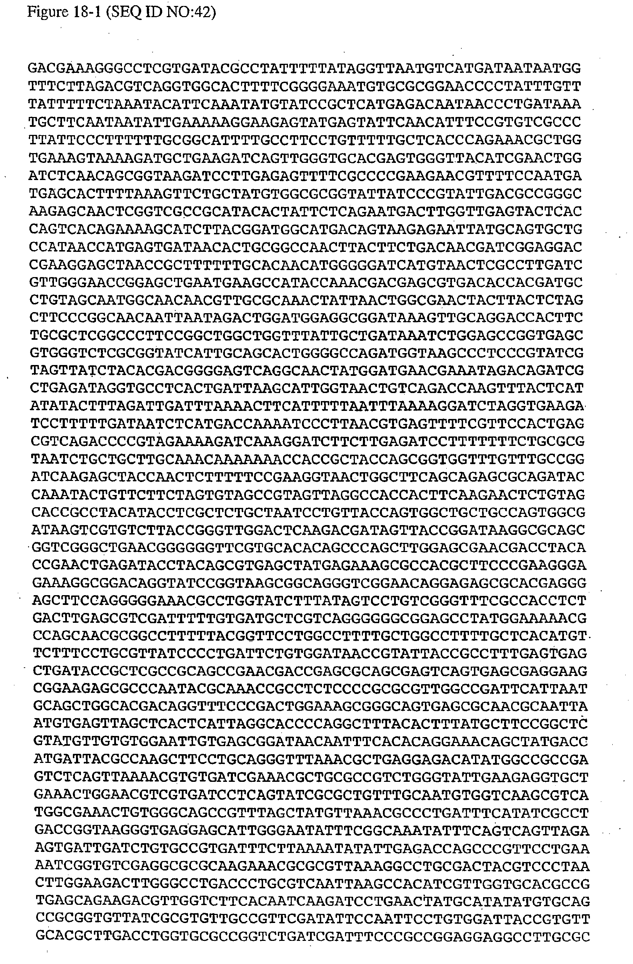

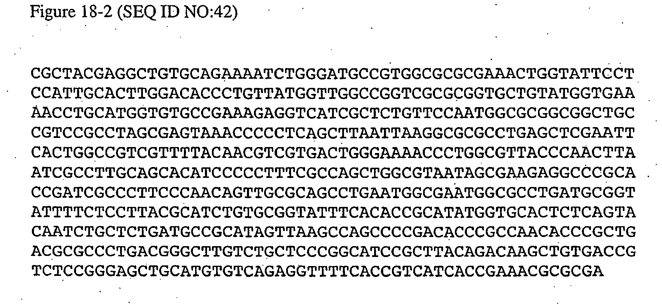

Figure 18 shows the DNA sequence for plasmid pNEB0.92U (SEQ ID NO:42).

### DETAILED DESCRIPTION OF EMBODIMENTS

Embodiments of the invention provide improvements in the method for copying or more specifically amplifying polynucleotides. Enhancing at least one of yield and fidelity of copied or amplified polynucleotides is associated with obtaining a detectable yield of polynucleotides after copying or amplification of 50% of the polynucleotides needed in the absence of a reaction mixture containing selected repair enzymes as exemplified in present embodiments. 'Fidelity' relates to copying in which the error rate is less than would otherwise be the case in the absence of a reaction mixture containing selected repair enzymes as exemplified in present embodiments.

The inventive method utilizes a reaction mixture containing enzymes. If multiple types of damage are suspected, then a combination of enzymes can be constructed as illustrated by the specific cocktails used in the examples. If the damage to the polynucleotide is known, then a preferred cocktail of enzymes may be selected as illustrated in the examples. If the nature of the damage is unrecognized, a universal mixture of enzymes can be employed. In general, adding enzymes together in one step does not preclude adding enzymes sequentially.

Where polynucleotide copying leads to polymerase-dependent amplification, short amplicons that are less than about 500 bases in length (as short as 100 base pairs) or long amplicons that are greater than 500 bases or as much as about 50 kb may be amplified (for PCR, RT-PCR and qPCR amplification). Other types of amplification can produce amplicons having a wide range of sizes. For example, polynucleotides having a size as small as 100 bases or as large as a whole genome (3 billion bases for humans). Examples of such amplification methodologies include: whole genome amplification, rolling circle amplification (RCA) and helicase-dependent amplification (HDA). Other types of polynucleotide synthesis include primer extension reactions such as sequencing reactions. Embodiments of the methods have wide utility in molecular biology research and in solving problems in applied biology, including for example, analyzing fragmented and damaged DNA such as found in forensic analysis, in biological archeology in which it is desirable to analyze DNA from ancient sources, for taxonomy where it is desirable to analyze DNA from environmental samples such as required for the Barcode of Life Project, and for diagnostic assays including tissue biopsies to determine a disease susceptibility or status. Other uses include: high-fidelity sequencing, gene assembly, fragment analysis and one step copying and ligation for cloning.

### Source and Extent of Damage

Most polynucleotides that are isolated or *in vitro* replicated polynucleotides are damaged to some extent. Damage of a polynucleotide may result from chemical modification of individual nucleotides or disruption of the polynucleotide backbone. Polynucleotides experience damage from diverse sources such as chemicals including formaldehyde and ethanol, environmental factors, temperature extremes, oxidation, dessication and ultra violet light. Various types of damage include: (a) apurinic or apyrimidinic damage caused for example by heat, storage of polynucleotides in ethanol, and exposure to factors in the environment such as H₂O or extremes of pH; (b) modification of individual nucleotides, caused for example by deamination, alkylation, and oxidation; (c) nicks and gaps caused for example by heat, storage of polynucleotide in ethanol, and exposure to factors in the environment such as H₂O or extremes of pH; (d) cross-linking caused for example, by formaldehyde, light or environmental factors; (e) mismatched DNA caused by for example misincorporation of a nucleotide by a polymerase; and (f) fragmentation of DNA.

Damage is more severe in preserved tissues, dried specimens or polynucleotides that are exposed to the environment. Damage can occur as a result of the age of the sample or its source or its preparation. In addition, damage can occur during the application of a methodology for polynucleotide synthesis such as occurs during PCR amplification, which involves a high temperature step. Hence most polynucleotides are damaged to some extent. This damage has a greater influence when longer amplicons are analyzed since the likelihood of encountering damage during amplification is increased.

Polynucleotides can sustain damage in a variety of ways. Different polynucleotide preparations experience different types of damage depending upon, for example, the storage or handling of the polynucleotide preparation *in vitro*, how prokaryotic cells, archaea or eukaryotic cells containing the polynucleotides are stored and the characteristics of the cells from which the polynucleotides are extracted. Synthetic polynucleotides can sustain damage during chemical synthesis.

The term "polynucleotide" refers in particular to double-stranded DNA, double-stranded RNA, hybrid DNA/RNA duplex, single-stranded DNA and single-stranded RNA.

A "repair enzyme" refers in particular to a psychrophilic, mesophilic or thermophilic enzyme that participates in the process of repair of a polynucleotide. For example, a repair enzyme may induce breakage of the polynucleotide at a bond, thereby facilitating removal of damaged regions of the polynucleotide or removal of single nucleotides. Enzymes with a synthetic role such as ligases and polymerases are also repair enzymes. In embodiments of the invention, one or more selected repair enzymes are provided in a reaction mixture. The damaged DNA is subjected to the reaction mixture so as to enhance copying and/or amplification of DNA. "Enhancing" refers to obtaining an improved ratio of copied or amplified product to starting material compared to the ratio observed in the absence of the repair mixture.

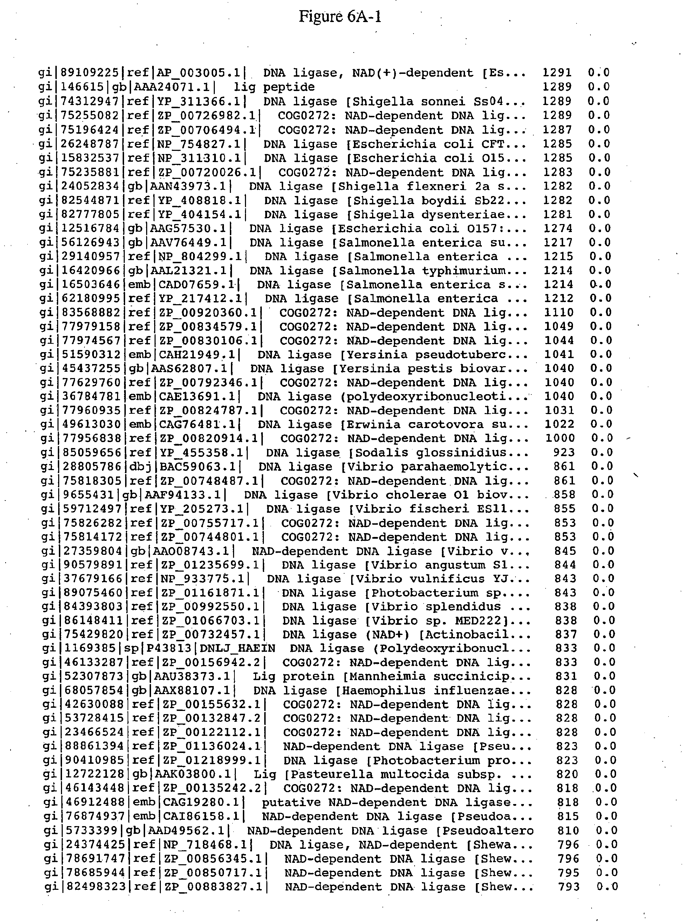

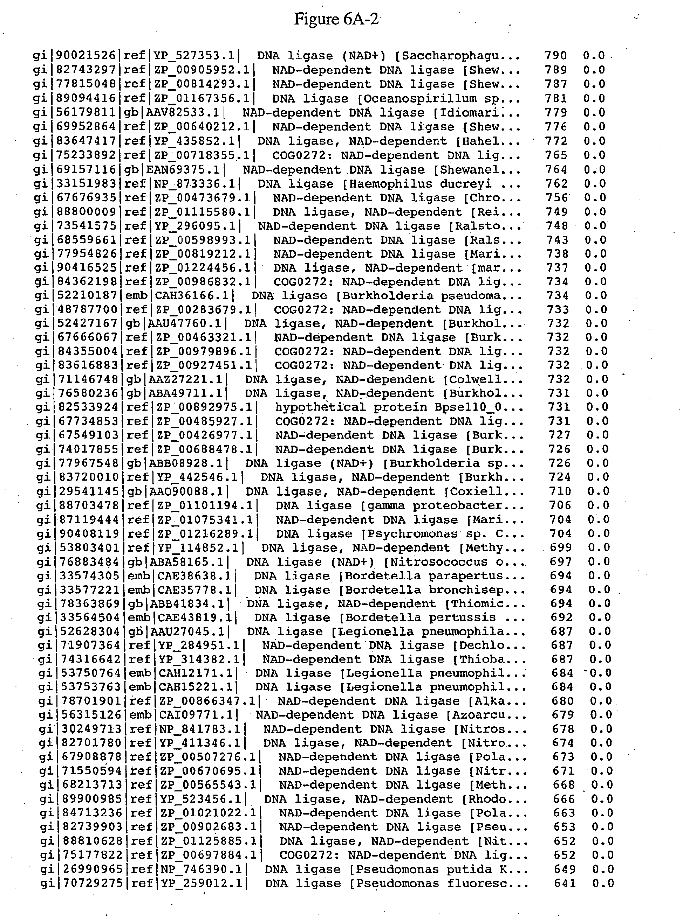

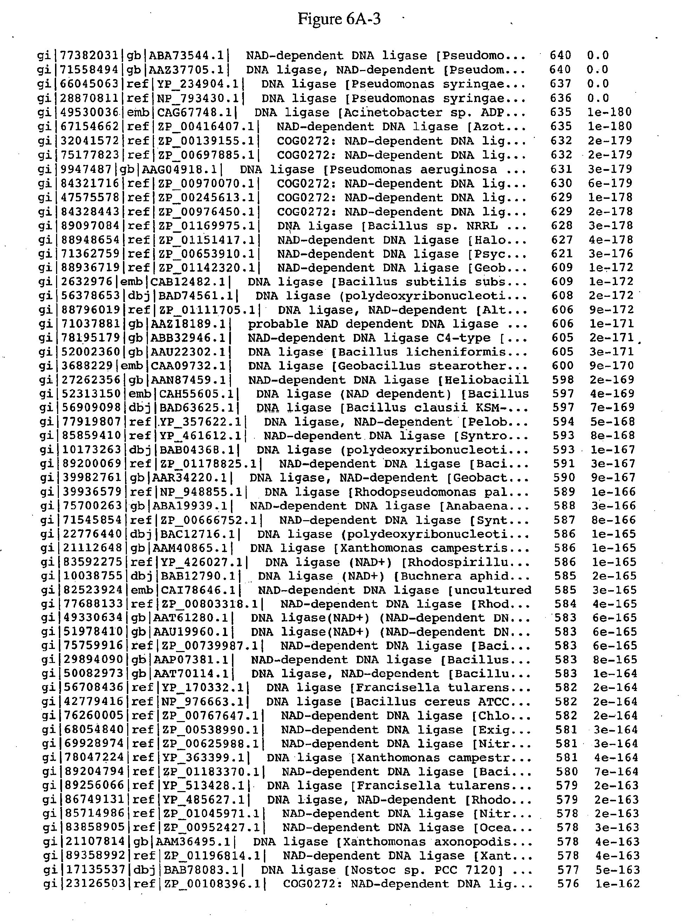

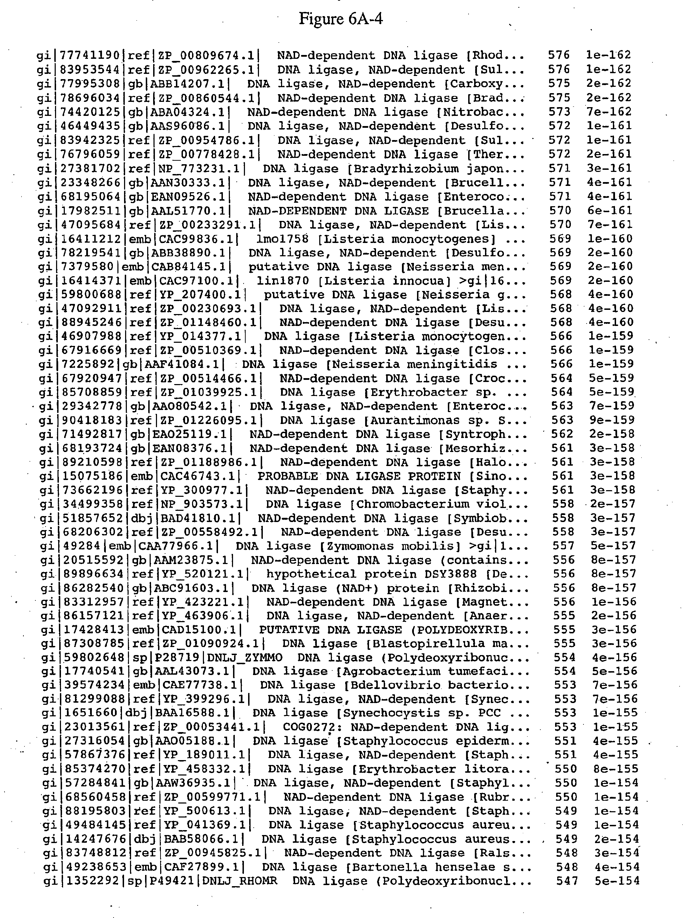

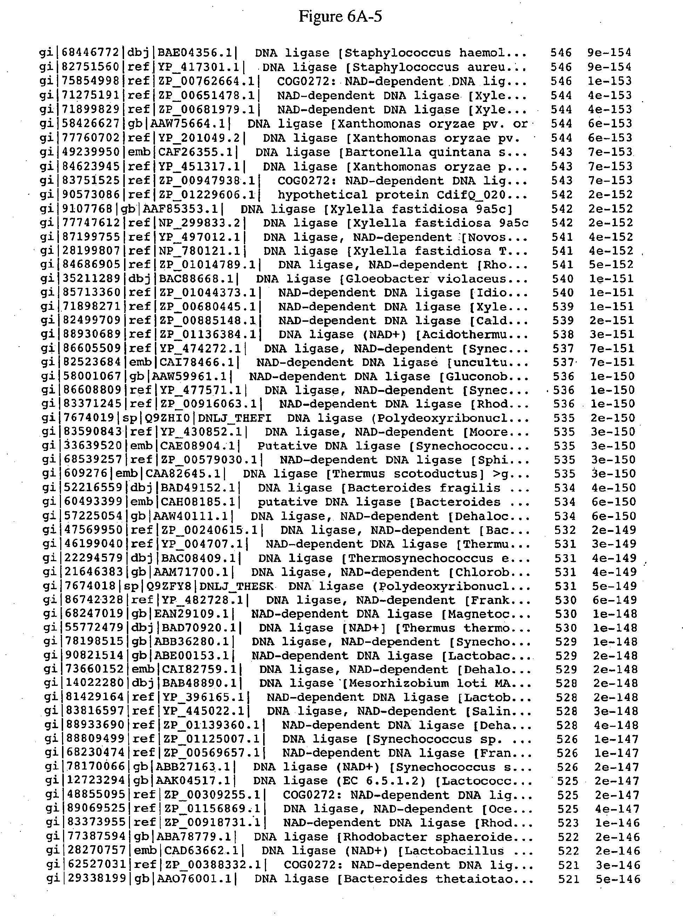

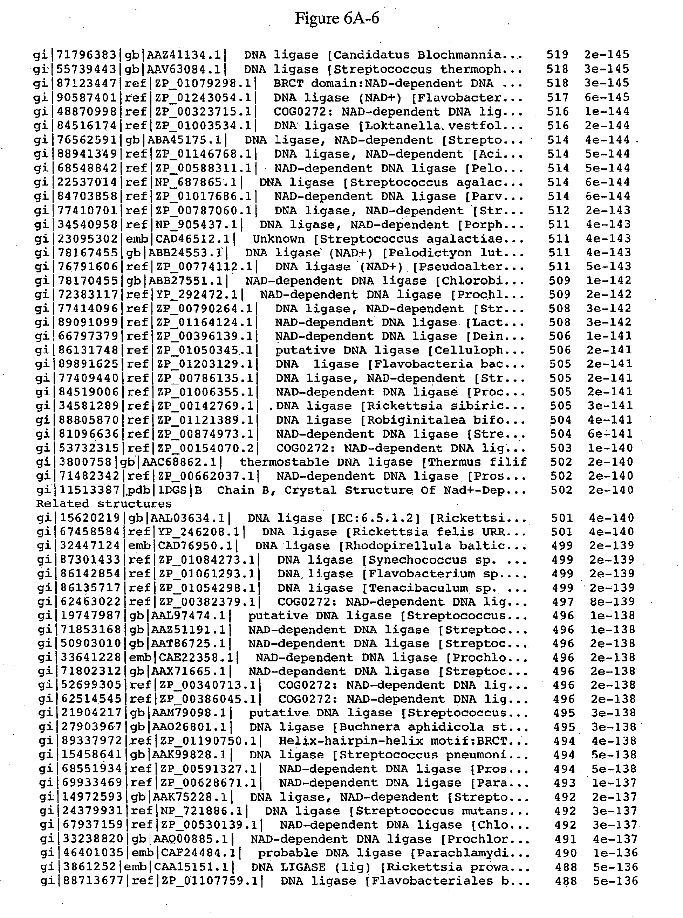

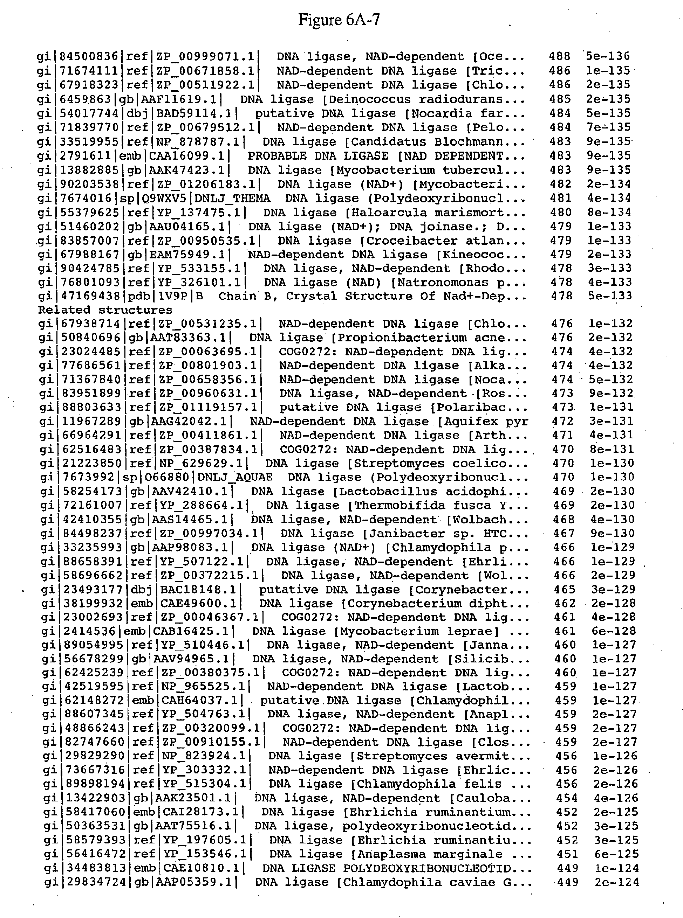

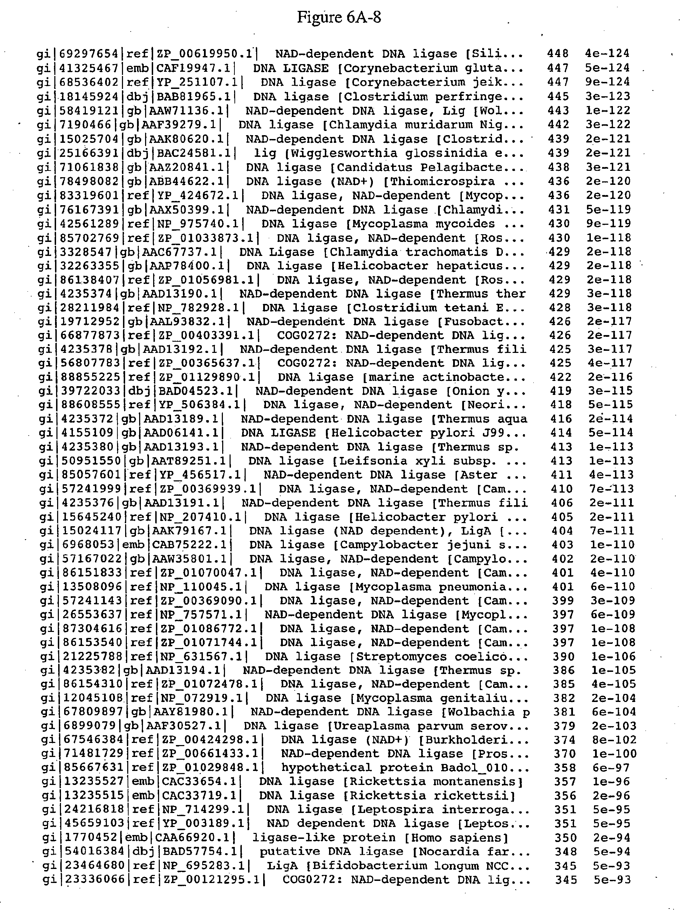

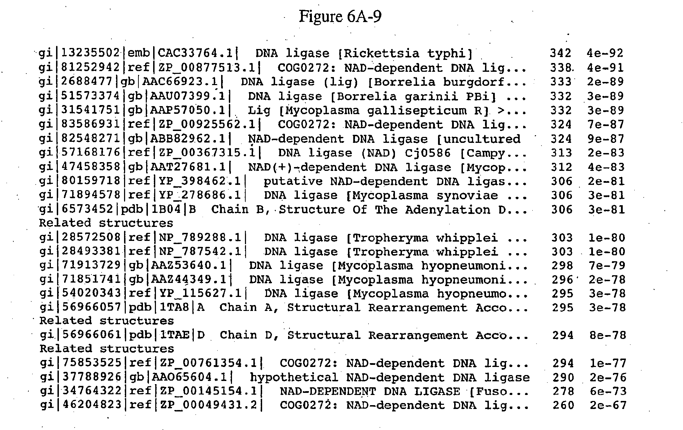

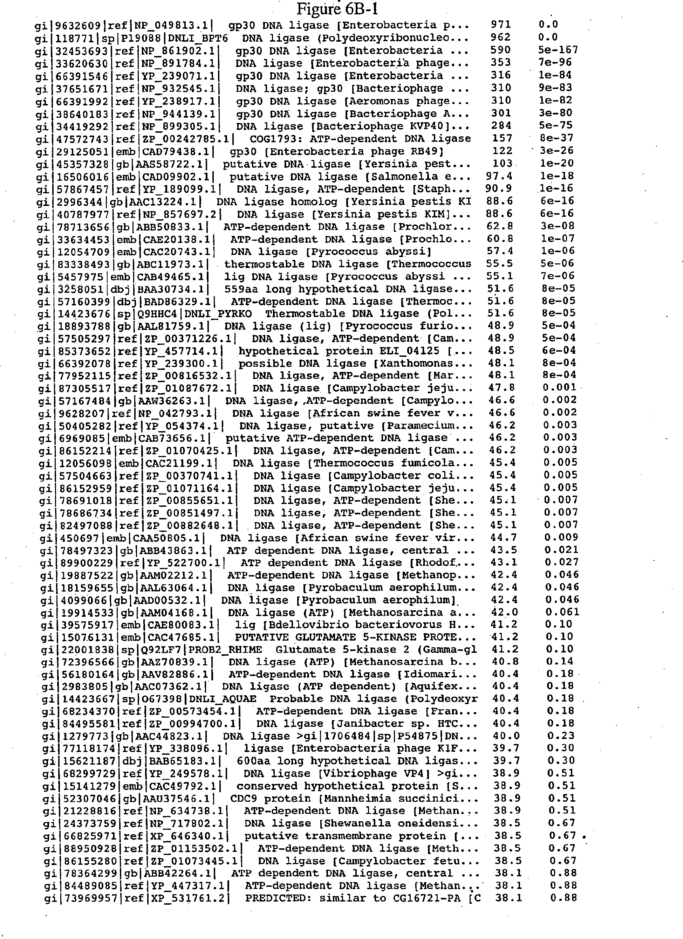

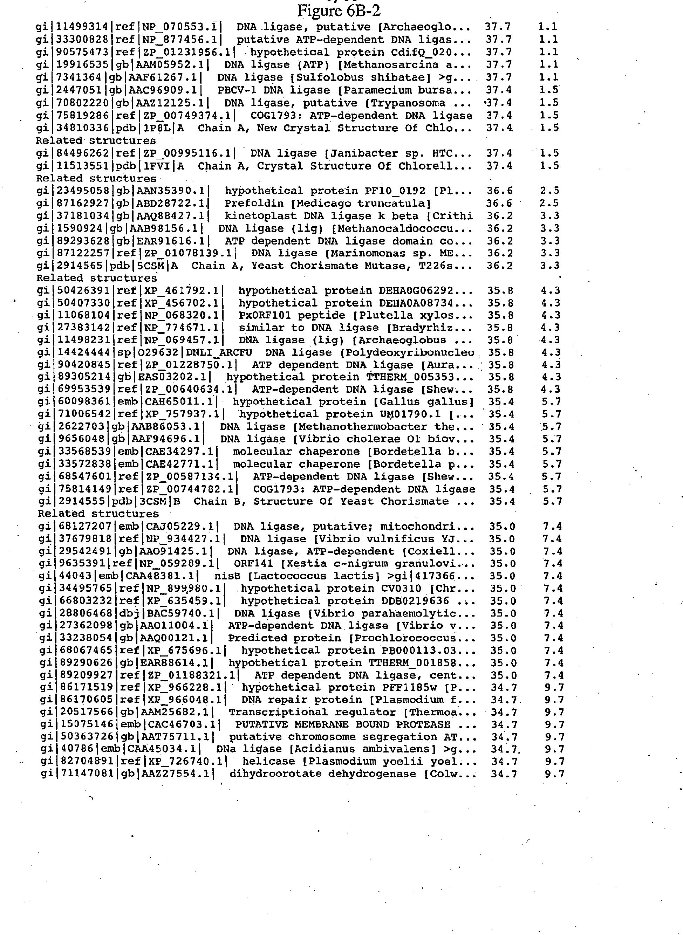

DNA repair enzymes are described in the scientific literature, for example, see Wood, R.D., et al. Mutat. Res. 577(1-2):275-83 (2005) and Eisen, J.A. and Hanawalt, P.C. Mutat. Res. 435(3):171-213 (1999). A list of human repair enzymes is provided in Table 1 below. Although not described in Table 1, the homologs of the listed enzymes and other functionally related enzymes are included in the description of repair enzymes. Any of the above enzymes may be naturally occurring, recombinant or synthetic. Any of the enzymes may be a native or *in vitro*-created chimeric protein with several activities. The methods of searching the databases to identify related enzymes that share conserved sequence motifs and have similar enzyme activity are known to a person of ordinary skill in the art. For example, the NCBI web site (www.ncbi.com) provides a conserved domain database. If, for example, the database is searched for homologs of Endo IV, 74 sequence matches are recovered. (Also see Figures 6A-1 - 6A-9 and 6B-1 - 6B-2 for ligases.)

A "polynucleotide cleavage enzyme" used in enzyme mixtures for repairing damaged DNA refers in particular to a class of repair enzymes and includes AP endonucleases, glycosylases and lyases responsible for base excision repair.

A damaged base can be removed by a DNA glycosylase enzyme which hydrolyses an N-glycosylic bond between the deoxyribose sugar moiety and the base. For example, an *E. coli* glycosylase, UDG endonuclease, repairs deaminated cytosine while two 3-mAde glycosylases from *E. coli* (TagI and TagII) repair damage from alkylating agents.

The product of removal of a damaged base by a glycosylase is an apurinic or apyrimidinic site (AP site) that must be correctly replaced. This can be achieved by an endonuclease, which nicks the sugar phosphate backbone adjacent to the AP site. The abasic sugar is removed and a new nucleotide is inserted by polymerase/ligase activity. These repair enzymes are found in prokaryotic and eukaryotic cells. Some enzymes having applicability herein have glycosylase and AP endonuclease activity in one molecule. Abasic sites can be recognized and cleaved by AP endonucleases and/or AP lyases. Class II AP endonucleases cleave at AP sites to leave a 3' OH that can be used in polynucleotide polymerization. Furthermore, AP endonucleases can remove moieties attached to the 3' OH that inhibit polynucleotide polymerization. For example a 3' phosphate can be converted to a 3' OH by *E. coli* Endo IV. AP endonucleases can work in conjunction with glycosylases.

Examples of glycosylase substrates include Uracil, Hypoxanthine, 3-methyladenine (3-mAde), Formamidopyrimidine, 7,8 dihydro-8-oxyguanine and Hydroxymethyluracil. The presence of uracil in DNA may occur due to mis-incorporation or deamination of cytosine by bisulfate, nitrous acids, or spontaneous deamination. Hypoxanthine generally occurs due to deamination of adenine by nitrous acids or spontaneous deamination. In this context, 3-mAde is a product of alkylating agents. Formamidopyrimidine (FAPY) (7 -mGua) is a common product of methylating agents of DNA. 7,8-dihydro-8 oxoguanine is a mutagenic oxidation product of guanine. Gamma radiation produces 4,6-diamino-5-FAPY. Hydroxymethyuracil is created by ionizing radiation or oxidative damage to thymidine.

These different types of damage may be repaired using glycosylases of the sort described above and in Table 1.

Another type of repair enzyme is a lyase. This enzyme can break the phosphodiester bond in a polynucleotide.

Examples of AP endonucleases belong to 4 classes.
(I) cleaves 3' --> 3'-OH + 5'-P - and has associated glycosylase activity.
(II) cleaves 5' --> 3'-OH + 5'-P
(III) cleaves 3' --> 3'-P + 5'-OH
(IV) cleaves 5' --> 3'-P + 5'-OH

Several enzymes have been isolated that appear to have AP endonuclease or lyase and glycosylase activities that are coordinated either in a concerted manner or sequentially.

Examples of polynucleotide cleavage enzymes now found to be suitable for use in enhancing at least one of yield or fidelity in a copying or amplification reaction include the following types of enzymes derived from but not limited to any particular organism or virus: 1) AP endonucleases, such as *E. coli* Endo IV, *Tth* Endo IV, and human AP endonuclease; 2) glycosylases, such as UDG, *E. coli* AlkA and human Aag; 3) glycosylase/lyases, such as *E. coli* Endo III, *E. coli* Endo VIII, *E. coli* Fpg, human OGG1, and T4 pyrimidine dimer glycosylase (T4 pdg); and 4) lyases such as *E. coli* Endo V.

Endo VI (also termed Exo III) is known to be capable of degrading a substantial portion of a polynucleotide outside the damaged regions of interest in a polynucleotide in a few hours under normal reaction conditions, hence is not to be included in the present enzyme mixtures for treating damaged polynucleotides.

A "polymerase" for present purposes refers to an enzyme that has polymerase activity even though it may have other activities. The same polymerase or polymerases may be used throughout the repair and copying reactions or different polymerases may be employed at different stages of the present methods.

Examples of polymerases include thermostable bacterial polymerases such as *Taq* DNA and *Thermus thermophilus* polymerases and archeal polymerases such as Vent^{®}, Deep Vent^{®} and Pfu; less thermostable enzymes such as Bst polymerase, *thermomicrobium roseum* polymerases and mesophilic polymerases such as phage polymerases (such as phi29 polymerase, T7 polymerase and T4 polymerase), *E. coli* polI and *E. coli* polII Y family polymerases such as *E. coli* pol IV, *E. coli* pol V, human pol kappa, human pol eta, Sso Dpo 4, Sac Dbh, Sce pol zeta, human pol iota (MacDonald et al. Nucleic Acids Res. 34:1102-1111 (2006); Vaisman et al. DNA Repair 5:210 (2006); Ohmori et al. Mol. Cell. 8:7-8 (2001); Goodman Ann. Rev. Biochem. 71:17-50 (2002)) or mutants, derivatives or modifications therefrom. Examples of derivatives include Phusion™ enzyme (Finnzymes, Espoo, Finland) and other polymerases that combine a double strand binding protein with polymerase sequences from one or several sources.

A "ligase" as used in the enzyme mixtures described here refers to an enzyme that joins a 5' end of a single strand of a polynucleotide to a 3' end of another single strand of a polynucleotide. Such ligases are found in substantially all eukaryotic, prokaryotic, and archaeal cells, and can also be found in some bacteriophages and viruses. Examples of suitable ligases include 9°N ligase, *E. coli* ligase, T4 ligase and *Taq* ligase. Other ligases include LIGA (NP-416906.1), TthDNALGS (AAA27486.1), LIG3 (NM-013975) and LIG4 (NM-002312).

Other ligases or ligase-like proteins that may have utility herein are revealed by a Blast search using T4 ligase or *E. coli* ligase to search the database (see Figures 6A-1 - 6A-9 and Figures 6B-1 and 6B-2) in which any enzyme sharing at least 6 contiguous amino acids with these known ligases maybe included in a repair mixture according to embodiments of the invention.

Contrary to a published use of ligase in combination with Exo III in the absence of any cofactors (U.S. Publication No. 2005-0026147), it has been found here that NAD⁺ or ATP is required in enzyme mixtures that include ligase. More specifically, *Taq* ligase and *E. coli* ligase require NAD⁺ while T4 ligase and 9°N ligase require ATP. 60/717,296 and International Publication No. WO 2005/052124. In addition, thermostable repair enzymes can be used interchangeably with thermolabile repair enzymes in a preamplification mixture. Thermostable enzymes retain activity at above 40°C or more particularly 65°C or above.

Embodiments of present methods improve the yield or fidelity of products resulting from copying polynucleotide sequences, amplification or other synthesis reaction. This can be achieved, for example, when a damaged polynucleotide is treated with a preparation of enzyme(s) in a pre-incubation mixture and/or during amplification.

Amplification protocols that may benefit from the above described pre-incubation include polymerase chain reaction (PCR), Strand-Displacement Amplification (SDA) (U.S. Patent Nos. 5,455,166 and 5,470,723); Helicase-Dependent Amplification (HAD) (U.S. Publication No. 2004-0058378-A1); Transcription-Mediated Amplification (TMA) (Guatelli et al., Proc. Natl. Acad. Sci. USA 87:1874-1878 (1990)); Rolling Circle Amplification (RCA) which generates multiple copies of a sequence for the use in *in vitro* DNA amplification adapted from *in vivo* rolling circle DNA replication (see, for example, Fire and Xu, Proc. Natl. Acad Sci. USA 92:4641-4645 (1995); Lui, et al., J. Am. Chem. Soc. 118:1587-1594 (1996); Lizardi, et al., Nature Genetics 19:225-232 (1998)); and whole genome amplification methods. (Hawkins et al. Current Opinions in Biotechnology 13:65-67 (2002)).

A "universal" enzyme mixture has now been found to be useful in a reaction mixture for generally repairing damaged polynucleotides prior to or during copying or amplification. In accordance with the present embodiments, there are also specific advantageous combinations in given situations.

The universal enzyme mixture contains a ligase and a cofactor such as NAD⁺ or ATP. The mixture preferably additionally includes a polymerase and an AP endonuclease as described above within a suitable buffer such as Thermopol (New England Biolabs, Inc., Ipswich, MA), AccuTaq LA DNA polymerase buffer (Takara Bio Inc., Shiga, Japan) or any other conventional *Taq* buffer. In various illustrative embodiments, the universal enzyme mixture contains *E. coli* PolI or *Taq* polymerase and an AP endonuclease such as a mesophilic Endo IV, e.g., *E. coli* Endo IV or a thermophilic Endo IV, e:g., Tth Endo IV and a ligase selected from *E. coli* ligase, *Taq* ligase or an archaeal ligase such as 9°N ligase. In a particular embodiment, the enzyme mixture contains 1-100 units Endo IV, 0.05-0.25 units *E. coli* PolI, and 5-500 units of a *Taq* DNA ligase suitable for repairing 1-1000 ng DNA in a reaction volume of 10-1000 µL prior to or during amplification. It will be understood that the concentration range for endonucleases and polymerases other than those specified in the universal mixture above may vary with the enzyme used and the temperature of the reaction. However, the concentration range can be readily ascertained using the assays described in the Examples. For example, a standard preparation of lambda DNA can be heat-treated according to Example 1. The DNA can then be subjected to a series of enzyme mixtures containing ligase and cofactors. An additional enzyme is titrated to determine a preferred concentration for that enzyme in the mixture. In this way, DNA repair can be optimized. After amplification of each sample, the amount of the amplified DNA can be determined by gel electrophoresis revealing the preferred concentration range for the test enzyme.

The universal enzyme mixture can be used prior to or during polynucleotide amplification or other synthesis.

As illustrated in the Examples, depending on the type of damage, it may be desirable to supplement the universal enzyme mixture with additional repair enzymes depending on the nature of the DNA damage. The utility of individual repair enzymes or mixtures of repair enzymes can be determined using the assays described in the Examples and in the Figures to determine their suitability for repairing a particular polynucleotide.

### Repair of general or specific damage to polynucleotides

### (a) General damage

Determining the nature of damage in a polynucleotide is time-consuming. If some form of damage to a polynucleotide is suspected, for example, the polynucleotide is poorly amplified, it is preferable not to have to identify the lesion or lesions. In these circumstances, a universal mix of enzymes such as described above may be utilized to determine whether improved amplification is obtained. If the improvement is sufficient using the universal mixture then no further action is required. If the improvement is not sufficient, additional enzymes can be added to the mixture as described herein until the preferred result is obtained. The entire assay may be achieved in a single reaction vessel such as a 96 well dish. Each micro-well in the dish is available for a different enzyme mixture including the universal mixture plus enzymes selected to address each class of damage outlined below.
The protocol for selecting enzymes for repair of general damage or unknown damage of DNA is provided in Figure 12 (flow chart) and in the assays described in the Examples.

### (b) Specific damage

### (i) AP sites

The loss of a base is the most common form of spontaneous DNA damage. Polymerases and polymerase-based techniques are adversely affected by the presence of these abasic sites. The effectiveness of primer extension reactions is enhanced by repairing any abasic sites found in a polynucleotide. This is achieved in one embodiment by Endo IV activity that cleaves the phosphate backbone at the abasic site. This leaves an extendable 3' OH on the DNA fragment 5' to the cleaved abasic site. It also leaves a deoxyribose-5'-phosphate (dR5P) on the DNA fragment 3' to the cleaved abasic site. A polymerase can extend from the free 3' OH replacing the cleaved abasic site with a correct nucleotide. The dR5P may be removed by an enzyme that specifically targets dR5Ps such as mammalian pol beta or the 8Kd N-terminal portion of mammalian pol beta (Deterding J Biol Chem 275:10463-71 (2000)), by a flap endonuclease activity present in certain polymerases such as *E. coli* DNA polymerase I or by a separate flap endonuclease such as FENI. The removal of dR5P can also occur by cleavage downstream of this group by the flap endonuclease activity. After removal of the dR5P and the generation of a 5' phosphate adjacent to the 3' OH, a ligase can seal this nick finishing the repair (see Examples 1-3).

### (ii) Modified nucleotides

### (a) Thymidine dimers

Light can damage DNA by inducing the formation of pyrimidine dimers. Pyrimidine dimers block the DNA extension reaction catalyzed by DNA polymerases such as *Taq* DNA polymerase and hence inhibit DNA amplification (Wellinger, et al. Nucleic Acids Res. 24(8):1578-79 (1996)). Consequently it is desirable to repair pyrimidine dimers prior to or during amplification. This can be achieved by adding a pyrimidine dimer glycosylase/lyase (Vande Berg, et al. J. Biol. Chem. 273(32):20276-20284 (1998)) to the universal enzyme mixture. The DNA backbone is cleaved 5' to the pyrimidine dimer and leaves a 3' hydroxyl moiety that is extendable by a DNA polymerase. In certain embodiments, extension at the 3' hydroxyl and subsequent formation and then cleavage of the lesion-containing flap generated during DNA extension results in a nick that is sealed by an enzyme capable of sealing the nick. Cleavage of the flap can be achieved by the extending polymerase, for example, *E. coli* polymerase I or by the action of a flap endonuclease ((Xu, Y., et al. J. Biol. Chem. 275(27):20949-20955 (2000), Liu, Y., et al., Annu. Rev. Biochem. 73:589-615 (2004)).

### (b) Oxidative damage, alkylation and deamination

Inaccuracies can be introduced into the products of DNA amplification reactions because of undesired nucleotide incorporation opposite a damaged base (Gilbert, et al. Am. J. Hum. Gen. 72:48-61 (2003); Hofreiter et al. Nucl. Acids Res. 29:4793-9 (2001)). These inaccuracies can be discovered after amplifying, cloning and sequencing the same sample many times. Inaccuracies due to base damage can also be identified by comparing sequence data before and after sample treatment with an enzyme such as UDG, which removes one of the common types of mutagenic DNA lesions (Hofreiter, et al. Nucl. Acids Res 29:4793-9 (2001)). However, treatment with UDG creates an abasic site within the DNA that inhibits DNA amplification by primer extension. This may cause DNA samples to be refractory to amplification after UDG treatment. This AP site can then be repaired by a reaction mixture containing a ligase and preferably also an AP endonuclease and a polymerase. Removal of a uracil enables a polymerase in an amplification reaction that would normally be stopped at this site to continue amplifying the DNA. For example, Vent^{®} polymerase activity is inhibited on DNA templates containing uracil. The ability to remove the uracil permits the polymerase to have enhanced effectiveness.

In contrast, it is here shown that including UDG with an enzyme mixture that includes a ligase and a polymerase can be successfully used to enhance the yield and fidelity of the product of polynucleotide copying or amplification. Examples 12-14 provide descriptions of various beneficial enzyme mixes that include UDG.

Modified nucleotides that are the product of oxidative damage can also be removed from the polynucleotide by Fpg or hOGG to leave a blocked polynucleotide where the blocked polynucleotide is repairable by an AP endonuclease such as Endo IV.

The effectiveness of enzyme pretreatment to repair oxidative damage to a polynucleotide prior to copying or amplification is illustrated in Example 9 in which improved fidelity of the copied polynucleotide product is demonstrated using an enzyme mixture containing a ligase, a polymerase, Endo IV and Fpg.

Other modified nucleotides such as alkylated bases or deaminated bases where cytosine is converted to uracil, guanine to xanthine or adenine to hypoxanthine give rise to miscoding. Removal of these modified nucleotides is desirable. These modified bases can be removed by UDG as discussed above or by AlkA or Aag as described in Example 10.

### (iii) Cross-links

Additional nucleotide excision repair (NER) proteins (Minko et al. Biochemistry 44:3000-3009 (2005); Costa et al. Biochimie 85(11):1083-1099 (2003); Sancar Ann. Rev. Biochem 65:43-81 (1996)) can be used to repair damage resulting from formaldehyde and bulky adducts as well as damage that results in chemically-modified bases that form DNA-protein cross-links. At least one of *E. coli* UvrA, UvrB, mutant UvrB, UvrC, UvrD or Cho (Moolenar et al. Proc. Natl Acad. Sci USA 99:1467-72 (2002)) can be used to make incisions at the 5' end and optionally the 3' end around a damaged site. Details about the properties and purification protocols of these enzymes can be obtained from Zou, Y., et al. Biochemistry 43:4196-4205 (2004). The repair process can be completed by means of a DNA polymerase, a DNA ligase and optionally a flap endonuclease.

The generation of a 3' hydroxyl at a 5' incision site can be useful if the NER enzyme(s) cleave the DNA but leave a blocked 3' end on the DNA that inhibits primer extension. An example would be if the NER enzyme(s) cleaved the DNA and left a 3' phosphate. This would not be extendable by known DNA polymerases unless the 3' phosphate was removed by, for example, *E. coli* Endo IV.

If the NER enzyme or enzymes cleaves 5' and 3' to the DNA lesion then the damage is removed when the newly released oligonucleotide dissociates from the DNA. A polymerase can simply fill in the excised region of DNA leaving a nick, which ligase then seals to complete the repair. In certain cases, the polymerase may fill in the DNA and then proceed to displace the remaining DNA strand. In these circumstances, an enzyme with flapase activity permits a nick to be formed that a ligase can seal. In cases in which the NER enzyme or enzymes only cleaves 5' to the damage, the polymerase preferably displaces the original DNA strand until it is past the damage, at which point a flapase cleaves the DNA flap to create a ligatable nick. The flapase may be active before and after the DNA lesion is reached. Preferably, the polymerase and flapase activities work to eventually displace and remove the DNA lesion leaving a ligatable nick, thus repairing the DNA template. An example of the effectiveness of the above approach is provided in Example 7.

### (iv) Nicks, Gaps and Mismatched Polynucleotides

Nicks and gaps in the DNA backbone can lead to truncated primer extension products and formation of chimera by undesirable hybridization of single strand regions. Heteroduplex DNA can be a problem in multi-template PCR and in homogeneous template PCR (Lowell, J. L. & Klein, D. A. Biotechniques 28:676-681 (2000); Thompson, J. R., et al. Nucl. Acids Res. 30(9):2083-2088 (2002); Smith, J. & Modrich, P. Proc. Natl. Acad. Sci. USA 94:6847-6850 (1997)). For example, chimera can be formed at the mismatch sites.

The combined effect of a ligase and a polymerase together optionally with an enzyme that recognizes and cleaves at heteroduplex sites (T7 EndoI and mutants thereof) contained within a universal enzyme mixture results in repairs of nicks, gaps, heteroduplexes and chimera in the DNA thus enhancing yield and fidelity of polynucleotide copying and amplification reactions. Example 8 demonstrates the beneficial effects of using T7 Endo I and a ligase as illustrative of the above. The useful range of the T7 endonuclease or mutant:DNA ratio can be expanded by including a DNA ligase activity to minimize the detrimental effects of non-specific cleavage in the heteroduplex cleavage reaction. This approach does not require quantitation of DNA and avoids the extra steps after the PCR reaction required by Lowell, et al. Biotechniques 28:676-681 (2000); and Smith, et al. Proc. Natl. Acad. Sci. USA 94:6847-6850 (1997).

For some polynucleotides, the nature of the damage might be known. By way of illustration, a mixture of enzymes can be selected according to section (b) above for repairing the specific damage. Where the damage is unknown or the sources are mixed, the universal mix described herein including in the examples may be employed.

### DNA microarrays

DNA microarrays are a powerful methodology used to analyze DNA samples (Lipshutz et al. Curr Opinion in Structural Biology 4:376-380 (1994); Kozal, et al. Nat Med 2(7):753-9 (1996)). The amount and quality of information from microarray analysis of damaged DNA would benefit from first repairing the damaged DNA.

### Discussion of the Examples and Figures

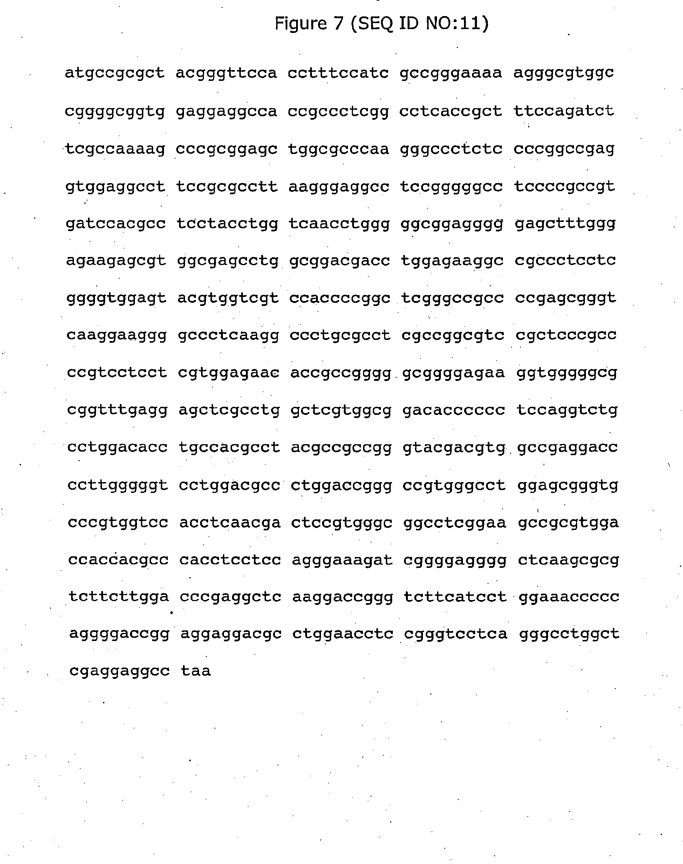

The enhancement in yields of product after amplification is shown both generally and for specific sources of damage: Figures 1A-1D, and 4 (heat damage), 2A-2B (citrate), 8A-8C (UV irradiation), 12, 3, 5 and 17 (general damage to DNA, environmental DNA, stored krill DNA and cave bear DNA) and 9A-9B (subject to undesirable heteroduplex formation) as well as the impact of different incubation times at different temperatures (Figures 13 and 14). The enhancement of fidelity is demonstrated in Figures 10A-10B and 11. Figures 6A-1 to 6A-9, 6B-1 and 6B-2 provide details of sequences that are homologous with *Taq* ligase and T4 ligase sequences. Figure 7 describes a DNA sequence for *Tth* Endo IV and Figures 18-1 to 18-2 describe a sequence for plasmid pNEB0.920. Figure 15 shows that multiple fragmented DNA can be repaired to form a template suitable for amplification to produce an amplicon. Benefits of repair mixes for cloning and transformation are illustrated in Figures 15 and 16.

Example 1 and Figures 1A-1D show that amplicon yields obtained from PCR amplification are substantially negatively affected when the template DNA is damaged beyond a certain threshold of damage (e.g., about 90 seconds heat treatment) (see Figure 1A). The effect of this damage on amplification can be reversed and amplicon yields enhanced by incubating the DNA with a mixture of enzymes before amplification (see Figures 1B, 1C and 1D). In addition, if amplicon yields of assumed "undamaged" DNA can be enhanced by adding the enzyme mixture described then in fact, the DNA is deemed to have been damaged.

Example 1 shows that the effect of the enzyme mixture on amplification of DNA is not dependent on a single type of AP endonuclease or ligase, but instead endonucleases or ligases from multiple alternative sources can be used. For example, thermostable *Tth* Endo IV was found to be as effective as *E. coli* Endo IV and *E. coli* ligase was as effective as the thermostable *Taq* ligase.

Example 2 and Figures 2A-2B show the negative effect on amplification yields of another type of DNA damage - depurination, which is induced in the presence of low pH. Moreover, the example shows that the effect of a mixture of enzymes on amplification of DNA is not dependent on a single type of polymerase but rather polymerases from multiple alternative sources can be used. For example, *E. coli* PolI can be substituted by *Taq* DNA polymerase or a mixture of *Taq* and Vent^{®} DNA polymerases to produce enhanced yields.

Example 3 and Figure 3 show that the enhancement of amplification yields can be observed with short (200 bp) fragments. In fact, enhancement of amplification yields should be observable for a wide range of sizes of DNA templates from as short as 100 bases to as long as 100 kb and it is believed that amplification yields for DNA even larger than 100 kb can be achieved.

Figure 3 also shows that even when the DNA has been damaged through storage in a crude form (for example, within the cells of an organism that has itself been stored), amplification yields are significantly enhanced by the addition of a mixture of enzymes prior to amplification. Although the mixture of enzymes was added to template DNA prior to amplification, a similar yield effect can be seen when the template DNA is incubated with the mixture of enzymes that are thermostable equivalents during amplification or during a pre-amplification step.

Example 4 and Figure 4 show that ligase alone can enhance amplicon yield, but adding an AP endonuclease further enhances the yield. The best result was observed in this example when a ligase, an AP endonuclease, and a DNA polymerase were used prior to amplification. Furthermore, this example demonstrates that repair is not DNA size dependent. For example, similar results were obtained with 5 kb and 10 kb amplicons.

Example 5 and Figure 5 show that an enhanced yield from amplification can be achieved using a ligase, which may be an NAD⁺ dependent or ATP dependent enzyme and that this effect can be achieved without limitation to a single source of ligase. Figure 5 shows that *Taq* ligase and T4 ligase are both effective in enhancing amplification yield even when used without additional enzymes in a pre-incubation mix. This effect is also believed to occur if the ligase is added to the amplification mix (if thermostable). Figure 5 also shows the benefit of this approach when amplifying environmental DNA obtained directly from soil samples that have been exposed in nature to a variety of damaging agents.

Figures 6A-1 to 6A-9, 6B-1 and 6B-2 and 7 are descriptions of enzymes or the sequence encoding enzymes used in the universal mixture of enzymes, which is so named because it can be broadly applied to damaged polynucleotides without specific knowledge concerning the damage itself. While the examples refer to DNA, amplification or copying of polynucleotides is more generally envisaged. In general, the conditions under which amplification is found to be improved are also applicable to enhancing copying of polynucleotide generally even if this is not explicitly stated in all cases.

### EXAMPLES

### Example 1: Enhancing amplicon from DNA damaged by heat treatment

An assay was developed for optimizing the use of selected reagents to repair DNA prior to amplification.

### Generation of various extents of heat damage

Various amounts of DNA damage were induced by heat treatment. This was achieved as follows: 100 µL lambda DNA (NEB#N3011, New England Biolabs, Inc., Ipswich, MA) at 0.5 mg/ml was aliquoted into separate tubes for heat treatment at 99°C for 30 sec, 60 sec, 90 sec, 120 sec, and 180 sec, respectively in a PE2700 thermal cycler. A sample was used as a template for amplification without pretreatment.

The remaining damaged DNA was pretreated by the mixture of enzymes as follows: The damaged DNA templates were incubated at room temperature in the following mixture for 10 minutes:
DNA (5ng, 2ng and 1ng);
100 µM dNTPs (NEB#M0447, New England Biolabs, Ipswich, MA);
1 mM NAD⁺ (Sigma#N-7004, Sigma, St. Louis, MO);
80 units *Taq* ligase (NEB#M0208, New England Biolabs, Ipswich, MA) or 40-100 units of *E. coli* ligase (NEB#M0205S);
0.1 units *E. coli* DNA polymerase I (*E. coli* polI) NEB#M0209, New England Biolabs, Inc., Ipswich, MA);
10 units *E. co*/*i* Endo IV (NEB#M0304, New England Biolabs, Inc., Ipswich, MA) or 10 units of *Tth* Endo IV;
1X Thermopol buffer (NEB#B9004, New England Biolabs, Inc., Ipswich, MA) to a final volume of 96 µL.

At the end of the reaction, the samples were transferred to ice and then amplified.

### DNA Amplification Reaction

DNA amplification of lambda was performed using the following primers: CGAACGTCGCGCAGAGAAACAGG (L72-5R) (SEQ ID NO:1) and CCTGCTCTGCCGCTTCACGC (L30350F) (SEQ ID NO:2) according to the method of Wang et al. Nucl. Acids Res. 32: 1197-1207(2004).

4 µl of amplification mixture was added to 96 µl of the above repair mixture. The amplification mixture contained 100 µM dNTPs, 5 units *Taq* DNA polymerase, 0.1 units Vent^{®} (exo+) DNA polymerase, 5x10⁻¹¹ M primer L72-5R and 5x10⁻¹¹ M primer L30350F in 1X Thermopol buffer.

To correct for any enzyme storage buffer effects, when a repair enzyme was omitted from a reaction, the appropriate volume of its storage buffer was added to the reaction. In all cases, the amplification reactions were processed in a thermal cycler using the following parameters: 20 sec at 95°C for 1 cycle followed by 5 sec at 94°C, then 5 min at 72°C for 25 cycles. The size of the amplicon being amplified was 5 kb.

The results of amplification of DNA (5 kb) were determined by 1% agarose gel electrophoresis. 6X loading dye (Molecular Cloning:A Laboratory Manual, 3rd ed., eds. Sambrook and Russell, Cold Spring Harbor Press, Cold Spring Harbor, NY, 2001, pp. 5.4-5.17) was added to the 100 µl amplification reactions. 20 µl of this solution was then loaded onto the agarose gel along with 1 µg of 2-log ladder (NEB#N3200, New England Biolabs, Inc., Ipswich, MA) as a size standard.

The amount of amplified DNA for each sample was compared by gel electrophoresis and the results are shown in Figures 1A-D. When the samples were treated with a mixture of enzymes after heat treatment but prior to amplification, significant enhancement of amplification yields was achieved (Figures 1B, 1C and 1D).

### Example 2: Enhanced amplicon yields from DNA with low pH-induced abasic sites following pretreatment with an enzyme mixture

### Generation of various extents of damage resulting from abasic sites

To assay the extent of repair of damaged DNA, various amounts of DNA damage was first induced by acidic pH. This was achieved as follows:
DNA was depurinated as described by Ide, H., et al. Biochemistry 32(32):8276-83 (1993). Lambda DNA (NEB#N3011, New England Biolabs, Inc., Ipswich, MA) was ethanol precipitated. The DNA was resuspended in depurination buffer (100 mM NaCl, 10 mM citrate, pH 5.0) at a concentration of 0.5 mg/ml and incubated at 70°C for 0, 20, 40, 80, 120, and 160 minutes. The sample was then ethanol precipitated and resuspended in 0.01 M Tris, 0.001 M EDTA, pH 8.0. The DNA concentration was determined by measuring the A₂₆₀ of the DNA-containing solutions after calibrating with a buffer control.

### Pretreatment of DNA with a mixture of enzymes

The damaged DNA was incubated at room temperature for 10 minutes in the following mixture:
DNA (2.5 ng of damaged DNA after 120 minute of low pH treatment);
100 µM dNTPs;
1 mM NAD⁺;
80 units *Taq* ligase;
0.1 units *Taq* DNA polymerase or 0.1 units *E. coli* PolI (NEB#M0209, New England Biolabs, Inc., Ipswich, MA)) or 0.1 units *Taq:0.002* units of Vent^{®} Pol, (NEB#M0254, New England Biolabs, Inc., Ipswich, MA));
10 units *E. coli* Endo IV;
1X Thermopol buffer to a final volume of 96 µl.

The above mixture was incubated at room temperature for 10 minutes and then transferred to ice prior to amplification.

### DNA amplification reaction

Amplification was performed as described in Example 1 to generate a 5 kb amplicon. Amplicon yields were increased as compared with negative controls (Figure 2A) by treating lambda DNA containing abasic sites with the mixture of enzymes. The results are shown in Figure 2B for a series of pretreatments using different enzyme mixtures. The enzyme mixtures were varied with respect to the polymerase (*E. coli* Poll or Tag:Vent^{®}).

### Example enhanced amplicons yields of DNA extracted from an intact organism after storage in a preservative

Genomic DNA was isolated from *Meganyctiphanes norvegica* (Krill) as described in Bucklin, A. & Allen, L. D. Mol. Phylogenet. Evol. 30(3):879-882 (2004). The Krill had been stored in ethanol for about 5 years.

Pretreatment of the Krill DNA by a mixture of enzymes was carried out as follows:
50 ng of *M. norvegica* genomic DNA;
100 µM dNTPs;
1 mM NAD⁺;
40 units of *Taq* ligase;
0.5 units *Taq* DNA polymerase, 0.2 units Vent^{®} (exo+) DNA polymerase, or a *Taq*:Vent^{®} (exo+) mix containing 0.05 units of *Taq* DNA polymerase and 0.001 units of Vent^{®} (exo+);
10 units *E. coli* Endo IV;
1X Thermopol buffer to a final volume of 96 µl.
This reaction was incubated 15 minutes at room temperature before proceeding to the amplification step.

### DNA amplification reaction

The amplification primers corresponded to 52F and 233R as described in Bucklin, A. & Allen, L. D. Mol. Phylogenet. Evol. 30(3):879-82 (2004) generating a 200 bp amplicon.
52F: TTTTTAGCAATACACTACACAGCAA (SEQ ID NO:3)
233R: ATTACGCCAATCGATCACG (SEQ ID NO:4)

Primers were added to a final concentration of 0.5 µM, and each dNTP to a final concentration of 200 µM. 1 µl of the 50:1 Taq:Vent^{®} mix (5 units *Taq* DNA polymerase and 0.1 units Vent^{®} (exo+) DNA polymerase added to the reaction) was then added to each reaction to a final volume of 100 µL.

For the control reaction (Lane 1), no Endo IV, *Taq* ligase or pretreatment polymerase was added. Volumes were adjusted accordingly. In reactions in which repair enzymes were omitted, the appropriate volume of enzyme storage buffer was added to control for buffer effects.

Cycling conditions were as follows: 30 sec at 94°C, 30 sec at 52°C and 1 min 40 sec at 72°C for 40 cycles. 25µL (one quarter of the reaction) was prepared, loaded on a 1% agarose gel, electrophoresed, and visualized as described above.

Increased amplicon yield from krill genomic DNA was observed after preincubation of the samples using the enzyme mixtures described above (Figure 3).

### Example 4: Enhanced yields of a large (10 kb) amplicon from heat-damaged DNA

Heat-damaged DNA was prepared as described in Example 1. Lambda DNA was heated to 99°C for 180 sec.

Pretreatment of damaged DNA by a mixture of enzymes was carried out as follows:
Lambda DNA (1 µg of 180 sec heat-treated DNA);
100 µM dNTPs;
1 mM NAD⁺;
80 units of *Taq* ligase;
0.1 unit of *E. coli* PolI;
100 units of *E. coli* Endo IV;
1X Thermopol buffer to a volume of 96 µL.

The mixture was incubated for 10 minutes prior to amplification.

DNA amplification was performed as described in Example 1, except where specified below. Primers were added to the above 96 µl of preincubation mixture. Primer L71-10R (sequence GCACAGAAGCTATTATGCGTCCCCAGG) (SEQ ID NO:5) replaced L72-5R in Example 1. The iCycler thermal cycler program (Bio-Rad, Hercules, CA) was: 20 sec at 95°C for 1 cycle, 5 sec at 95°C, 10 min at 72°C for 25 cycles and then 10 min at 72°C for 1 cycle. Amplicon size was 10 kb.

The DNA was visualized as described in Example 1 with the following exceptions. 20 µl of 6X loading buffer was added to the 100 µl amplification reaction. 10 µl of this solution was diluted to 100 µl with H₂O and 1X loading buffer. 20 µl of this was loaded into each lane. The gel was a 0.8% agarose gel. The results are shown in Figure 4.

### Example 5: Improved amplification yield of DNA from environmental DNA (extracted from soil samples)

Environmental DNA was isolated from the soil using an UltraClean Soil DNA Kit from MoBio Laboratories, Inc., Carlsbad, CA (catalog # 12800-50).

### Pretreatment of DNA with a ligase

A final volume of 100 µl containing 0.6 µg of environmental DNA isolated from soil and one of the two ligases described below in (a) and (b) formed the reaction mixture. This reaction mixture was then incubated at room temperature for 15 min.
(a) 1X *Taq* ligase buffer (New England Biolabs, Inc., Ipswich, MA) and 80 units of *Taq* ligase.
(b) 1X T4 ligase buffer (New England Biolabs, Inc., Ipswich, MA) and 800 units of T4 ligase (NEB#M0202, New England Biolabs, Inc.., Ipswich, MA).

1 µl of reaction mixture was used in the amplification reaction described below.

### DNA Amplification Reaction

DNA amplification was performed using primers:
GGGGGXAGAGTTTGATCMTGGCTCA (SEQ ID NO:6) and
GGGGGXTACGGYTACCTTGTTACGACTT (SEQ ID NO:7)
(M = C or A, Y = C or T, X = 8-oxo-Guanine). These primers target 16S rDNA having an amplicon size of 1.6Kb.

The 50 µl reaction contained 10 pmol of each of the primers, 1 µl of the repaired environmental DNA, 200 µM dNTPs, 1X Thermopol buffer, and 1.25 units *Taq* DNA polymerase. The amplification was performed using the following cycling parameters: 5 min at 94°C for 1 cycle, 30 sec at 94°C, 1 min at 55°C, 1 min 40 sec at 72°C for 32 cycles, then 5 min at 72°C for 1 cycle.

Gel electrophoresis was performed as described in Example 1. The results are shown in Figure 5.

### Example 6: Enhanced amplicons yield of ultraviolet light-damaged DNA

To determine conditions for assaying the effectiveness of DNA repair, 50 µg lambda DNA (NEB#N3011, New England Biolabs, Inc., Ipswich, MA) was diluted in TE buffer (10 mM Tris-HCl, 1 mM EDTA, pH 7.5) to a concentration of 50 µg/ml and irradiated with 36 J/m² UV light for 0, 10, 20, 30, 40 and 50 sec.

Pretreatment of damaged DNA by a mixture of enzymes was carried out as follows:
The damaged DNA was incubated at room temperature for 15 minutes in the following mixture:
   DNA (50 ng of lambda DNA-damaged for 0, 10, 20, 30, 40, or 50 seconds);
   200 µM dNTPs;
   1 mM NAD⁺;
   400 units *Taq* ligase;
   0.1 units *E. coli* DNA polymerase I;
   10 units *E. coli* Endo IV;
   80 units or 10 units T4 PDG (also referred to as T4 Endo V). (Trevigen, Gaithersburg, MD);
   1XThermopol buffer
   Adjust volume with water to 50 µl.

After the 15 minutes incubation, the 50 µl reaction mixture was added to 50 µl of an amplification solution. The amplification solution consisted of 40 pmol of each primer (L72-5R and L30350F as described in Example 1 or L72-2R (the DNA sequence was CCATGATTCAGTGTGCCCGTCTGG) (SEQ ID NO:8), 1X Thermopol buffer, 1 mM NAD⁺, 200 µM dNTPs, 2.5 units *Taq* DNA polymerase (NEB#M0267, New England Biolabs, Inc., Ipswich, MA), and H₂O to a final volume of 50 µL. Combining the 50 µL repair reaction with the 50 µl amplification solution gave a final volume of 100 µl.

The 100 µl solutions were placed into a thermal cycler.
For the L72-5R and L30350F primer combination:
5 min at 94°C for 1 cycle; 30 sec at 94°C, 60 sec at 58°C, and 4 min at 72°C for 30 cycles; 5 min at 72°C for 1 cycle.
For the L72-2R and L30350F primer combination:
5 min at 94°C for 1 cycle; 30 sec at 94°C, 60 sec at 58°C, and 2 min at 72°C for 30 cycles; 5 min at 72°C for 1 cycle.

The presence of amplification product was visualized on a 1.8% agarose gel using ethidium bromide. The size of any band was compared against a lane containing the 2-log ladder (NEB#N3200S, New England Biolabs, Inc., Ipswich, MA) size standards. The results are shown in Figure 8.

### Example 7: Enhanced amplicon yield of DNA using the nucleotide excision repair proteins, UvrA, UvrB and UvrC

Increased amplicon yield from krill genomic DNA is determined after pre-incubation of the samples using an enzyme mixture containing proteins involved in nucleotide excision repair.

Pretreatment of stored DNA by a mixture of enzymes is carried out as follows:
Stored DNA is incubated for 1-180 minutes at 4-37°C in the following mixture:
   DNA: 50 ng of *M. norvegica* genomic DNA;
   100 µM dNTPs;
   1 mM ATP;
   400 units of *Taq* ligase;
   0.1 units *E. coli* DNA polymerase I;
   10 nM *E. coli* UvrA, 250 nM *E. coli* UvrB (or mutant UvrB*), plus or minus 50 nM *E. coli* UvrC;
   1X Thermopol buffer to a final volume of 96 µl.
   * for mutant UvrB, see Zou, Y., et al. Biochemistry 43:4196-4205 (2004).

DNA amplification reactions are conducted as described in Example 3.

### Example 8: Increasing sequence accuracy of a DNA after removal of incorrect nucleotides on at least one strand by means of enzyme cleavage of heteroduplexes

### Experimental Conditions

A. Adding *Taq* ligase to T7 Endo I was demonstrated to permit the use of an increased concentration of T7 Endo I in a DNA preparation without randomly degrading the DNA.

The assay relies on treating a supercoiled DNA containing a cruciform structure with increasing amounts of T7 Endo I.

0, 1.6, 3.1, 6.2, 12.5, 25, 50, 100, 200, or 400 units of T7 Endo I (NEB#M0302, New England Biolabs, Inc., Ipswich, MA) was added to 50 µl reactions composed of 1 µg of pUC(AT) (Guan, C., et.al. Biochemistry 43:4313-4322 (2004)) and 1X NEBuffer 2 (NEB#B7002S, New England Biolabs, Inc., Ipswich, MA). Plasmid pAT25*tet*A can be used in place of pUC(AT) (Parkinson, M. J. & Lilley, D. M. J. Mol. Biol. 270:169-178 (1997) and Bowater, R. P., et. al. Biochemistry 33:9266-9275 (1994)). Another set of reactions were set up simultaneously and used the same components as described above with the addition of 1 mM NAD⁺ (Sigma catalog#N-7004, Sigma, St. Louis, MO) and 100 units of *Taq* ligase (using a stock of NEB#M0208 at a concentration of 100 u/µl, New England Biolabs, Inc., Ipswich, MA). All reactions were incubated at 37°C for 60 minutes.

The results were analyzed by running the reactions on a 0.9% TBE agarose gel, stained with ethidium bromide, and visualized using UV light (see Figures 9A and 9B.). With no T7 Endo I present the pUC(AT) plasmid produced 2 bands on the gel corresponding to the supercoiled form (lower band) and the relaxed circular form (upper band).

T7 Endo I resolved the supercoiled pUC(AT) into the relaxed circular form and a linear form that ran intermediate to the supercoiled and relaxed circular forms. At certain T7 Endo I:DNA ratios, a smear was produced indicating that the T7 Endo I had degraded the DNA by non-specific enzymatic activity. The presence of *Taq* ligase significantly increased the usable T7 Endo I to DNA ratio. This ratio is further improved by substituting T7 Endo I with the mutant T7 Endo I described in International Publication No. WO 2005/052124.

B. The use of Method A to remove heterduplexes from PCR reactions.

Isolation of DNA from soil and amplification of the purified DNA is performed as described in Example 5 with the optional addition of 5 units T7 Endo I or mutant thereof. When T7 Endo I or mutant thereof is added, an additional amplification cycle is added (37°C for 15 minutes for 1 cycle). The last step is to allow the T7 endonuclease to cleave any heteroduplexes formed.

Gel electrophoresis is performed as described in Example 1. Heteroduplex DNA is visualized on the gel as described in Lowell, J. L. & Klein, D. A. Biotechniques 28:676-681 (2000). Absence of heteroduplex DNA in the presence of T7 Endo I or mutants thereof shows the effectiveness of T7 Endo I or mutants thereof with a ligase.

Unit definitions are described with the product description for each of the enzymes recited herein in the NEB catalog, New England Biolabs, Inc., Ipswich, MA. For example, the unit definition for T7 Endo I or mutant thereof is the amount of enzyme required to convert greater than 90% of 1 µg of supercoiled plasmid into greater than 90% linear DNA in a reaction volume of 50 µl in 1 hour at 37°C.

The T7 Endo I: DNA ratio can be increased without increasing non-specific cleavage of DNA in the presence of ligase.

### Example 9: Enhancing the sequence accuracy of a DNA implication reaction after oxidative damage

### Generating DNA with oxidative damage

The DNA subject to oxidative damage was pWB407 (Kermekchiev, M. B. et al. Nucl. Acids Res. 31:6139-47 (2003)). The damage was incurred using a combination of methylene blue (MB) and visible light as described previously (Sattler, et al. Arch. Biochem Biophys. 376(1):26-3 (2000)). Plasmid DNA (200 µg/ml in distilled water) was spotted on parafilm stretches (50 µl drops). MB was added to the drops to a final concentration ranging from 0 to 50 (0, 3, 6, 12.5, 25 and 50) µg/ml (100 µl final volume). Plates with these parafilm stretches were placed on ice and illuminated for 8 min. with a 1 x 100-W lamp. The MB-light-treated DNA was precipitated, dried, and then re-suspended in 50 µl of TE buffer (pH 8.0). Final DNA concentration was determined by the absorbance of light at 260 nm.

### DNA amplification conditions

A portion of pWB407 that contained the lacZ gene was amplified using primers 316-138, TGTCGATCAGGATGATCTGGAGGAAGAGC (SEQ ID NO:9), and 316-137, CGAAAGCTTTCAAGGATCTTACCGCTGTTGAGA (SEQ ID NO:10). Primers 316-138 and 316-137 were based on the previously-described primers Kfd-29 and H3Bla34, respectively (Kermekchiev, M. B. et al. Nucl. Acids Res. 31:6139-47 (2003)). The 100 µL PCR reactions contained either 10 or 50 ng of template DNA, indicated where appropriate, and 40 picomoles of each primer. The cycling conditions utilized varied with the thermal stable polymerase used for amplification.

Cycling conditions when using *Taq* DNA polymerase (NEB cat#M0267S, New England Biolabs, Inc., Ipswich, MA) had an initial denaturation step of 5 min at 94°C for 1 cycle, then 30 sec at 94°C, 60 sec at 58°C, and 3 min 30 sec at 72°C for 30 cycles, and finally 5 minutes at 72°C.

Cycling conditions when using Phusion™ DNA polymerase (NEB cat#F-530S, New England Biolabs, Inc., Ipswich, MA) had an initial denaturation step of 30 sec at 98°C for 1 cycle, then 10 sec at 98°C, 30 sec at 62°C, and 1 min 30 sec at 72°C for 30 cycles, and finally 5 min at 72°C.

The reaction outcomes were analyzed by loading 25 µL of the reaction on a 1.6% agarose gel, prepared, electrophoresed and visualized as described above. The marker used was the 2-log DNA ladder (NEB cat#N3200S, New England Biolabs, Inc., Ipswich, MA).

### Amplification accuracy determination

The accuracy of DNA amplification from the pWB407 template was determined as described by Barnes, et al. Gene 112:29-35 (1992) and Kermekchiev, et al. Nucl. Acids Res. 31:6139-47 (2003). Amplicons containing the lacZ gene were generated from plasmids pWB407 that had been subjected to differing amounts of oxidative damage. The oxidative damage was performed using methylene blue as described above. The PCR reactions were performed using 50 ng of template as described above. After cycling, 10 units of the restriction endonuclease, DpnI, was added to each 100 µL PCR reaction and incubated for 2 hours at 37°C. This step eliminated the original template plasmid. Next, the resulting amplification products were extracted with phenol/chloroform and precipitated using isopropanol (Molecular Cloning: A Laboratory Manual, 3rd ed., eds. Sambrook and Russell, Cold Spring Harbor Press, Cold Spring Harbor, NY, 2001, pp. 6.25, A8.12-A8.24). Precipitated products were re-suspended in H₂O and cut with the restriction endonucleases StyI and HindIII using conditions recommended by the manufacturer (New England Biolabs, Inc., Ipswich, MA). The DNA digestion reactions were stopped by inactivating the HindIII and StyI enzymes by heating to 65°C for 20 min. The restriction digestion products were purified using a microcon YM-100 column (Millipore, Billerica, MA) to eliminate short DNA fragments.

The repair reaction mixtures in a total of 50 µl contained 10 or 50 ng of pWB407 amplicons +/- methylene blue incubation. The repair reactions contained 20 mM Tris-HCl (pH 8.8 at 25°C), 10 mM KCI, 10 mM (NH₄)₂SO₄, 2 mM MgSO₄, 0.1% Triton X-100, 1 mM NAD⁺, 200 µM dNTPs (dATP, dTTP, dCTP, and dGTP), and various repair enzyme mixtures.

The repair enzyme mixtures used separately or in various combinations in a total volume of 50 µL were:
0.4 units Fpg, NEB cat#M0240S, New England Biolabs, Inc., Ipswich, MA);
200 units *Taq* ligase;
0.1 units *E*. *coli* DNA polymerase I;
10 units *E*. *coli* Endo IV;
1 mM NAD⁺;
100 µM dNTPs;
1X Thermopol buffer.

The reactions were incubated at 25°C for 15 minutes. After the incubation, 50 µL of a PCR mix (20 mM Tris-HCl (pH 8.8 at 25°C), 10 mM KCl, 10 mM (NH₄)₂SO₄, 2 n-M MgSO₄, 0.1% Triton X-100, 1 mM NAD⁺, 200 µM dNTPs (dATP, dTTP, dCTP, and dGTP), and either 2.5 units *Taq* DNA polymerase (NEB cat#M0267S, New England Biolabs, Inc., Ipswich, MA) or 1 unit of Phusion™ DNA polymerase was added to the 50 µL repair reaction and this new solution was subjected to thermal cycling conditions for PCR. The amplicons from these reactions were purified and restriction enzyme digested as described for other amplicons above.

The amplicons were cloned into the pWB407 plasmid. Plasmid pWB407 was prepared by digestion with the restriction endonucleases StyI and HindIII followed by a 30-minute incubation at 37°C with 1 unit/µg DNA of antarctic phosphatase (NEB cat#M0289S, New England Biolabs, Inc., Ipswich, MA). The dephosphorylated pWB407 vector backbone was purified by agarose gel electrophoresis. Gel extraction was performed with a QIAquick Gel Extraction Kit (Qiagen, Valencia, CA).

The digested amplicons were ligated into the prepared pWB407 plasmids in 30 µL reactions using approximately 0.1 µg vector DNA and about 0.5 µg amplicon. T4 ligase was used to perform the ligation following the manufacturers recommended conditions (New England Biolabs, Inc., Beverly, MA). Ligation products were electroporated into *E. coli* strain WB441 (Barnes, W. Gene 112:29-35 (1992)). The selective indicator plates used were LB plates containing 50 µg/ml ampicilin and 80 µg/ml Xgal. Before plating, the bacteria were incubated in rich both for 1 hour at 37°C to allow expression of the ampicilin resistance. Control transformations lacking ligase treatment resulted in zero colonies. Colonies were scored for blue color after one day at 37°C, and one or two days at 25°C. The results are shown in Figures 10A-10B and 11.

### Example 10: Enhancing the sequence accuracy of a DNA amplification reaction after deamination damage

### Generating deaminated DNA

The DNA subjected to deamination is pWB407 (Kermekchiev, et al. Nucl Acids Res 31: 6139-6147 (2003)). The damage is incurred using random mutagenesis with nitrous acid as described in Yan, W. et al. J Virol. 77(4):2640-50 (2003). Nitrous acid can deaminate guanine in DNA to xanthine, cytosine to uracil, and adenine to hypoxanthine.

Plasmid DNA (2mg) is treated with 0.7 M NaNO₂ in 1M acetate buffer, pH 4.6. The reaction is terminated at various time points by addition of 4 volumes of ice-cold 1 M Tris-HCl (pH 7.9). The plasmid DNA is alcohol precipitated, dried and then resuspended in 100 µL of TE buffer.

### Pretreatment reaction to repair deaminated bases

The repair enzyme mixtures used separately or in various combinations in a total volume of 50 µL are:
(a)
   1 unit Human Aag, New England Biolabs, Inc., Ipswich, MA;
   2 units Endo III (NEB cat # M0268S), New England Biolabs, Inc., Ipswich, MA;
   2 units Endo V (NEB cat # M0305S), New England Biolabs, Inc., Ipswich, MA;
   2 units UDG (NEB cat # M0280S), New England Biolabs, Inc., Ipswich, MA;
   200 units *Taq* ligase;
   0.1 units *E. coli* DNA polymerase I;
   10 units *E. coli* Endo IV;
   1mM NAD⁺;
   100 µM dNTPs;
   1x Thermopol buffer.
(b)
   2 units Endo V (NEB cat # M0305S), New England Biolabs, Inc.,.Ipswich, MA;
   2 units UDG (NEB cat # M0280S), New England Biolabs, Inc., Ipswich, MA;
   200 units Taq DNA ligase;
   0.1 units *E. coli* DNA polymerase I;
   10 units *E. coli* Endo IV;
   1mM NAD⁺;
   100 µM dNTPs;
   1x Thermopol buffer.
(c)
   2 units Endo V (NEB cat # M0305S), New England Biolabs, Inc., Ipswich, MA;
   200 units Taq Ligase;
   0.1 units *E. coli* DNA polymerase I;
   10 units *E. coli* Endo IV;
   1mM NAD⁺;
   100 µM dNTPs;
   1x Thermopol buffer.
(d)
   1 unit Human Aag, New England Biolabs, Inc., Ipswich, MA;
   2 units Endo III (NEB cat # M0268S), New England Biolabs, Inc., Ipswich, MA;
   200 units Taq Ligase;
   0.1 units *E. coli* DNA polymerase I;
   10 units *E. coli* Endo IV;
   1mM NAD⁺;
   100 µM dNTPs;
   1x Thermopol buffer.
(e)
   1 unit Human Aag, New England Biolabs, Inc., Ipswich, MA;
   2 units UDG (NEB cat # M0280S), New England Biolabs, Inc., Ipswich, MA;
   200 units Taq ligase;
   0.1 units *E. coli* DNA polymerase I;
   10 units *E*. coli Endo IV;
   1 mM NAD⁺;
   100 µM dNTPs;
   1x Thermopol buffer.
(f)
   1 unit Human Aag, New England Biolabs, Inc., Ipswich, MA;
   2 units Endo V (NEB cat # M0305S), New England Biolabs, Inc., Ipswich, MA;
   200 units Taq ligase;
   0.1 unit *E. coli* DNA polymerase I;
   10 units *E. coli* Endo IV;
   1 mM NAD⁺;
   100 µM dNTPs;
   1x Thermopol buffer.

The amplification reaction conditions and amplification accuracy determination are performed as described in Example 9.

### Example 11: Unit Definitions

### Thermophilic ligase unit

One unit is defined as the amount of enzyme required to give 50% ligation of 1 µg of BstE II-digested lambda DNA in a total reaction volume of 50 µl in 15 minutes at 45°C. Taq ligase is available from New England Biolabs, Inc., Ipswich, MA.

### Mesophilic ligase unit

One unit is defined as the amount of enzyme required to give 50% ligation of Hind III digested lambda DNA (5' DNA termini concentration of 0.12 µM, 300 µg/ml) in a total reaction volume of 20 µl in 30 minutes at 16°C. *E. coli* ligase is available from New England Biolabs, Inc., Ipswich, MA.

### AP endonuclease unit

One unit is defined as the amount of enzyme required to cleave 1 pmol of a 34-mer oligonucleotide duplex containing a single AP site in a total reaction volume of 10 µl in 1 hour at 37°C.

### Mesophilic polymerase unit

One unit is defined as the amount of enzyme that will incorporate 10 nmol of dNTP into acid-insoluble material in a total reaction volume of 50 µl in 30 minutes at 37°C with 33 µM dNTPs including [³H]-dTTP and 70 µg/ml denatured herring sperm DNA.

### Thermophilic polymerase Unit

One unit is defined as the amount of enzyme that will incorporate 10 nmol of dNTP into acid-insoluble material in a total reaction volume of 50 µl in 30 minutes at 75°C with 200 µM dNTPs including [3H]-dTTP and 200 µg/ml activated Calf Thymus DNA. Thermophilic polymerases-Taq polymerase and archaeal polymerases are available from New England Biolabs, Inc., Ipswich, MA.

The unit definitions for thermophilic UDG, Fpg , Endo III and Endo VIII are the same as those for the mesophilic equivalents listed (NEB catalog, New England Biolabs, Inc., Ipswich, MA).

| Gene Name | Activity | Accession Number |
|---|---|---|
| UNG | Uracil-DNA glycosylase | NM_080911 |
| SMUG1 | Uracil-DNA glycosylase | NM_014311 |
| MBD4 | Removes U or T opposite G at CpG sequences | NM_003925 |
| *TDG* | Removes U, T or ethenoC opposite G | NM_003211 |
| *OGG1* | Removes 8-oxoG opposite C | NM_016821 |
| *MUTYH (MYH)* | Removes A opposite 8-oxoG | MM_012222 |
| *NTHL1 (NTH1)* | Removes Ring-saturated or fragmented pyrimidines | NM_002528 |
| *MPG* | Removes 3-meA, ethenoA, hypoxanthine | NM_002434 |
| *NEIL1* | Removes thymine glycol | NM_024608 |
| *NEIL2* | Removes oxidative products of pyrimidines | NM_145043 |
| *XPC* | Binds damaged DNA as complex | MM_004628 |
| *RAD23B (HR23B)* | XPC, RAD23B, CETN2 | NM_002874 |
| *CETN2* | | MM_004344 |
| *RAD23A (HR23A)* | Substitutes for HR23B | NM_005053 |
| *XPA* | Binds damaged DNA in preincision complex | MM_000380 |
| *RPA1* | Binds DNA in preincision complex | MM_002945 |
| *RPA2* | RPA1, RPA2, RPA3 | MM_002946 |
| *RPA3* | | NM_002947 |
| *ERCC5 (XPG)* | 3' incision | NM_000123 |
| *ERCC1* | 5' incision subunit | NM_001983 |
| *ERCC4 (XPF)* | 5' incision subunit | NM_005236 |
| *LIG1* | DNA joining | NM_000234 |
| *CKN1 (CSA)* | Cockayne syndrome; Needed for transcription-coupled NER | NM_000082 |
| *ERCC6 (CSB)* | CKN1, ERCC6, XAB2 | NM_000124 |
| *XAB2 (HCNP)* | | NM_020196 |
| *DUDB1* | Complex defective in XP group E | NM_001923 |
| *DDB2* | DDB1, DDB2 | NM_000107 |
| *MMS19L (MMS19)* | Transcription and NER | NM_022362 |
| *FEN1 (DNase IV)* | Flap endonuclease | NM_004111 |
| *SPO11* | endonuclease | MM_012444 |
| *FLJ35220 (ENDOV)* | incision 3' of hypoxanthine and uracil | MM_173627 |
| *FANCA* | Involved in tolerance or repair of DNA crosslinks | NM_000135 |
| *FANCB* | FANCA, FANCB, FANCC, FANCD2, FANCE, FANCF, FANCG, FANCL | NM_152633 |
| *FANCC* | | NM_000136 |
| *FANCD2* | | NM_033084 |
| *FANCE* | | NM_021922 |
| *FANCF* | | MM_022725 |
| *FANCG (XRCC9)* | | NM_004629 |
| *FANCL* | | NM_018062 |
| *DCLRE1A (SNM1)* | DNA crosslink repair | NM_014881 |
| *DCLRE1B (SNM1B)* | Related to SNM1 | NM_022836 |
| *NEIL3* | Resembles NEIL1 and NEIL2 | NM_018248 |
| *ATRIP (TREX1)* | ATR-interacting protein 5' alternative ORF of the | MM_130384 |
| | TREX1/ATRIP gene | |
| *NTH* | Removes damaged pyrimidines | NP_416150.1 |
| *NEI* | Removes damaged pyrimidines | NP_415242.1 |
| *NFI* | Deoxyinosine 3' endonuclease | NP_418426.1 |
| *MUTM* | Formamidopyrimidine DNA glycosylase | NP_418092.1 |
| *UNG* | Uracil-DNA glycosylase | NP_417075.1 |
| *UVRA* | DNA excision repair enzyme complex | NP_418482.1 |
| *UVRB* | | NP_415300.1 |
| *UVRC* | UVRA, UVRB, UVRC | NP_416423.3 |
| *DENV* | Pyrimidine dimer glycosylase | NP_049733.1 |

### Example 12: Repair of DNA prior to use in DNA sequencing reactions to increase the sensitivity of the sequencing reactions

The sensitivity of the sequencing reaction is intended to mean that the amount of template DNA having a correct sequence prior to sequencing results in reduced background noise and increased signal. This makes possible longer and/or more complete sequence reads. The improved fidelity of the sequence read is an additional benefit. The beneficial use of a repair mix such as described below can be observed for sequencing methods in general. For example, sequencing methods include 454 sequencing, single molecule sequencing, Sanger sequencing and Maxam-Gilbert sequencing.

Two DNA samples are subjected to DNA sequencing before and after DNA repair. The two DNA samples are UV treated for 40 seconds (see Example 6) and lambda DNA is exposed to light in the presence of 25 µg/mL methylene blue.

Prior to use in the DNA sequencing reaction the DNA to be sequenced is contacted with one or more repair enzymes under conditions that permit activity of the repair enzymes. For example, 0.5 µg template DNA for sequencing is mixed with NEB Thermopol buffer to 1X concentration (New England Biolabs, Inc., Ipswich, MA) (1X concentration of Thermopol buffer contains 2.0 mM Tris-HCI, pH 8.8 at 25°C, 10 mM KCl, 10 mM (NH₄)₂SO₄, 2 mM MgSO₄, 0.1% Triton X-100) and incubated for 15 minutes at room temperature with a DNA repair mixture (200 units *Taq* DNA ligase, 0.1 units *E*. *coli* PolI, 1 unit T4 pdg, 15 units Endo IV, 2.5 units Endo VIII, 0.1 unit Fpg, and optionally 0.5 unit *E. coli* UDG). The repaired DNA is either used immediately for sequencing or is purified and concentrated using a commercial kit (a Qiagen, Inc., Valencia, CA kit, for example) prior to DNA sequencing. The sequencing reaction may be performed by the classical Sanger sequencing reactions or by methods described in U.S. Publication No. 2005/0100932, U.S. Patent No. 6,897,023, or Margulies, et al. Nature 437(7057):376-80 (2005).

The sensitivity of the sequencing reaction and fidelity of the results are improved as a result of the pre-incubation with the repair mixture.

### Example 13: A multi-enzyme repair mix for repairing damaged DNA is effective at a single temperature

Lambda DNA was treated by 30s irradiation with UV (see Example 6). L72-5R (SEQ ID NO:1) and L30350F (SEQ ID NO:2) primers were selected for amplifying a 5 kilobase amplicon from the UV-treated lambda DNA either with or without prior repair. The DNA repair mix contained 200 units/µL Taq DNA ligase, 0.1 units/µL *E. coli* PolI, 1 unit/µL T4 pdg, 15 units/µL Endo IV, 0.5 unit/µL *E. coli* UDG, 2.5 units/µL Endo VIII, and 0.1 unit/µL Fpg stored in 20 mM Tris-HCl, pH 7.5 at 25°C, 100 mM NaCl, and 50% glycerol. Fifty ng of the 30 s UV-treated lambda DNA was added to thermocycler tubes each containing, 1X Thermopol buffer, 100 µM dNTPs, and 0.5 mM NAD⁺. 1 µL of the repair enzyme mix was added to 4 out of the 8 tubes and all were brought to a final volume of 47 µL with H₂O. Two tubes containing repair enzymes and 2 tubes lacking the enzymes were incubated at room temperature for 15 minutes. The remaining solutions were incubated overnight at 4°C. After the indicated incubation times, the primers (1 µM), dNTPs (100 µM), and 2.5 units *Taq* DNA polymerase were added to each thermocycler tube and the solutions placed into a MyCycler thermocycler running the program (Bio-Rad, Hercules, CA): 95°C for 2 min, one cycle; 95°C for 10 sec, 60°C for 30 sec and 72°C for 5 min, 25 cycles; 72°C for 5 min, one cycle; and a 4°C hold. 25 µL of each reaction was analyzed on a 1% agarose gel.

In contrast to the findings of others (U.S. Publication No. 2006/0014154), which required the use of multiple different temperatures to achieve repair, incubation of UV-damaged DNA with the above repair mixture at room temperature for 15 minutes or 4°C overnight produced an amplification product of the correct size (Figures 13A and 13B).

### Example 14: Repair of plasmid DNA containing multiple uracils and amplification using Vent® DNA polymerase

Plasmid pNEB0.92U was purified from *E. coli* CJ236 (NEB# E4141S, New England Biolabs, Inc., Ipswich, MA). The sequence is shown in Figure 18. Because *E. coli* CJ236 lacks dUTPase and uracil-N-glycosylase, this plasmid contains uracils randomly distributed throughout its sequence. The archaeal polymerase Vent® DNA polymerase is inhibited by uracil containing templates. Amplification of a 920 base amplicon from pNEB0.92U DNA was examined using primers S1224S (CGCCAGGGTTTTCCCAGTCACGAC) (SEQ ID NO:12 and S1233S (AGCGGATAACAATTTCACACAGGA) (SEQ ID NO:13) either with or without prior repair. The DNA repair mix contained 200 units/µL Taq DNA ligase, 0.1 units/µL *E. coli* PolI, 1 unit/µL T4 pdg, 15 units/µL Endo IV, 0.5 unit/µL *E*. *coli* UDG, 2.5 units/µL Endo VIII, and 0.1 unit/µL Fpg and stored in 20 mM Tris-HCl, pH 7.5 at 25°C, 100 mM NaCl, and 50% glycerol. One µL of the repair enzyme mix was added to 2 of 4 thermocycler tubes each containing 0.5 ng pNEB0.92U, 1X Thermopol buffer, 100 µM dNTPs, and 0.5 mM NAD⁺. All were brought to a final volume of45 µL with H₂O. The reaction solutions were incubated at room temperature for 15 minutes after which the primers (final concentration: 0.4 µM), dNTPs (final concentration: 100 µM), and 1 unit Vent® DNA polymerase were added to each tube. The solutions were placed into a MyCycler thermocycler running the program: 95°C for 2 min, one cycle; 95°C for 10 sec, 65°C for 30 sec and 72°C for 1 min, 25 cycles; 72°C for 5 min, one cycle; then a 4°C hold. 25 µL of each reaction was examined by electrophoresis on a 1% agarose gel.

Amplification from pNEB0.92U without removal of uracils using Vent® DNA polymerase produced a barely detectable product of the desired size. Treatment of the pNEB0.92U with the repair mix significantly increased the amount of amplicon produced from this plasmid using Vent® DNA polymerase (Figure 14).

### Example 15: Enhanced amplicon yield from DNA fragments

The template in this reaction was a set of 20 overlapping synthetic single strand oligonucleotides with an average size of approximately 45 nucleotides. The oligonucleotide sequences are shown below:
NEB oligo No. 316-219 (New England Biolabs, Inc., Ipswich, MA):
   GGCGGCCTCGAGGCGAAACGCCGCAACTGCITfCCGGGCGATACC (SEQ ID NO:14)
NEB oligo No. 316-220 (New England Biolabs, Inc., Ipswich, MA):
   CGGCACGCCATCAATCTGCACCAGAATGCGGGTATCGCCCGGAAA (SEQ ID NO:15)
NEB oligo No. 316-221 (New England Biolabs, Inc., Ipswich, MA):
   ATTGATGGCGTGCCGCAGAAAATTACCCTGCGCGAACTGTATGAA (SEQ ID NO:16)
NEB oligo No. 316-222 (New England Biolabs, Inc., Ipswich, MA):
   CATGTTTTCATAGCGTTCATCTTCAAACAGTTCATACAGTTCGCG (SEQ ID NO:17)
NEB oligo No. 316-223 (New England Biolabs, Inc., Ipswich, MA):
   CGCTATGAAAACATGGTGTATGTGCGCAAAAAACCGAAACGCGAA (SEQ ID NO:18)
NEB oligo No. 316-224 (New England Biolabs, Inc., Ipswich, MA):
   GGTTTCCAGGTCAATGCTATACACTTTAATTTCGCGTTTCGGTTT (SEQ ID NO:19)
NEB oligo No. 316-227 (New England Biolabs, Inc., Ipswich, MA):
   ATTGACCTGGAAACCGGCAAAGTGGTGCTGACCGATATTGAAGAT (SEQ ID NO:20)
NEB oligo No. 316-228 (New England Biolabs, Inc., Ipswich, MA):
   CAGATGATCGGTCGCCGGCGCTTTAATCACATCTTCAATATCGGT (SEQ ID NO:21)
NEB oligo No. 316-229 (New England Biolabs, Inc., Ipswich, MA):
   GCGACCGATCATCTGATTCGCTTTGAACTGGAAGATGGCCGCAGC (SEQ ID NO:22)
NEB oligo No. 316-230 (New England Biolabs, Inc., Ipswich, MA):
   CAGCACCGGATGATCCACGGTGGTTTCAAAGCTGCGGCCATCTTC (SEQ ID NO:23)
NEB oligo No. 316-233 (New England Biolabs, Inc., Ipswich, MA):
   GATCATCCGGTGCTGGTGTATGAAAACGGCCGCTTTATTGAAAAA (SEQ ID NO:24)
NEB oligo No. 316-234 (New England Biolabs, Inc., Ipswich, MA):
   TTTATCGCCTTCTTTCACTTCAAACGCGCGTTTTTCAATAAAGCG (SEQ ID NO:25)
NEB oligo No. 316-237 (New England Biolabs, Inc., Ipswich, MA):
   AAAGAAGGCGATAAAGTGCTGGTGAGCGAACTGGAACTGGTGGAA (SEQ ID NO:26)
NEB oligo No. 316-238 (New England Biolabs, Inc., Ipswich, MA):
   TTTCGGGTTATCCTGGCTGCTGCTGCTCTGTTCCACCAGTTCCAG (SEQ ID NO:27)
NEB oligo No. 316-239 (New England Biolabs, Inc., Ipswich, MA):
   CAGGATAACCCGAAAAACGAAAACCTGGGCAGCCCGGAACATGAT (SEQ ID NO:28)
NEB oligo No. 316-240 (New England Biolabs, Inc., Ipswich, MA):
   ATATTTAATGTTTTTAATTTCCAGCAGCTGATCATGTTCCGGGCT (SEQ ID NO:29)
NEB oligo No. 316-247 (New England Biolabs, Inc., Ipswich, MA):
   AAAAACATTAAATATGTGCGCGCGAACGATGATTTTGTGTTTAGCCTG (SEQ ID NO:30)
NEB oligo No. 316-248 (New England Biolabs, Inc., Ipswich, MA):
   TTAATAATCACGTTATGATAIIIIIICGCGTTCAGGCTAAACACAAA (SEQ ID NO:31)
NEB oligo No. 316-265 (New England Biolabs, Inc., Ipswich, MA):
   TAACGTGATTATTAACGAAAACATTGTGACCCATGCGTGCGATG (SEQ ID NO:32)
NEB oligo No. 316-266(New England Biolabs, Inc., Ipswich, MA):
   GCCGCCCTGCAGACCGGTCAGATCTTCATCGCCATCGCACGCATGGG (SEQ ID NO:33)

The assembly reaction consisted of two parts: an assembly step and an amplification step. For the assembly step the standard reaction was 50 µL and contained 70 nM of each oligo, 100 µM dNTP, 0.5 mM NAD+, 10 mM Tris-HCl, pH 7.5 at 25°C, 2 mM MgCl₂, and 50 mM NaCl. No enzymes were added to the control reaction. The first experimental reaction also contained 400 units *Taq* DNA ligase, 0.1 units *E. coli* PolI, 5 units T4 pdg, and 20 units Endo IV. The second set of reactions contained enzymes used in the first reaction and lambda beta protein (Kmiec, et al. J. Biol. Chem. 256:12636-12639 (1981); Rybalchenko, N., et al, Proc. Natl. Acad. Sci. USA 101:17056-17060 (2004)) added at a 1:1 beta protein: nucleotide mole ratio. The third reaction set contained 400 units *Taq* DNA ligase, 0.1 units *E. coli* Poll, 5 units T4 pdg, 20 units Endo IV, 0.3 mM ATP, and a 3:1 nucleotide to RecA mole ratio. The RecA was from *E. coli* (NEB catalog #M0249L, New England Biolabs, Inc., Ipswich, MA). The fourth reaction contained 400 units *Taq* DNA ligase, 0.1 units *E. coli* PolI, 5 units T4 pdg, 20 units Endo IV, 0.3 mM ATP, a 3:1 nucleotide to RecA mole ratio, and a 1:1 beta protein nucleotide mole ratio. Glycerol content in each reaction was controlled for. The reaction mixtures were incubated for 30 minutes at room temperature.

After room temperature incubation, 5 µL of the assembly reaction was amplified using 200 µM dNTPs, 500 nM in oligonucleotides 316-219 and 316-266, 6 mM in MgSO₄, 1 unit of Vent® DNA Polymerase, and 1X Thermopol buffer final volume of 100 µL. The reactions were mixed and placed in a MyCycler (Bio-Rad, Hercules, CA) and the following thermal cycler touchdown program was used: 94°C for 2 minutes (1 cycle); 94°C for 30 seconds, 72°C - 62°C (decreasing 1°C per cycle) for 30 seconds, 72°C for 45 seconds (10 cycles); 94°C for 30 seconds, 62°C for 30 seconds, 72°C for 45 seconds (20 cycles); 72°C for 5 minutes (1 cycle), and a 4°C hold. Each reaction was performed in duplicate. 11 µL of 10X sample buffer was added to each sample and 25 µL was loaded onto a 1% agarose gel for electrophoresis.

The results are shown in Figure 15. When the assembly reaction contained no added repair proteins, no amplification product was detected after the amplification step. However, when repair proteins were added in the assembly reactions, the correct 620 bp amplicon was obtained from the amplification step. Inclusion of lambda beta protein and/or *E. coli* RecA further increased the yield of amplicon. It was concluded that in a system in which a DNA template is composed of fragments, the inclusion of DNA repair proteins facilitates the ability to produce an amplicon. Furthermore, this effect is enhanced when some of those DNA repair proteins are known to be involved in DNA recombination.

### Example 16: Enhanced transformation efficiency with damaged plasmid DNA for E. coli

The plasmid pUC19 (GenBank Accession #L09137) was applied to a 1% agarose gel and electrophoresed in the presence of ethidium bromide until the plasmid had moved into the gel. The DNA in the gel was subjected to 254 nm UV light for 60 seconds. During the UV exposure a gel plug containing the pUC19 plasmid was excised. The plasmid was extracted from the gel plug using a Qiagen gel extraction kit (Qiagen, Valencia, CA). 30 ng of UV irradiated DNA or non-irradiated DNA in a final volume of 25 µL was treated with 50 units *E. coli* DNA ligase (NEB#M0205S, New England Biolabs, Inc., Ipswich, MA), 0.1 units *E. coli* PolI, 5 units T4 pdg, and 20 units Endo IV in a buffer of 1X Thermopol buffer (NEB#B9004S, New England Biolabs, Inc., Ipswich, MA) with added NAD⁺ (Sigma product #N-7004, Sigma-Aldrich, St. Louis, MO) and dNTPs (NEB#N0447S, New England Biolabs, Inc., Ipswich, MA) to 0.5 mM and 100 µM, respectively. The reaction was incubated 15 minutes at room temperature before using the DNA to transform *E. coli* DH-5 alpha (NEB#C2991H, New England Biolabs, Inc., Ipswich, MA). As a control, both UV irradiated and non-irradiated DNA were treated as above in the absence of added enzymes. The DH5 alpha cells were transformed with UV irradiated and non-irradiated plasmid DNA that had been treated with repair enzymes or not so treated. The transformation was performed by heat shocking the *E. coli* in the presence of plasmid DNA. 50 µL of *E. coli* and plasmid were incubated on ice for 30 minutes before a 30 second incubation at 42°C. The transformation reaction was then placed on ice for 2 minutes before plating the cells on LB agar plates containing 100 µg/mL ampicillin. LB agar plates with differing dilutions of each transfomation were placed in a 37°C incubator overnight to determine the transformation efficiency of the plasmid.

Plasmid pUC19 that had been subjected to UV irradiation and not repaired had a significantly reduced transformation efficiency when compared to undamaged pUC19 plasmid that had been treated with a repair enzyme mix (see Figure 16).

### Example 17: Simultaneous repair and blunting of DNA for subsequent ligation required for PCR, cloning or immobilization

DNA libraries are commonly made from the environment, tissues, or cell cultures (Brady, S. F., et al. Applied and Environmental Microbiology, 70(11):6865-6870 (2004); Current Protocols in Molecular Biology, Vol. 1, Ausubel, F., et al (editors), John Wiley & Sons, Inc., Hoboken, NJ; Chapter 5: "Construction of Recombinant DNA Libraries" (2004); Courtois, S., et al., Applied and Environmental Microbiology, 69(1):49-55 (2003); US Patent No. 6,444,426). These libraries are routinely created by shearing the DNA from the desired source by sonication, enzymatic treatment, or nebulization, preparing the DNA ends and ligating the mixture to oligonucleotides or plasmid DNA (Weinmann, A. S., Molecular and Cellular Biology, 21(20):6820-6832 (2001)). Ligation to oligonucleotides permits subsequent PCR or immobilization on arrays containing DNA sequences complimentary to the ligated oligo, Ligation to a plasmid permits the propagation in a heterologous host. A recent use of libraries is in chromatin immunoprecipitation (Guenther, M. G., et al. Proc. Natl. Acad. Sci. USA 102(24):8603-8608 (2005); Ren, B., et al. Genes Dev. 16:245-256 (2002); and Odom, D. T., et al. Science 303:1378-1381 (2004)). As part of preparing DNA ends for blunt end ligation, researchers often use a polymerase such as T4 DNA polymerase. However, an enzyme mix is provided here that can not only repair damage that the DNA may have acquired during purification, preparation and storage, but can also create blunt ends. This enzyme mix includes a ligase and a proof-reading polymerase and any cofactors necessary to allow enzyme activity. Preferably, the mix is composed of a ligase, a proof-reading polymerase, an apurinic/apyrimidinic endonuclease, UDG, FPG, and T4 pdg.

As an example, Chromatin IP (ChIP) is performed on HeLa cell DNA using antibodies to E2F1, E2F2, E2F3, E2F4, E2F5, or E2F6 as described previously (Weinmann, A. S. Molecular and Cellular Biology 21(20):6820-6832 (2001)). The cloning of ChIP enriched DNA is as described previously (Weinmann, A. S. Molecular and Cellular Biology 21(20):6820-6832 (2001)); http://mcardle.oncology.wisc.edu/farnham). The use of T4 DNA polymerase alone to blunt the DNA is replaced by an enzyme mixture containing at least a combination of a ligase and a proof-reading polymerase. For example, the DNA is incubated with 400 units *Taq* DNA ligase, 0.1 units *E. coli* polymerase I, 20 units *E. coli* endonuclease IV, 5 units T4 PDG in 1X Thermopol buffer supplemented with 0.5 mM NAD⁺ and 100 µM dNTPs at room temperature for 15 minutes. Prior to the ligation step the blunted and repaired DNA can be incubated at 75°C for 20 minutes to inactivate the *E. coli* polymerase I.

The mix of a proof-reading polymerase and at least a ligase is able to blunt the DNA ends for subsequent ligation to either primers or a plasmid.

### Example 18: Enhanced amplicon yield from fragmented DNA. Production of larger DNA pieces from fragmented DNA for downstream processes such as amplification, DNA sequencing, microarray analysis, and hybridization analysis

Fragmented DNA, from 0.1-1000 ng, is incubated with a recombination/DNA annealing proficient protein, such as *E. coli* RecA (NEB# M0249S, New England Biolabs, Inc., Ipswich, MA; West, S.C. Ann. Rev. Biochem. 61, 603-640 (1992)) and/or lambda beta protein (Rybalchenko, N., et al. Proc. Natl. Acad. Sci. USA, 101(49):17056-17060 (2004); Kmeic, E., & Holloman, W. K., J. Biol. Chem. 256(24):12636-12639 (1981)) in a standard reaction buffer and any required cofactors in a final volume of 5-1000 µL. An example of a standard reaction buffer is 10 mM Tris-HCl, pH 7.5 at 25°C, 2 mM MgCl₂, and 50 mM NaCl. When using RecA, 1 mM ATP is included in the standard reaction. Either simultaneous with or subsequent to the incubation with the RecA and/or beta protein the DNA is also contacted with a repair mix composed of at least a DNA ligase activity a DNA polymerase activity and any required cofactors, i.e., ATP, NAD⁺ and dNTPs. The repair mix contains 400 *units Taq* DNA ligase and 0.1 units *E. coli* PolI and, in addition, 5 units T4 pdg, 20 units Endo IV, 0.5 units *E. coli* UDG, 2.5 units/µL Endo VIII, and/or 0.1 unit Fpg are added. Prior to incubation with the repair proteins the DNA fragments may be heat-denatured and the temperature reduced to less than 39°C. For example, the DNA in reaction buffer may be heated to 98°C for 5 minutes then cooled down to less than 39°C. A standard reaction volume is 5 to 1000 µL and the incubation time is 1 to 60 minutes at 4-37°C. Typically, the RecA or beta protein is used at a 0.5:1 to 5:1 nucleotide to protein mole ratio.

Modifications to the above method include substituting RecA and/or beta protein with thermostable equivalents. Some examples of these proteins are ttRecA (Kato R, & Kuramitsu S., Eur J Biochem. 259(3):592-601 (1999)), *Taq* RecA, Tma RecA, and Apy RecA (Wetmur, J. G., et al. J Biol Chem. 269(41):25928-35 (1994)). The use of thermostable proteins means that thermostable RecA or beta-like protein can be mixed with the DNA during the denaturation step. Any co-factors required for the protein activity are also included. In addition, repair enzymes as described above are added prior to or after denaturation. Note that for thermostable recombination proteins (RecA or beta-like protein) the proteins can be added to the reaction mixture for 1-60 minutes at 45-75°C to permit the optimal activity before the addition of non-thermostable repair proteins at temperatures of less than 39°C.

The repaired DNA can then be used in a subsequent process, for example PCR. For example, as a test system human genomic DNA is fragmented using sonication and size fractionated to give average fragment sizes clustered around 200 bp. 500 ng of the size-fractionated material is treated as described above. A titration of 5-100 ng of this repaired material is used in PCR reactions using primers that reliably generate 1, 2, and 4 kb amplicons from undamaged human genomic DNA. Examples of a primer set are
DNMT-R:
GGGGCACCTTCTCCAACTCATACT (SEQ ID NO:34),
DNMT-1Fb:
cctcatttggggaggggttatct (SEQ ID NO:35),
DN MT-2Fc:
cctgaaacaaggttgtggcatagc (SEQ ID NO:36), and
DNMT-4Fb:
gagtgagttgaaagtgctccataca (SEQ ID NO:37).
The same template titration is performed with the fragmented DNA. When the non-repaired and repaired DNA are compared, the repaired templates permit a visible amplicon on an agarose gel, visualized with UV light and ethidium bromide, to be generated with at least two fold lower amounts of template DNA.

The use of RecA and/or beta protein-like activities in conjunction with at least ligase and polymerase activities results in the detection of PCR amplicons at lower template amounts as compared to unrepaired DNA.

### Example 19: Amplification of DNA from stored ancient cave bear tissue samples after repair of DNA damage

In contrast to modern material, the DNA extracted from ancient bones shows a variety of types of damage. The most common type of damage is fragmentation caused by single stand breaks, which lead to a reduced average molecule length of the extracted DNA, as well as non-enzymatic attacks such as irradiation and reactive oxygen species. (See: Hoss, et al. Nucleic Acids Res. 24(7):1304-7 (1996)). Repairing ancient DNA (aDNA) damage is important to improve the utility of the extracted DNA.

An ancient DNA was extracted as described in Paabo Proc Natl Acad Sci USA 86(6): 1939-43 (1989). Amplification of a 330 bp cave bear DNA (~44,000 years old) was performed using primers CB F1 (CTATTTAAACTATTCCCTGGTACATAC) (SEQ ID NO:38) and CB R1 (GGAGCGAGAGGTACACGT) (SEQ ID NO:39) either with or without prior repair. The DNA repair mix was composed of 200 units/µL *Taq* DNA ligase, 0.1 units/µL *E. coli* PolI, 1 unit/µL T4 pdg, 15 units/µL Endo IV, 0.5 unit/µL *E*. *coli* UDG, 2.5 units/µL Endo VIII, and 0.1 unit/µL Fpg and stored in 20 mM Tris-HCl, pH 7.5 at 25°C, 100 mM NaCl, and 50% gycerol. To 2 of 4 thermocycler tubes each containing 2 µl aDNA, 1X Phusion™ polymerase buffer, 100 µM dNTPs, and 0.5 mM NAD⁺ was added 1 µL of the repair enzyme mix. All were brought to a final volume of 45 µL with H₂O. The reaction solutions were incubated at room temperature for 15 minutes after which the primers (to 0.4 µM), dNTPs (to 100 µM), and 1 unit Phusion™ DNA polymerase were added to each. The solutions were placed into a MyCycler thermocycler (Bio-Rad, Hercules, CA) running the program: 98°C for 30 s, one cycle; 98°C for 10 sec, 58°C for 20 s and 72°C for 20 s, 30 cycles; 72°C for 5 min, one cycle; then a 4°C hold. The repaired PCR amplified DNA and control PCR amplified DNA (no repair) was used immediately in a second PCR amplification using nested primers. The amplification reaction with the nested primers used 1X *Taq* Master Mix (Catalog # M0270S, New England Biolabs, Inc., Ipswich, MA), 2 µL the previous amplification, and primers CB F1 (CTATTTAAACTATTCCCTGGTACATAC) (SEQ ID NO:40) and CB F3 (GCCCCATGCATATAAGCATG) (SEQ ID NO:41) at a final concentration of 0.2 µM. The total reaction volume was 50 µL. The reaction was analyzed by applying 5 µl of each reaction to a 1% agarose gel, prepared, electrophoresed and visualized as described above.

The amount of mitochondrial DNA in the cave bear bone samples was estimated with the TaqMan® assay (Applied Biosystems, Foster City, CA) using primers 5'-AAAATGCCCTTTGGATCTTAAA-3' (SEQ ID NO:43) and 5'-ACTGCTGTATCCCGTGGG-3' (SEQ ID NO:44).

The amplified DNA is either used immediately in the DNA sequencing methodology or subjected to DNA purification and concentration. After purification the DNA is subjected to DNA sequencing (see Example 12).

Amplification from cave bear DNA using Phusion DNA polymerase and Taq polymerase in nested PCR produced a detectable product of the desired amplicon size in one sample (CB3A). Treatment with the repair mix produced another amplicon from sample CB3B. Sequence analysis of amplicons from treated and untreated cave bear DNAs (CB3B sample) will reveal whether treatment with the repair mix significantly helped to remove PCR amplification errors associated with DNA modifications described in Hoss, et al. Nucleic Acids Res. 24(7):1304-7 (1996).

Treatment of the cave bear DNA template with the repair enzyme mix permitted Phusion™ DNA polymerase, to more effectively produce the desired amplicon and to remove PCR amplification errors (Figure 17).

## Claims

1. A method for enhancing at least one of fidelity and yield of a copied or amplified product by repairing a damaged polynucleotide, **characterized in that** it comprises:
incubating the polynucleotide in the absence of endonuclease (Endo) VI in a reaction mixture comprising an NAD-dependent ligase, an NAD⁺ cofactor, a polymerase and at least one AP endonuclease;
so as to enhance at least one of fidelity and yield of the copied or amplified product.

2. A method according to claim 1 wherein the NAD-dependent ligase is *Taq* ligase or *E. coli* ligase; or wherein at least one AP endonuclease comprises an endonuclease obtainable from *E*. *coli, human* or *Thermus* species; or wherein at least one AP endonuclease is *E*. *coli* Endo IV; or wherein the polymerase is selected from a *Taq* polymerase, an *E. coli* polymerase, a Bst polymerase, and an archaeal polymerase or mutant thereof; or wherein the polymerase is a member of the Y family of polymerases.

3. A method according to claim 2 wherein the archaeal polymerase is selected from Pfu, Vent^{®}, Deep Vent^{®}, 9°North, KOD or Pwo polymerases; or wherein the Y family polymerase is selected from *E. coli* polIV, *E. coli* polV, human pol kappa, human pol eta, Sso Dpo4, Sac Dbh, Sce pol zeta and human pol iota.

4. A method according to claim 1 wherein the reaction mixture further comprises: a T7 Endo I or mutant thereof; or T4 pyrimidine dimer glycosylase; or [fapy]-DNA glycosylase (Fpg); or at least one of a UvrA, UvrB, UvrC, UvrD and Cho; or at least one of Endo VIII, Endo V or Endo III; or UDG and Aag.

5. A method according to claim 1 wherein amplification occurs by means selected from PCR amplification, helicase-dependent amplification, strand-displacement amplification, rolling circle amplification and whole genome amplification; or wherein the polynucleotide is an RNA and the amplification is RT-amplification; or wherein the polynucleotide is DNA and the reaction mixture comprising the polymerase and AP endonuclease comprises 1-100 units of *E. coli* Endo IV, 0.05-0.25 units of *E. coli* PolI and 5-500 units of Taq ligase added to 1-1000 ng DNA in 10-1000 µl; or wherein amplification of the polynucleotide is capable of producing an amplicon in a size range of 50 to 100,000 nucleotides in a polymerase chain reaction.

6. A composition which may be used in a method according to any of claims 1 to 5, **characterized in that** it comprises:
an effective amount of an NAD-dependent ligase, a polymerase and an AP endonuclease, not including Endo VI, for incubating with a polynucleotide at a single temperature so as to provide enhancement of at least one of yield and fidelity of a copied polynucleotide compared with a copied polynucleotide in the absence of the composition.

7. A composition according to claim 6 wherein the AP endonuclease is present at a concentration of 1-100 units, the polymerase is present at 0.05-5 units, and the NAD-dependent ligase is present at 5-500 units in a volume of 10-500 µl, which may be suitable for enhanced copying of 1-1000 ng DNA.

8. A composition according to claim 6 wherein the AP endonuclease is a Type II AP endonuclease; or wherein the polymerase is *E. coli* polI or Bst polymerase.

9. A composition according to claim 8, wherein the AP endonuclease *is E. coli* Endo IV.

10. A composition according to claim 6 wherein it further comprises: T4 pyrimidine dimer glycosylase; or T4 pyrimidine dimer glycosylase and Fpg; or at least one of UvrA, UvrB, UvrC, UvrD and Cho; or at least one of Endo VIII, Endo V or Endo III; or at least one of UDG and Aag.

11. A method for using a composition according to any of claims 6 to 10 **characterized in that** it is a method for cloning or sequencing a polynucleotide fragment, comprising:
repairing the polynucleotide fragment by means of the composition;
and cloning or sequencing the polynucleotide fragment.

12. A method for using a composition according to any of claims 6 to 10 **characterized in that** it is a method for enhancing the yield of a copied or amplified polynucleotide, comprising:
(a) obtaining at least a first pair and a second pair of primers wherein the second pair of primers is nested within the first set of primers when hybridized to the polynucleotide;
(b) subjecting the polynucleotide to the composition;
(c) amplifying the polynucleotide with the first set of primers;
(d) amplifying the product of (c) with the second set of primers;
and
(e) obtaining an enhanced yield of amplified polynucleotide.

13. A method according to Claim 12, wherein the composition comprises an NAD-dependent ligase, a polymerase, a pdg, an endonuclease IV, an endonuclease VIII and Fpg; or wherein the composition comprises an NAD-dependent ligase, a polymerase, a pdg, an endonuclease IV, an endonuclease VIII, Fpg and a UDG.

14. A method according to Claim 13, wherein the NAD-dependent ligase is a *Taq* DNA ligase, the polymerase is an *E. Coli* PolI, the PDG is a T4 pdg; and the Endo IV, Endo VIII, and Fpg are obtained from *E.Coli.*

15. A method according to Claim 14, wherein the UDG is obtained from *E. Coli.*

16. A method for using a composition according to any of claims 6 to 10 **characterized in that** it is a method for sequencing a polynucleotide, comprising:
(a) contacting the polynucleotide with the composition;
and
(b) sequencing the polynucleotide.

17. A method for using a composition according to any of claims 6 to 10 **characterized in that** it is a method for copying or amplifying a fragmented DNA, comprising:
(a) contacting the fragmented DNA with the composition; and
(b) amplifying or copying the fragmented DNA.

18. A method according to Claim 17, further comprising adding a recombinant competent protein.

19. A kit **characterized in that** it comprises an NAD-dependent ligase, a polymerase and at least one AP endonuclease, not including Endo VI; wherein the ligase, polymerase and AP endonuclease are formulated for addition to a polynucleotide preparation to enhance copying of the polynucleotide; and instructions for the use thereof.

## Patentansprüche

1. Verfahren zum Erhöhen der Fidelität und/oder Ausbeute eines kopierten oder amplifizierten Produkts durch Reparieren eines beschädigten Polynucleotids, **dadurch gekennzeichnet, dass** es Folgendes beinhaltet:
Inkubieren des Polynucleotids in Abwesenheit von Endonuclease (Endo) VI in einem Reaktionsgemisch, das eine NAD-abhängige Ligase, einen NAD⁺-Cofaktor, eine Polymerase und wenigstens eine AP-Endonuclease beinhaltet;
um die Fidelität und/oder Ausbeute des kopierten oder amplifizierten Produkts zu erhöhen.

2. Verfahren nach Anspruch 1, wobei die NAD-abhängige Ligase *Taq*-Ligase oder *E*. *coli*-Ligase ist; oder wobei wenigstens eine AP-Endonuclease eine Endonuclease beinhaltet, die von *E. coli, humanen* oder *Thermus*-Spezies erhältlich ist; oder wobei wenigstens eine AP-Endonuclease *E. coli* Endo IV ist; oder wobei die Polymerase aus einer *Taq*-Polymerase, einer *E. coli*-Polymerase, einer Bst-Polymerase und einer Archaeal-Polymerase oder Mutante davon ausgewählt ist; oder wobei die Polymerase ein Mitglied der Y-Familie von Polymerasen ist.

3. Verfahren nach Anspruch 2, wobei die Archaeal-Polymerase aus Pfu-, Vent®-, Deep Vent®-, 9°North-, KOD- oder Pwo-Polymerasen ausgewählt ist; oder wobei die Polymerase der Y-Familie aus *E. coli* Pol IV, *E. coli* Pol V, humaner Pol-Kappa, humaner Pol-Eta, Sso Dpo4, Sac Dbh, Sce Pol-Zeta und humaner Pol-Iota ausgewählt ist.

4. Verfahren nach Anspruch 1, wobei das Reaktionsgemisch ferner Folgendes beinhaltet: T7 Endo I oder eine Mutante davon; oder T4-Pyrimid-Dimer-Glykosylase; oder [Fapy]-DNA-Glykosylase (Fpg); oder wenigstens eines aus UvrA, UvrB, UvrC, UvrD und Cho; oder wenigstens eines aus Endo VIII, Endo V oder Endo III; oder UDG und Aag.

5. Verfahren nach Anspruch 1, wobei die Amplifikation mit einem Mittel durchgeführt wird, das aus PCR-Amplifikation, Helikase-abhängiger Amplifikation, Strangverlagerungs-Amplifikation, Rolling-Circle-Amplifikation und Ganzgenomamplifikation ausgewählt ist; oder wobei das Polynucleotid eine RNA ist und die Amplifikation RT-Amplifikation ist; oder wobei das Polynucleotid DNA ist und das Reaktionsgemisch, das die Polymerase und AP-Endonuclease beinhaltet, 1-100 Einheiten von *E. coli* Endo IV, 0,05-0,25 Einheiten von *E. coli* Pol I und 5-500 Einheiten von Taq-Ligase zugegeben zu 1-1000 ng DNA in 10-1000 µl beinhaltet; oder wobei die Amplifikation des Polynucleotids ein Amplikon in einem Größenbereich von 50 bis 100.000 Nucleotiden in einer Polymerasekettenreaktion produzieren kann.

6. Zusammensetzung, die in einem Verfahren nach einem der Ansprüche 1 bis 5 verwendet werden kann, **dadurch gekennzeichnet, dass** sie Folgendes beinhaltet:
eine wirksame Menge einer NAD-abhängigen Ligase, eine Polymerase und eine AP-Endonuclease, Endo VI nicht eingeschlossen, zum Inkubieren mit einem Polynucleotid bei einer einzigen Temperatur, um die Ausbeute und/oder Fidelität eines kopierten Polynucleotids im Vergleich zu einem kopierten Polynuleotid in Abwesenheit der Zusammensetzung zu erhöhen.

7. Zusammensetzung nach Anspruch 6, wobei die AP-Endonuclease in einer Konzentration von 1-100 Einheiten vorliegt, die Polymerase mit 0,05-5 Einheiten vorliegt und die NAD-abhängige Ligase mit 5-500 Einheiten in einem Volumen von 10-500 µl vorliegt, was für ein verbessertes Kopieren von 1-1000 ng DNA geeignet sein kann.

8. Zusammensetzung nach Anspruch 6, wobei die AP-Endonuclease eine AP-Endonuclease vom Typ II ist; oder wobei die Polymerase *E. coli* Pol I- oder Bst-Polymerase ist.

9. Zusammensetzung nach Anspruch 8, wobei die AP-Endonuclease *E. coli* Endo IV ist.

10. Zusammensetzung nach Anspruch 6, die ferner Folgendes beinhaltet: T4-Pyrimidin-Dimer-Glykosylase; oder T4-Pyrimidin-Dimer-Glykosylase und Fpg; oder wenigstens eines aus UvrA, UvrB, UvrC, UvrD und Cho; oder wenigstens eines aus Endo VIII, Endo V oder Endo III; oder wenigstens eines aus UDG und Aag.

11. Verfahren zum Verwenden einer Zusammensetzung nach einem der Ansprüche 6 bis 10, **dadurch gekennzeichnet, dass** es ein Verfahren zum Klonieren oder Sequenzieren eines Polynucleotidfragments ist, das Folgendes beinhaltet:
Reparieren des Polynucleotidfragments mit Hilfe der Zusammensetzung;
und Klonieren oder Sequenzieren des Polynucleotidfragments.

12. Verfahren zum Verwenden einer Zusammensetzung nach einem der Ansprüche 6 bis 10, **dadurch gekennzeichnet, dass** es ein Verfahren zum Erhöhen der Ausbeute eines kopierten oder amplifizierten Polynucleotids ist, das Folgendes beinhaltet:
(a) Erhalten von wenigstens einem ersten Paar und einem zweiten Paar von Primern, wobei das zweite Paar von Primern innerhalb des ersten Satzes von Primern verschachtelt ist, wenn mit dem Polynucleotid hybridisiert;
(b) Aussetzen des Polynucleotids der Zusammensetzung;
(c) Amplifizieren des Polynucleotids mit dem ersten Satz von Primern;
(d) Amplifizieren des Produkts von (c) mit dem zweiten Satz von Primern; und
(e) Erhalten einer erhöhten Ausbeute des amplifizierten Polynucleotids.

13. Verfahren nach Anspruch 12, wobei die Zusammensetzung eine NAD-abhängige Ligase, eine Polymerase, eine Pdg, eine Endonuclease IV, eine Endonuclease VIII und Fpg beinhaltet; oder wobei die Zusammensetzung eine NAD-abhängige Ligase, eine Polymerase, eine Pdg, eine Endonuclease IV, eine Endonuclease VIII, Fpg und eine UDG beinhaltet.

14. Verfahren nach Anspruch 13, wobei die NAD-abhängige Ligase eine *Taq* DNA-Ligase ist, die Polymerase eine *E. coli* Pol I ist, die PDG eine T4 Pdg ist; und Endo IV, Endo VIII und Fpg von *E. Coli* erhalten werden.

15. Verfahren nach Anspruch 14, wobei die UDG von *E. coli* erhalten wird.

16. Verfahren zum Verwenden einer Zusammensetzung nach einem der Ansprüche 6 bis 10, **dadurch gekennzeichnet, dass** es ein Verfahren zum Sequenzieren eines Polynucleotids ist, das Folgendes beinhaltet:
(a) Inkontaktbringen des Polynucleotids mit der Zusammensetzung; und
(b) Sequenzieren des Polynucleotids.

17. Verfahren zum Verwenden einer Zusammensetzung nach einem der Ansprüche 6 bis 10, **dadurch gekennzeichnet, dass** es ein Verfahren zum Kopieren oder Amplifizieren einer fragmentierten DNA ist, das Folgendes beinhaltet:
(a) Inkontaktbringen der fragmentierten DNA mit der Zusammensetzung; und
(b) Amplifizieren oder Kopieren der fragmentierten DNA.

18. Verfahren nach Anspruch 17, das ferner das Zugeben eines rekombinanten kompetenten Proteins beinhaltet.

19. Kit, der **dadurch gekennzeichnet ist, dass** er Folgendes enthält: eine NAD-abhängige Ligase, eine Polymerase und wenigstens eine AP-Endonuclease, Endo VI nicht eingeschlossen; wobei die Ligase, Polymerase und AP-Endonuclease für die Zugabe zu einem Polynucleotidpräparat formuliert sind, um das Kopieren des Polynucleotids zu verbessern; und Anweisungen für seine Verwendung.

## Revendications

1. Procédé d'amélioration d'au moins une caractéristique parmi la fidélité et le rendement d'un produit copié ou amplifié par réparation d'un polynucléotide endommagé, **caractérisé en ce qu'**il comprend :
la mise en incubation du polynucléotide en l'absence d'endonucléase (Endo) VI dans un mélange réactionnel comprenant une ligase NAD-dépendante, un cofacteur NAD⁺, une polymérase et au moins une endonucléase AP ;
de façon à améliorer au moins une caractéristique parmi la fidélité et le rendement du produit copié ou amplifié.

2. Procédé selon la revendication 1, dans lequel la ligase NAD-dépendante est une ligase *Taq* ou une ligase d'*E*. *coli ;* ou dans lequel au moins une endonucléase AP comprend une endonucléase qui peut être obtenue à partir d'une espèce *E. coli, human* ou *Thermus ;* ou dans lequel au moins une endonucléase AP est une Endo IV d'*E*. *coli ;* ou dans lequel la polymérase est sélectionnée parmi une polymérase *Taq,* une polymérase d'*E*. *coli,* une polymérase Bst, et une polymérase archéale ou une mutante de celle-ci ; ou dans lequel la polymérase est un membre de la famille Y de polymérases.

3. Procédé selon la revendication 2, dans lequel la polymérase archéale est sélectionnée parmi des polymérases Pfu, Vent®, Deep Vent®, 9°North, KOD ou Pwo ; ou dans lequel la polymérase de la famille Y est sélectionnée parmi une polymérase IV d'*E*. *coli,* une polymérase V d'*E*. *coli,* une polymérase kappa humaine, une polymérase et a humaine, une polymérase Dpo4 Sso, une polymérase Dbh Sac, une polymérase zeta Sce et une polymérase iota humaine.

4. Procédé selon la revendication 1, dans lequel le mélange réactionnel comprend en outre : une endonucléase Endo I T7 ou une mutante de celle-ci ; ou une glycosylase T4 spécifique des dimères de pyrimidine ; ou une [fapy]-ADN glycosylase (Fpg) ; ou au moins l'une d'une UvrA, d'une UvrB, d'une UvrC, d'une UvrD et d'une Cho ; ou au moins l'une d'Endo VIII, d'Endo V ou d'Endo III ; ou UDG et Aag.

5. Procédé selon la revendication 1, dans lequel l'amplification est réalisée par un moyen sélectionné parmi l'amplification PCR, l'amplification hélicase-dépendante, l'amplification par déplacement de brins, l'amplification par cercle roulant et l'amplification du génome entier ; ou dans lequel le polynucléotide est un ARN et l'amplification est une amplification RT ; ou dans lequel le polynucléotide est un ADN et le mélange réactionnel comprenant la polymérase et l'endonucléase AP comprend 1 à 100 unités d'Endo IV d'*E*. *coli,* 0,05 à 0,25 unité de polymérase I d'*E*. *coli* et 5 à 500 unités de ligase Taq ajoutées à 1 à 1 000 ng d'ADN dans 10 à 1 000 µl ; ou dans lequel l'amplification du polynucléotide est capable de produire un amplicon dans une plage de tailles de 50 à 100 000 nucléotides dans une réaction en chaîne par polymérase.

6. Composition qui peut être utilisée dans un procédé selon l'une quelconque des revendications 1 à 5, **caractérisée en ce qu'**elle comprend :
une quantité efficace d'une ligase NAD-dépendante, d'une polymérase et une d'endonucléase AP, ne comprenant pas d'Endo IV, pour la mise en incubation avec un polynucléotide à une seule température de façon à produire une amélioration d'au moins une caractéristique parmi le rendement et la fidélité d'un polynucléotide copié par comparaison avec un polynucléotide copié en l'absence de la composition.

7. Composition selon la revendication 6, dans laquelle l'endonucléase AP est présente à une concentration de 1 à 100 unités, la polymérase est présente à une concentration de 0,05 à 5 unités, et la ligase NAD-dépendante est présente à une concentration de 5 à 500 unités dans un volume de 10 à 500 µl, qui peut convenir à une utilisation pour l'amélioration de la copie de 1 à 1 000 ng d'ADN.

8. Composition selon la revendication 6, dans laquelle l'endonucléase AP est une endonucléase AP de Type II, ou dans laquelle la polymérase est une polymérase I d'*E*. *coli* ou une polymérase Bst.

9. Composition selon la revendication 8, dans laquelle l'endonucléase AP est une Endo IV d'*E*. *coli.*

10. Composition selon la revendication 6, laquelle comprend en outre : une glycosylase T4 spécifique des dimères de pyrimidine ; ou une glycosylase T4 spécifique des dimères de pyrimidine et une Fpg ; ou au moins l'une d'une UvrA, d'une UvrB, d'une UvrC, d'une UvrD et d'une Cho ; ou au moins l'une d'Endo VIII, d'Endo V ou d'Endo III ; ou au moins l'une d'UDG et d'Aag.

11. Procédé d'utilisation d'une composition selon l'une quelconque des revendications 6 à 10, **caractérisé en ce qu'**il s'agit d'un procédé de clonage ou de séquençage d'un fragment de polynucléotide, comprenant :
la réparation du fragment de polynucléotide au moyen de la composition ;
et le clonage ou le séquençage du fragment de polynucléotide.

12. Procédé d'utilisation d'une composition selon l'une quelconque des revendications 6 à 10, **caractérisé en ce qu'**il s'agit d'un procédé d'amélioration du rendement d'un polynucléotide copié ou amplifié, comprenant :
(a) l'obtention d'au moins une première paire et une deuxième paire d'amorces, la deuxième paire d'amorces étant imbriquée dans la première paire d'amorces quand elle est hybridée avec le polynucléotide ;
(b) l'exposition du polynucléotide à la composition ;
(c) l'amplification du polynucléotide avec le premier ensemble d'amorces ;
(d) l'amplification du produit de (c) avec le deuxième ensemble d'amorces ;
et
(e) l'obtention d'un rendement amélioré de polynucléotide amplifié.

13. Procédé selon la revendication 12, dans lequel la composition comprend une ligase NAD-dépendante, une polymérase, une Pdg, une endonucléase IV, une endonucléase VIII et une Fpg ; ou dans lequel la composition comprend une ligase NAD-dépendante, une polymérase, une Pdg, une endonucléase IV, une endonucléase VIII, une Fpg et une UDG.

14. Procédé selon la revendication 13, dans lequel la ligase NAD-dépendante est une ADN ligase *Taq,* la polymérase est une polymérase I d'*E*. *coli,* la Pdg est une Pdg T4 ; et l'Endo IV, l'Endo VIII et la Fpg sont obtenues à partir d'*E*. *coli.*

15. Procédé selon la revendication 14, dans lequel l'UDG est obtenue à partir d'*E*. *coli.*

16. Procédé d'utilisation d'une composition selon l'une quelconque des revendications 6 à 10, **caractérisé en ce qu'**il s'agit d'un procédé de séquençage d'un polynucléotide, comprenant :
(a) la mise en contact du polynucléotide avec la composition ;
et
(b) le séquençage du polynucléotide.

17. Procédé d'utilisation d'une composition selon l'une quelconque des revendications 6 à 10, **caractérisé en ce qu'**il s'agit d'un procédé de copie ou d'amplification d'un ADN fragmenté, comprenant :
(a) la mise en contact de l'ADN fragmenté avec la composition ;
et
(b) l'amplification ou la copie de l'ADN fragmenté.

18. Procédé selon la revendication 17, comprenant en outre l'addition d'une protéine compétente recombinante.

19. Kit **caractérisé en ce qu'**il comprend : une ligase NAD-dépendante, une polymérase et au moins une endonucléase AP, ne comprenant pas d'Endo VI ; dans lequel la ligase, la polymérase et l'endonucléase AP sont formulées pour une addition à une préparation de polynucléotide pour améliorer la copie du polynucléotide, et un mode d'emploi pour ledit kit.
